(19) Europäisches Patentamt / European Patent Office / Office européen des brevets

(11) **EP 4 372 001 A1**

(12) **EUROPEAN PATENT APPLICATION**
published in accordance with Art. 153(4) EPC

(43) Date of publication:
**22.05.2024 Bulletin 2024/21**

(21) Application number: **22841462.9**

(22) Date of filing: **14.07.2022**

(51) International Patent Classification (IPC):
**C07K 16/00** $^{(2006.01)}$    **A61K 39/395** $^{(2006.01)}$
**C07K 16/28** $^{(2006.01)}$    **C12N 15/13** $^{(2006.01)}$

(52) Cooperative Patent Classification (CPC):
**A61K 39/395; C07K 16/00; C07K 16/28**

(86) International application number:
**PCT/CN2022/105714**

(87) International publication number:
**WO 2023/284829 (19.01.2023 Gazette 2023/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.07.2021 CN 202110794137**

(71) Applicants:
• **Jiangsu Hengrui Pharmaceuticals Co., Ltd.
Lianyungang, Jiangsu 222047 (CN)**
• **Shanghai Hengrui Pharmaceutical Co., Ltd.
Shanghai 200245 (CN)**

(72) Inventors:
• **YE, Xin**
**Shanghai 200245 (CN)**
• **SUN, Le**
**Shanghai 200245 (CN)**
• **ZHANG, Weiwei**
**Shanghai 200245 (CN)**
• **TAO, Weikang**
**Shanghai 200245 (CN)**

(74) Representative: **Dragotti & Associati S.R.L.
Via Nino Bixio, 7
20129 Milano (IT)**

Remarks:
The complete document including Reference
Table(s) and the Sequence Listing(s) can be
downloaded from the EPO website

(54) **ANTIGEN-BINDING MOLECULE SPECIFICALLY BINDING TO HGFR AND EGFR, AND PHARMACEUTICAL USE THEREOF**

(57) An antigen-binding molecule specifically binding to HGFR and EGFR, and the pharmaceutical use thereof. The antigen-binding molecule can be used for treating tumor-related diseases.

**Description**

The present application claims priority to Chinese Patent Application No. 202110794137.9 filed on July 14, 2021.

**TECHNICAL FIELD**

The present disclosure belongs to the field of biotechnology, and specifically, the present disclosure relates to an antigen-binding molecule and use thereof.

**BACKGROUND**

The statements herein merely provide background information related to the present disclosure and may not necessarily constitute the prior art.

Epidermal growth factor receptor (EGFR) and hepatocyte growth factor receptor (HGFR) are two receptor tyrosine kinases, which are commonly and highly expressed in a variety of tumor cells, such as non-small cell lung cancer (NSCLC), colorectal cancer (CRC), head and neck cancer (HNC), and the like.

The EGFR signaling pathway plays an important role in tumor biology by regulating cell proliferation, angiogenesis, and metastasis and survival of cancer cells, so that the dysregulation of this pathway leads to tumorigenesis. HGFR is also associated with the development of many tumors due to the overexpression of HGFR, activating mutations, and abnormal signal activation caused by autocrine or paracrine signal transduction or gene amplification. An important link between the EGFR and HGFR signaling pathways was found by investigation of cancer efficacy. NSCLC accounts for 83% of all lung cancers, and EGFR activating mutations are the most common type (10%-15% for white, 50% for Asian). EGFR tyrosine kinase inhibitors (TKIs) have been used as first-line therapy for NSCLC. Despite the high initial response rates (70%-80%), resistance usually occurs within a year. There are two major mechanisms of drug resistance. One is the occurrence of another EGFR mutation, such as EGFR T790M, and the other is the compensatory activation of the HGFR signaling pathway. These factors both affect the therapeutic effects of the tumor.

**SUMMARY**

The present disclosure provides an antigen-binding molecule comprising at least one antigen-binding moiety 1 specifically binding to HGFR and at least one antigen-binding moiety 2 specifically binding to EGFR.

In one aspect, the present disclosure provides an antigen-binding molecule comprising at least one antigen-binding moiety 1 specifically binding to HGFR and at least one antigen-binding moiety 2 specifically binding to EGFR, wherein

the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3, wherein:

(i) the M-HCDR1, the M-HCDR2, and the M-HCDR3 comprise amino acid sequences of a HCDR1, a HCDR2, and a HCDR3 set forth in SEQ ID NO: 16, respectively, and the M-LCDR1, the M-LCDR2, and the M-LCDR3 comprise amino acid sequences of a LCDR1, a LCDR2, and a LCDR3 set forth in SEQ ID NO: 17, respectively; or
(ii) the M-HCDR1, the M-HCDR2, and the M-HCDR3 comprise amino acid sequences of a HCDR1, a HCDR2, and a HCDR3 set forth in SEQ ID NO: 12, respectively, and the M-LCDR1, the M-LCDR2, and the M-LCDR3 comprise amino acid sequences of a LCDR1, a LCDR2, and a LCDR3 set forth in SEQ ID NO: 13, respectively; or
(iii) the M-HCDR1, the M-HCDR2, and the M-HCDR3 comprise amino acid sequences of a HCDR1, a HCDR2, and a HCDR3 set forth in SEQ ID NO: 14, respectively, and the M-LCDR1, the M-LCDR2, and the M-LCDR3 comprise amino acid sequences of a LCDR1, a LCDR2, and a LCDR3 set forth in SEQ ID NO: 15, respectively;

the antigen-binding moiety 2 comprises a heavy chain variable region E-VH and a light chain variable region E-VL, wherein the E-VH comprises an E-HCDR1, an E-HCDR2, and an E-HCDR3, and the E-VL comprises an E-LCDR1, an E-LCDR2, and an E-LCDR3, wherein the E-HCDR1, the E-HCDR2, and the E-HCDR3 comprise amino acid sequences of a HCDR1, a HCDR2, and a HCDR3 set forth in SEQ ID NO: 3, respectively, and the E-LCDR1, the E-LCDR2, and the E-LCDR3 comprise amino acid sequences of a LCDR1, a LCDR2, and a LCDR3 set forth in SEQ ID NO: 5, respectively.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to Kabat, IMGT, Chothia, AbM, or Contact numbering scheme.

In some embodiments, the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to the Kabat numbering scheme.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to the IMGT numbering scheme.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to the Chothia numbering scheme.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to the AbM numbering scheme.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, E-LCDR3, HCDR1, HCDR2, HCDR3, LCDR1, LCDR2, and LCDR3 are defined according to the Contact numbering scheme.

In some embodiments, provided is the antigen-binding molecule according to any one of the embodiments described above, wherein the M-HCDR1, M-HCDR2, M-HCDR3, M-LCDR1, M-LCDR2, and M-LCDR3 are defined according to the Kabat numbering scheme, wherein

(i) the M-HCDR1 comprises an amino acid sequence set forth in SEQ ID NO: 30, the M-HCDR2 comprises an amino acid sequence set forth in SEQ ID NO: 31, the M-HCDR3 comprises an amino acid sequence set forth in SEQ ID NO: 32, the M-LCDR1 comprises an amino acid sequence set forth in SEQ ID NO: 27, the M-LCDR2 comprises an amino acid sequence set forth in SEQ ID NO: 33, and the M-LCDR3 comprises an amino acid sequence set forth in SEQ ID NO: 29; or

(ii) the M-HCDR1 comprises an amino acid sequence set forth in SEQ ID NO: 18, the M-HCDR2 comprises an amino acid sequence set forth in SEQ ID NO: 19, the M-HCDR3 comprises an amino acid sequence set forth in SEQ ID NO: 20, the M-LCDR1 comprises an amino acid sequence set forth in SEQ ID NO: 21, the M-LCDR2 comprises an amino acid sequence set forth in SEQ ID NO: 22, and the M-LCDR3 comprises an amino acid sequence set forth in SEQ ID NO: 23; or

(iii) the M-HCDR1 comprises an amino acid sequence set forth in SEQ ID NO: 24, the M-HCDR2 comprises an amino acid sequence set forth in SEQ ID NO: 25, the M-HCDR3 comprises an amino acid sequence set forth in SEQ ID NO: 26, the M-LCDR1 comprises an amino acid sequence set forth in SEQ ID NO: 27, the M-LCDR2 comprises an amino acid sequence set forth in SEQ ID NO: 28, and the M-LCDR3 comprises an amino acid sequence set forth in SEQ ID NO: 29; and

the E-HCDR1, the E-HCDR2, the E-HCDR3, the E-LCDR1, the E-LCDR2, and the E-LCDR3 are defined according to the Kabat numbering scheme, the E-HCDR1 comprises an amino acid sequence set forth in SEQ ID NO: 6, the E-HCDR2 comprises an amino acid sequence set forth in SEQ ID NO: 7, the E-HCDR3 comprises an amino acid sequence set forth in SEQ ID NO: 8, the E-LCDR1 comprises an amino acid sequence set forth in SEQ ID NO: 9, the E-LCDR2 comprises an amino acid sequence set forth in SEQ ID NO: 10, and the E-LCDR3 comprises an amino acid sequence set forth in SEQ ID NO: 11.

In some embodiments, provided is the antigen-binding molecule according to any one of the embodiments described above, wherein in the antigen-binding moiety 1 specifically binding to HGFR, an M-VH thereof has: an M-HCDR1 comprising the amino acid sequence set forth in SEQ ID NO: 18, an M-HCDR2 comprising the amino acid sequence set forth in SEQ ID NO: 19, and an M-HCDR3 comprising the amino acid sequence set forth in SEQ ID NO: 20, and an M-VL thereof has: an M-LCDR1 comprising the amino acid sequence set forth in SEQ ID NO: 21, an M-LCDR2 comprising the amino acid sequence set forth in SEQ ID NO: 22, and an M-LCDR3 comprising the amino acid sequence set forth in SEQ ID NO: 23; and

in the antigen-binding moiety 2 specifically binding to EGFR, an E-VH thereof has: an E-HCDR1 comprising the amino acid sequence set forth in SEQ ID NO: 6, an E-HCDR2 comprising the amino acid sequence set forth in SEQ ID NO: 7, and an E-HCDR3 comprising the amino acid sequence set forth in SEQ ID NO: 8, and an E-VL thereof has: an E-LCDR1 comprising the amino acid sequence set forth in SEQ ID NO: 9, an E-LCDR2 comprising the amino acid sequence set forth in SEQ ID NO: 10, and an E-LCDR3 comprising the amino acid sequence set forth in SEQ ID NO: 11.

In some embodiments, the antigen-binding molecule according to any one of the embodiments described above comprises two antigen-binding moieties 1 specifically binding to HGFR, wherein in one of the antigen-binding moieties 1, an M-VH thereof has: an M-HCDR1 comprising the amino acid sequence set forth in SEQ ID NO: 30, an M-HCDR2 comprising the amino acid sequence set forth in SEQ ID NO: 31, and an M-HCDR3 comprising the amino acid sequence set forth in SEQ ID NO: 32, and an M-VL thereof has: an M-LCDR1 comprising the amino acid sequence set forth in SEQ ID NO: 27, an M-LCDR2 comprising the amino acid sequence set forth in SEQ ID NO: 33, and an M-LCDR3 comprising the amino acid sequence set forth in SEQ ID NO: 29; in the other antigen-binding moiety 1, an M-VH thereof has: an M-HCDR1 comprising the amino acid sequence set forth in SEQ ID NO: 18, an M-HCDR2 comprising the amino acid sequence set forth in SEQ ID NO: 19, and an M-HCDR3 comprising the amino acid sequence set forth in SEQ ID NO: 20, and an M-VL thereof has: an M-LCDR1 comprising the amino acid sequence set forth in SEQ ID NO: 21, an M-LCDR2 comprising the amino acid sequence set forth in SEQ ID NO: 22, and an M-LCDR3 comprising the amino acid sequence set forth in SEQ ID NO: 23; and

an antigen-binding moiety 2 specifically binding to EGFR, wherein in the antigen-binding moiety 2, an E-VH thereof has: an E-HCDR1 comprising the amino acid sequence set forth in SEQ ID NO: 6, an E-HCDR2 comprising the amino acid sequence set forth in SEQ ID NO: 7, and an E-HCDR3 comprising the amino acid sequence set forth in SEQ ID NO: 8, and an E-VL thereof has: an E-LCDR1 comprising the amino acid sequence set forth in SEQ ID NO: 9, an E-LCDR2 comprising the amino acid sequence set forth in SEQ ID NO: 10, and an E-LCDR3 comprising the amino acid sequence set forth in SEQ ID NO: 11.

In some embodiments, provided is the antigen-binding molecule described above, wherein the M-HCDR1, M-HCDR2, M-HCDR3, M-LCDR1, M-LCDR2, M-LCDR3, E-HCDR1, E-HCDR2, E-HCDR3, E-LCDR1, E-LCDR2, and E-LCDR3 are defined according to the Kabat numbering scheme.

In some embodiments, provided is the antigen-binding molecule according to any one of the embodiments described above, wherein:

(i) the M-VH comprises an amino acid sequence having at least 90%, 95%, 96%, 97%, 98%, 99%, or 100% sequence identity to SEQ ID NO: 16, and the M-VL comprises an amino acid sequence having at least 90%, 95%, 96%, 97%, 98%, 99%, or 100% sequence identity to SEQ ID NO: 17, or

the M-VH comprises an amino acid sequence having at least 90%, 95%, 96%, 97%, 98%, 99%, or 100% sequence identity to SEQ ID NO: 12, and the M-VL comprises an amino acid sequence having at least 90%, 95%, 96%, 97%, 98%, 99%, or 100% sequence identity to SEQ ID NO: 13, or
the M-VH comprises an amino acid sequence having at least 90%, 95%, 96%, 97%, 98%, 99%, or 100% sequence identity to SEQ ID NO: 14, and the M-VL comprises an amino acid sequence having at least 90%, 95%, 96%, 97%, 98%, 99%, or 100% sequence identity to SEQ ID NO: 15; and/or

(ii) the E-VH comprises an amino acid sequence having at least 90%, 95%, 96%, 97%, 98%, 99%, or 100% sequence identity to SEQ ID NO: 3, and the E-VL comprises an amino acid sequence having at least 90%, 95%, 96%, 97%, 98%, 99%, or 100% sequence identity to SEQ ID NO: 5 or SEQ ID NO: 4.

In some embodiments, the antigen-binding molecules described above are equivalent in the following functions, including binding to the same epitope, having the same affinity (e.g., within KD $\pm$ 50%), or other functions disclosed herein.

In some embodiments, provided is the antigen-binding molecule according to any one of the embodiments described above, wherein:

(i) the M-VH comprises the amino acid sequence set forth in SEQ ID NO: 16, and the M-VL comprises the amino acid sequence set forth in SEQ ID NO: 17, or

the M-VH comprises the amino acid sequence set forth in SEQ ID NO: 12, and the M-VL comprises the amino acid sequence set forth in SEQ ID NO: 13, or
the M-VH comprises the amino acid sequence set forth in SEQ ID NO: 14, and the M-VL comprises the amino acid sequence set forth in SEQ ID NO: 15; and/or

(ii) the E-VH comprises the amino acid sequence set forth in SEQ ID NO: 3, and the E-VL comprises the amino acid sequence set forth in SEQ ID NO: 5 or SEQ ID NO: 4.

In some embodiments, provided is the antigen-binding molecule according to any one of the embodiments described above, wherein:

(i) the antigen-binding molecule comprises two antigen-binding moieties 1 specifically binding to HGFR, wherein the M-VH of one of the antigen-binding moieties 1 comprises the amino acid sequence set forth in SEQ ID NO: 16, and the M-VL comprises the amino acid sequence set forth in SEQ ID NO: 17; the M-VH of the other antigen-binding moiety 1 comprises the amino acid sequence set forth in SEQ ID NO: 12, and the M-VL comprises the amino acid sequence set forth in SEQ ID NO: 13; and the E-VH comprises the amino acid sequence set forth in SEQ ID NO: 3, and the E-VL comprises the amino acid sequence set forth in SEQ ID NO: 5; or

(ii) the antigen-binding molecule comprises at least one antigen-binding moiety 1 specifically binding to HGFR and at least one antigen-binding moiety 2 specifically binding to EGFR, wherein the E-VH comprises the amino acid sequence set forth in SEQ ID NO: 3, the E-VL comprises the amino acid sequence set forth in SEQ ID NO: 5, the M-VH comprises the amino acid sequence set forth in SEQ ID NO: 12, and the M-VL comprises the amino acid sequence set forth in SEQ ID NO: 13.

In some embodiments, the antigen-binding molecule according to any one of the embodiments described above comprises two antigen-binding moieties 1 specifically binding to HGFR, an antigen-binding moiety 2 specifically binding to EGFR, and an Fc region, wherein the Fc region comprises a first subunit Fc1 and a second subunit Fc2 capable of associating with each other.

In some embodiments, the antigen-binding moiety 2 specifically binding to EGFR is an Fv specifically binding to EGFR; the two antigen-binding moieties 1 specifically binding to HGFR are a first Fab specifically binding to HGFR and a substituted Fab specifically binding to HGFR, respectively; the substituted Fab comprises an M-VH, an M-VL, a Titin chain, and an Obscurin chain, the Titin chain and the Obscurin chain being capable of forming a dimer, wherein the C-terminus of the M-VH of the substituted Fab is fused to the N-terminus of the Titin chain directly or via a linker, and the C-terminus of the M-VL of the substituted Fab is fused to the N-terminus of the Obscurin chain directly or via a linker, or the C-terminus of the M-VH of the substituted Fab is fused to the N-terminus of the Obscurin chain directly or via a linker, and the C-terminus of the M-VL of the substituted Fab is fused to the N-terminus of the Titin chain directly or via a linker.

In some embodiments, the C-terminus of an E-VH of the Fv specifically binding to EGFR is fused to the N-terminus of a heavy chain of the first Fab specifically binding to HGFR directly or via a linker, the C-terminus of an E-VL of the Fv specifically binding to EGFR is fused to the N-terminus of a light chain of the first Fab specifically binding to HGFR directly or via a linker, the C-terminus of the heavy chain of the first Fab specifically binding to HGFR is fused to the N-terminus of the Fc1 directly or via a linker, and the C-terminus of the Titin chain or the C-terminus of the Obscurin chain is fused to the N-terminus of the Fc2 directly or via a linker. In some embodiments, the M-VH of the substituted Fab is on the same chain as the Fc2.

In some embodiments, the antigen-binding molecule according to any one of the embodiments described above comprises an antigen-binding moiety 1 specifically binding to HGFR, an antigen-binding moiety 2 specifically binding to EGFR, and an Fc region, wherein the Fc region comprises a first subunit Fc1 and a second subunit Fc2 capable of associating with each other. In some embodiments, the antigen-binding moiety 1 is a Fab; the antigen-binding moiety 2 is a substituted Fab comprising an E-VH, an E-VL, a Titin chain, and an Obscurin chain, the Titin chain and the Obscurin chain being capable of forming a dimer, wherein the C-terminus of the E-VH is fused to the N-terminus of the Titin chain directly or via a linker, and the C-terminus of the E-VL is fused to the N-terminus of the Obscurin chain directly or via a linker, or the C-terminus of the E-VH is fused to the N-terminus of the Obscurin chain directly or via a linker, and the C-terminus of the E-VL is fused to the N-terminus of the Titin chain directly or via a linker. In some embodiments, the C-terminus of a heavy chain of the Fab specifically binding to HGFR is fused to the N-terminus of the Fc1 directly or via a linker, and the C-terminus of the Titin chain or the C-terminus of the Obscurin chain is fused to the N-terminus of the Fc2 directly or via a linker. In some embodiments, the E-VH is on the same chain as the Fc2.

In some embodiments, the antigen-binding molecule according to any one of the embodiments described above comprises an Fc region, wherein the Fc region is preferably an IgG Fc region, more preferably an $IgG_1$ Fc region. In some embodiments, the Fc region comprises a first subunit Fc1 and a second subunit Fc2 capable of associating with each other, and the Fc1 and the Fc2 each independently have one or more amino acid substitutions that reduce homodimerization of the Fc region.

In some embodiments, (i) the Fc1 has a protuberance structure according to the knob-into-hole technique, and the Fc2 has a pore structure according to the knob-into-hole technique; preferably, the Fc1 has an amino acid residue at position 366 being W, the Fc2 has an amino acid residue at position 366 being S, an amino acid residue at position 368 being A, and an amino acid residue at position 407 being V, and the amino acid residue positions are numbered according to the EU index; and/or (ii) an engineered disulfide bond is present between the first subunit Fc1 and the second subunit Fc2; preferably, the Fc1 has an amino acid residue at position 354 being C, the Fc2 has an amino acid residue at position 349 being C, and the amino acid residue positions are numbered according to the EU index; more preferably, the Fc1 has an amino acid residue at position 354 being C, an amino acid residue at position 356 being E, an amino acid residue at position 358 being M, and an amino acid residue at position 366 being W, the Fc2 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V, and the amino acid residue positions are numbered according to the EU index.

In some other embodiments, (i) the Fc2 has a protuberance structure according to the knob-into-hole technique, and the Fc1 has a pore structure according to the knob-into-hole technique; preferably, the Fc2 has an amino acid residue at position 366 being W, the Fc1 has an amino acid residue at position 366 being S, an amino acid residue at position 368 being A, and an amino acid residue at position 407 being V, and the amino acid residue positions are numbered according to the EU index; and/or (ii) an engineered disulfide bond is present between the first subunit Fc2 and the second subunit Fc1; preferably, the Fc2 has an amino acid residue at position 354 being C, the Fc1 has an amino acid residue at position 349 being C, and the amino acid residue positions are numbered according to the EU index; more preferably, the Fc2 has an amino acid residue at position 354 being C, an amino acid residue at position 356 being E, an amino acid residue at position 358 being M, and an amino acid residue at position 366 being W, the Fc1 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V, and the amino acid residue positions are numbered according to the EU index.

In some embodiments, the Fc1 has an amino acid sequence set forth in SEQ ID NO: 107, and the Fc2 has an amino acid sequence set forth in SEQ ID NO: 108.

In some embodiments, provided is the antigen-binding molecule according to any one of the embodiments described above, wherein:

the antigen-binding molecule comprises: a first chain having a structure of formula (a), a second chain having a structure of formula (b), a third chain having a structure of formula (c), and a fourth chain having a structure of formula (d), wherein

formula (a) is [E-VH]-[linker 1]-[M-VH]-[CH1]-[Fc1],
formula (b) is [E-VL]-[linker 2]-[M-VL]-[CL],
formula (c) is [M-VH]-[linker 3]-[Titin chain]-[Fc2], and
formula (d) is [M-VL]-[linker 4]-[Obscurin chain].

In some embodiments, the linkers in formula (a), formula (b), formula (c), and formula (d) are identical or different peptide linkers. In some embodiments, the peptide linkers each independently have a structure of $L_1$-(GGGGS)n-$L_2$, wherein $L_1$ is a bond, A, GS, GGS, or GGGS (SEQ ID NO: 116), n is 0, 1, 2, 3, 4, 5, 6, 7, 8, 9, or 10, $L_2$ is a bond, G, GG, GGG, or GGGG (SEQ ID NO: 117), and the peptide linkers are not bonds. In some embodiments, the peptide linker is 3-15 amino acid residues in length. In some embodiments, the peptide linkers each independently have a structure of (GGGGS)n, wherein n is 1, 2, or 3. In some embodiments, the linkers in formula (a), formula (b), formula (c), and formula (d) comprise an amino acid sequence set forth in SEQ ID NO: 105 or SEQ ID NO: 106. In some embodiments, the linkers in formula (a) and formula (b) comprise the amino acid sequence set forth in SEQ ID NO: 105, and the linkers in formula (c) and formula (d) comprise the amino acid sequence set forth in SEQ ID NO: 106. In some embodiments, the antigen-binding molecule has a first chain, a second chain, a third chain, and a fourth chain. In some embodiments, the M-VH in formula (a) and the M-VH in formula (c) are not identical, and the M-VL in formula (b) and the M-VL in formula (d) are not identical. In some embodiments, the antigen-binding molecule has: a first chain comprising an amino acid sequence set forth in SEQ ID NO: 34, a second chain comprising an amino acid sequence set forth in SEQ ID NO: 35, a third chain comprising an amino acid sequence set forth in SEQ ID NO: 36, and a fourth chain comprising an amino acid sequence set forth in SEQ ID NO: 37.

In some embodiments, the antigen-binding molecule according to any one of the embodiments described above comprises: a first chain having a structure of formula (e), a second chain having a structure of formula (f), a third chain having a structure of formula (g), and a fourth chain having a structure of formula (h), wherein

formula (e) is [M-VH]-[CH1]-[Fc1],
formula (f) is [M-VL]-[CL],
formula (g) is [E-VH]-[linker 1]-[Titin chain]-[Fc2], and
formula (h) is [E-VL]-[linker 2]-[Obscurin chain].

In some embodiments, the linkers in formula (g) and formula (h) are identical or different peptide linkers. In some embodiments, the peptide linkers each independently have a structure of $L_1$-(GGGGS)n-$L_2$, wherein $L_1$ is a bond, A, GS, GGS, or GGGS (SEQ ID NO: 116), n is 0, 1, 2, 3, 4, 5, 6, 7, 8, 9, or 10, $L_2$ is a bond, G, GG, GGG, or GGGG (SEQ ID NO: 117), and the peptide linkers are not bonds. In some embodiments, the peptide linker is 3-15 amino acid residues in length. In some embodiments, the linkers in formula (h) and formula (g) comprise the amino acid sequence set forth in SEQ ID NO: 106. In some embodiments, the antigen-binding molecule has: a first chain comprising an amino acid sequence set forth in SEQ ID NO: 38, a second chain comprising an amino acid sequence set forth in SEQ ID NO: 39, a third chain comprising an amino acid sequence set forth in SEQ ID NO: 40, and a fourth chain comprising an amino acid sequence set forth in SEQ ID NO: 41.

In some embodiments, the antigen-binding molecule according to any one of the embodiments described above is a bispecific antibody.

In another aspect, the present disclosure provides an antigen-binding molecule comprising an Fv, a first Fab, a substituted Fab, and an Fc region, wherein the Fc region comprises a first subunit Fc1 and a second subunit Fc2 capable of associating with each other, the Fv comprises a heavy chain variable region Fv-VH and a light chain variable region Fv-VL, the first Fab comprises a heavy chain variable region Fab1-VH and a light chain variable region Fab1-VL, and the substituted Fab comprises a heavy chain variable region Fab-S-VH, a light chain variable region Fab-S-VL, a Titin chain, and an Obscurin chain, the Titin chain and the Obscurin chain being capable of forming a dimer; wherein:

the C-terminus of the Fab-S-VH is fused to the N-terminus of the Titin chain directly or via a linker, and the C-terminus of the Fab-S-VL is fused to the N-terminus of the Obscurin chain directly or via a linker, or the C-terminus of the Fab-S-VH is fused to the N-terminus of the Obscurin chain directly or via a linker, and the C-terminus of the Fab-S-VL is fused to the N-terminus of the Titin chain directly or via a linker; and

the C-terminus of a heavy chain of the first Fab is fused to the N-terminus of the Fc1 directly or via a linker; the C-terminus of the Fv-VH of the Fv is fused to the N-terminus of the heavy chain of the first Fab directly or via a linker, the C-terminus of the Titin chain or the C-terminus of the Obscurin chain is fused to the N-terminus of the Fc2 directly or via a linker, and the Fab-S-VH is on the same chain as the Fc2;

In some embodiments, the first Fab and the substituted Fab each independently bind to a first antigen, and the Fv binds to a second antigen. In some embodiments, the first antigen is HGFR, and the second antigen is EGFR. In some embodiments, the first Fab and the substituted Fab bind different epitopes of the first antigen.

In some embodiments, the antigen-binding molecule according to any one of the embodiments described above comprises an Fc region, wherein the Fc region is preferably an IgG Fc region, more preferably an IgG$_1$ Fc region. In some embodiments, the Fc region comprises a first subunit Fc1 and a second subunit Fc2 capable of associating with each other, and the Fc1 and the Fc2 each independently have one or more amino acid substitutions that reduce homodimerization of the Fc region. In some embodiments, (i) the Fc1 has a protuberance structure according to the knob-into-hole technique, and the Fc2 has a pore structure according to the knob-into-hole technique; preferably, the Fc1 has an amino acid residue at position 366 being W, the Fc2 has an amino acid residue at position 366 being S, an amino acid residue at position 368 being A, and an amino acid residue at position 407 being V, and the amino acid residue positions are numbered according to the EU index; and/or (ii) an engineered disulfide bond is present between the first subunit Fc1 and the second subunit Fc2; preferably, the Fc1 has an amino acid residue at position 354 being C, the Fc2 has an amino acid residue at position 349 being C, and the amino acid residue positions are numbered according to the EU index; more preferably, the Fc1 has an amino acid residue at position 354 being C, an amino acid residue at position 356 being E, an amino acid residue at position 358 being M, and an amino acid residue at position 366 being W, the Fc2 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V, and the amino acid residue positions are numbered according to the EU index. In some embodiments, (i) the Fc2 has a protuberance structure according to the knob-into-hole technique, and the Fc1 has a pore structure according to the knob-into-hole technique; preferably, the Fc2 has an amino acid residue at position 366 being W, the Fc1 has an amino acid residue at position 366 being S, an amino acid residue at position 368 being A, and an amino acid residue at position 407 being V, and the amino acid residue positions are numbered according to the EU index; and/or (ii) an engineered disulfide bond is present between the first subunit Fc2 and the second subunit Fc1; preferably, the Fc2 has an amino acid residue at position 354 being C, the Fc1 has an amino acid residue at position 349 being C, and the amino acid residue positions are numbered according to the EU index; more preferably, the Fc2 has an amino acid residue at position 354 being C, an amino acid residue at position 356 being E, an amino acid residue at position 358 being M, and an amino acid residue at position 366 being W, the Fc1 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V, and the amino acid residue positions are numbered according to the EU index. In some embodiments, the Fc1 has an amino acid sequence set forth in SEQ ID NO: 107, and the Fc2 has an amino acid sequence set forth in SEQ ID NO: 108.

In some embodiments, provided is the antigen-binding molecule according to any one of the embodiments described above, wherein the Fc region comprises one or more amino acid substitutions that are capable of reducing the binding of the Fc region to an Fc receptor. In some embodiments, the amino acid substitutions are capable of reducing the binding of the Fc region to an Fcγ receptor. In some embodiments, the Fc region is a human IgG$_1$ Fc region, and the amino acid residues at positions 234 and 235 are A, as numbered according to the EU index.

In some embodiments, provided is the antigen-binding molecule according to any one of the embodiments described above, wherein:

the antigen-binding molecule comprises: a first chain having a structure of formula (k), a second chain having a structure of formula (1), a third chain having a structure of formula (m), and a fourth chain having a structure of formula (n), wherein

formula (k) is [Fv-VH]-[linker 1]-[Fab1-VH]-[CH1]-[Fc1],
formula (1) is [Fv-VL]-[linker 2]-[Fab1-VL]-[CL],
formula (m) is [Fab-S-VH]-[linker 3]-[Titin chain]-[Fc2], and
formula (n) is [Fab-S-VL]-[linker 4]-[Obscurin chain];

In some embodiments, the linkers in formula (k), formula (1), formula (m), and formula (n) are identical or different peptide linkers. In some embodiments, the peptide linkers each independently have a structure of $L_1$-(GGGGS)n-$L_2$, wherein $L_1$ is a bond, A, GS, GGS, or GGGS (SEQ ID NO: 116), n is 0, 1, 2, 3, 4, 5, 6, 7, 8, 9, or 10, $L_2$ is a bond, G, GG, GGG, or GGGG (SEQ ID NO: 117), and the peptide linkers are not bonds. In some embodiments, the peptide linker is 3-15 amino acid residues in length. In some embodiments, the peptide linkers each independently have a structure of (GGGGS)n, wherein n is 1, 2, or 3. In some embodiments, the linkers in formula (k), formula (1), formula (m), and formula (n) comprise the amino acid sequence set forth in SEQ ID NO: 105 or SEQ ID NO: 106. In some embodiments, the linkers in formula (k) and formula (1) comprise the amino acid sequence set forth in SEQ ID NO: 105, and the linkers in formula (m) and formula (n) comprise the amino acid sequence set forth in SEQ ID NO: 106. In some embodiments, the antigen-binding molecule has a first chain, a second chain, a third chain, and a fourth chain.

In some embodiments, provided is the antigen-binding molecule according to any one of the embodiments described above, wherein

the Titin chain comprises an amino acid sequence set forth in SEQ ID NO: 45 or a variant thereof, wherein the variant of SEQ ID NO: 45 has one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 60S, 64T, 66S/66K, 70R, 75V, 77S, 79T, 81R, 82M, 83D, and 84L as compared to SEQ ID NO: 45; and
the Obscurin chain comprises an amino acid sequence set forth in SEQ ID NO: 64 or a variant thereof, or an amino acid sequence set forth in SEQ ID NO: 65 or a variant thereof, wherein the variant of SEQ ID NO: 64 has one or more amino acid residue substitutions selected from the group consisting of 2E, 3C, 7K/7R, 9C, 11L, 12S, 13Y/13S, 14T, 17E, 20L, 22M/22S, 25S, 30D, 32P/32F, 34E, 36T, 41K, 42L, 44I, 45T, 48V, 53L, 58V, 62E/62K/62H, 66C, 67Q/67T, 69S, 76S, 82H, 88C, 89L, 92E, 93C, 94G, and 97G as compared to SEQ ID NO: 64; the variant of SEQ ID NO: 65 has one or more amino acid residue substitutions selected from the group consisting of 6E, 26S, 74C, 77S, 84C, and 86C as compared to SEQ ID NO: 65.

In some embodiments, provided is the antigen-binding molecule according to any one of the embodiments described above, wherein the variant of SEQ ID NO: 45 has an amino acid residue substitution selected from any one of the following:

8C, 25S, and 39T;
20C, 25S, and 39T;
25S, 26C, and 39T;
22C, 25S, and 39T;
8C, 25S, 39T, 66S, and 77S;
8C, 25S, 39T, 66K, 70R, 79T, and 81R;
3W, 8C, 11I, 13L, 22M, 25S, 39T, and 82M;
8C, 111, 25S, 39T, 66K, 79T, and 81R;
8C, 25S, 39T, 40S, 42K, 45S, 47E, 49G, 56S, 58E, 75V, 83D, and 84L;
8C, 25S, 39T, 47E, 49G, 56S, 58E, and 75V;
8C, 25S, 39T, 56S, 58E, and 75V;
8C, 25S, 39T, 56S, 58E, 66S, and 77S;
8C, 11I, 25S, 39T, 60S, 64T, 66K, 79T, and 81R;
8C, 11I, 20C, 25S, 39T, 60S, 64T, 66K, 79T, and 81R; and
8C, 11I, 25S, 26C, 39T, 60S, 64T, 66K, 79T, and 81R.

In some embodiments, the variant of SEQ ID NO: 45 has an amino acid sequence of KAGIR (SEQ ID NO: 118) at the N-terminus as compared to SEQ ID NO: 45.

In some embodiments, the variant of SEQ ID NO: 45 has only the amino acid residue substitution according to any one of the embodiments described above as compared to SEQ ID NO: 45. In some embodiments, the variant of SEQ ID NO: 45 comprises an amino acid sequence having at least 70%, 75%, 80%, 85%, 90%, or 95% sequence identity to SEQ ID NO: 45.

In some embodiments, provided is the antigen-binding molecule according to any one of the embodiments described above, wherein the variant of SEQ ID NO: 64 has an amino acid residue substitution selected from any one of the following:

88C;
3C;
9C;
25S, 76S, and 88C;
25S, 76S, and 3C;
25S, 76S, and 9C;
7K, 25S, 62K, 76S, and 88C;
7K, 25S, 62H, 76S, and 88C;
7R, 25S, 62K, 76S, and 88C;
7R, 25S, 62H, 76S, and 88C;
11L, 25S, 62K, 76S, and 88C;
11L, 25S, 62H, 76S, and 88C;
12S, 13Y, 14T, 22S, 25S, 62K, 76S, and 88C;
2E, 11L, 17E, 25S, 30D, 32P, 34E, 36T, 44I, 45T, 58V, 62E, 67Q, 69S, 76S, 88C, and 97G;
11L, 20L, 22M, 25S, 53L, 62K, 76S, and 88C;
11L, 25S, 41K, 45T, 62K, 67Q, 69S, 76S, 88C, and 89L;
11L, 25S, 42L, 45T, 62K, 67T, 69S, 76S, 88C, 92E, and 94;
11L, 12S, 13Y, 22S, 25S, 42L, 45T, 62K, 67Q, 69S, 76S, 88C, 92E, and 94G;
25S, 32F, 41K, 45T, 48V, 62K, 67Q, 69S, 76S, 88C, and 89L;
13S, 25S, 32F, 41K, 45T, 48V, 62K, 67Q, 69S, 76S, 82H, 88C, and 89L;
3C, 13S, 25S, 32F, 41K, 45T, 48V, 62K, 67Q, 69S, 76S, 82H, 88C, and 89L;
9C, 13S, 25S, 32F, 41K, 45T, 48V, 62K, 67Q, 69S, 76S, 82H, 88C, and 89L;
13S, 32F, 41K, 45T, 48V, 62K, 67Q, 69S, 82H, 88C, and 89L;
3C, 13S, 32F, 41K, 45T, 48V, 62K, 67Q, 69S, 82H, 88C, and 89L;
9C, 13S, 32F, 41K, 45T, 48V, 62K, 67Q, 69S, 82H, 88C, and 89L;
13S, 25S, 32F, 41K, 45T, 48V, 62K, 66C, 67Q, 69S, 76S, 82H, 88C, 89L, and 93C;
3C, 13S, 25S, 32F, 41K, 45T, 48V, 62K, 66C, 67Q, 69S, 76S, 82H, 88C, 89L, and 93C; and
9C, 13S, 25S, 32F, 41K, 45T, 48V, 62K, 66C, 67Q, 69S, 76S, 82H, 88C, 89L, and 93C.

In some embodiments, the variant of SEQ ID NO: 64 has an amino acid sequence of DQPQF (SEQ ID NO: 119) at the N-terminus as compared to SEQ ID NO: 64.

In some embodiments, the variant of SEQ ID NO: 64 has only the amino acid residue substitution according to any one of the embodiments described above as compared to SEQ ID NO: 64. In some embodiments, the variant of SEQ ID NO: 64 comprises an amino acid sequence having at least 70%, 75%, 80%, 85%, 90%, or 95% sequence identity to SEQ ID NO: 64.

In some embodiments, provided is the antigen-binding molecule according to any one of the embodiments described above, wherein the variant of SEQ ID NO: 65 has an amino acid residue substitution selected from any one of the following:

6E and 74C;
6E and 84C;
6E and 86C;
6E, 26S, 77S, and 74C;
6E, 26S, 77S, and 84C; and
6E, 26S, 77S, and 86C.

In some embodiments, the variant of SEQ ID NO: 65 has only the amino acid residue substitution according to any one of the embodiments described above as compared to SEQ ID NO: 65. In some embodiments, the variant of SEQ ID NO: 65 comprises an amino acid sequence having at least 70%, 75%, 80%, 85%, 90%, or 95% sequence identity to SEQ ID NO: 65.

In some embodiments, provided is the antigen-binding molecule according to any one of the embodiments described above, wherein the Titin chain comprises an amino acid sequence selected from the group consisting of SEQ ID NO: 45 to SEQ ID NO: 63, and the Obscurin chain comprises an amino acid sequence selected from the group consisting of SEQ ID NO: 64 to SEQ ID NO: 104.

In some embodiments, provided is the antigen-binding molecule according to any one of the embodiments described above, wherein the Titin chain comprises the amino acid sequence set forth in SEQ ID NO: 61, and the Obscurin chain comprises the amino acid sequence set forth in SEQ ID NO: 99.

In some embodiments, the present disclosure further provides an antigen-binding molecule comprising an antigen-binding moiety 2 specifically binding to EGFR and two antigen-binding moieties 1 specifically binding to HGFR, wherein the two antigen-binding moieties 1 bind to different epitopes of HGFR.

In some embodiments, provided are the two antigen-binding moieties 1 specifically binding to HGFR, wherein one of the antigen-binding moieties 1 competes with an anti-HGFR antibody comprising a heavy chain variable region set forth in SEQ ID NO: 16 and a light chain variable region set forth in SEQ ID NO: 17 for binding to human HGFR; the other antigen-binding moiety 1 competes with an anti-HGFR antibody comprising a heavy chain variable region set forth in SEQ ID NO: 12 and a light chain variable region set forth in SEQ ID NO: 13 for binding to the human HGFR.

In some embodiments, provided are the two antigen-binding moieties 1 specifically binding to HGFR, wherein one of the antigen-binding moieties 1 is capable of competing with Omab for the binding, and the other antigen-binding moiety 1 is capable of competing with Ab10.1 for the binding.

In some embodiments, provided are the two antigen-binding moieties 1 specifically binding to HGFR, wherein one of the antigen-binding moieties 1 is capable of competing with Omab for the binding, and the other antigen-binding moiety 1 is capable of competing with Ab10 for the binding.

In some embodiments, provided are the two antigen-binding moieties 1 specifically binding to HGFR, wherein one of the antigen-binding moieties 1 binds to the same epitope as Omab, and the other antigen-binding moiety 1 is capable of binding to the same epitope as Ab10.1.

In some embodiments, provided are the two antigen-binding moieties 1 specifically binding to HGFR, wherein one of the antigen-binding moieties 1 binds to the same epitope as Omab, and the other antigen-binding moiety 1 binds to the same epitope as Ab10.

In some embodiments, provided is the antigen-binding molecule according to any one of the embodiments described above, wherein the antigen-binding moiety 2 specifically binding to EGFR comprises a heavy chain variable region E-VH and a light chain variable region E-VL, wherein the E-VH comprises an E-HCDR1, an E-HCDR2, and an E-HCDR3, and the E-VL comprises an E-LCDR1, an E-LCDR2, and an E-LCDR3, wherein the E-HCDR1, the E-HCDR2, and the E-HCDR3 comprise amino acid sequences of a HCDR1, a HCDR2, and a HCDR3 set forth in SEQ ID NO: 3, respectively, and the E-LCDR1, the E-LCDR2, and the E-LCDR3 comprise amino acid sequences of a LCDR1, a LCDR2, and a LCDR3 set forth in SEQ ID NO: 5, respectively; the antigen-binding moiety 1 specifically binding to HGFR comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3, wherein: the M-HCDR1, the M-HCDR2, and the M-HCDR3 of one of the antigen-binding moieties 1 comprise amino acid sequences of a HCDR1, a HCDR2, and a HCDR3 set forth in SEQ ID NO: 16, respectively, and the M-LCDR1, the M-LCDR2, and the M-LCDR3 comprise amino acid sequences of a LCDR1, a LCDR2, and a LCDR3 set forth in SEQ ID NO: 17, respectively; the M-HCDR1, the M-HCDR2, and the M-HCDR3 of the other antigen-binding moiety 1 comprise amino acid sequences of a HCDR1, a HCDR2, and a HCDR3 set forth in SEQ ID NO: 12, respectively, and the M-LCDR1, the M-LCDR2, and the M-LCDR3 comprise amino acid sequences of a LCDR1, a LCDR2, and a LCDR3 set forth in SEQ ID NO: 13, respectively. In some embodiments, the M-HCDR1, M-HCDR2, M-HCDR3, M-LCDR1, M-LCDR2, M-LCDR3, E-HCDR1, E-HCDR2, E-HCDR3, E-LCDR1, E-LCDR2, and E-LCDR3 are defined according to the Kabat, IMGT, Chothia, AbM, or Contact numbering scheme.

In some embodiments, the antigen-binding molecule according to any one of the embodiments described above has one or more of the following functions:

a. high affinity for HGFR and/or EGFR. In some embodiments, the antigen-binding molecule binds to human HGFR or EGFR with a KD value of less than 1.0E-08 M (e.g., less than 8.5E-09 M, less than 7.5E-09 M, less than 2.5E-09 M, or less). The KD value is determined by the Biacore method, and the specific test method is described in Test Example 1;

b. specific binding activity *in vitro* against cells expressing HGFR and/or EGFR, and the specific test method being described in Test Example 2;

c. blocking the binding of EGF to EGFR. In some embodiments, the binding of EGF to EGFR is blocked with an $IC_{50}$ of less than 2.7 μg/mL, and the specific test method is described in Test Example 3;

d. blocking the binding of HGF to HGFR. In some embodiments, the binding of HGF to HGFR is blocked with a maximum blocking force of greater than 90%, and the specific test method is described in Test Example 4;

e. inhibiting cellular EGFR phosphorylation, and the specific test method being described in Test Example 5;

f. inhibiting cellular HGFR phosphorylation, and the specific test method being described in Test Example 6;

g. inhibiting cellular AKT phosphorylation, and the specific test method being described in Test Example 7;

h. inhibiting the proliferation of SNU-5 cells, and the specific test method being described in Test Example 9;

i. ADCC killing against tumor cells, and the specific test method being described in Test Example 10; or

j. inhibiting the growth of tumor cells *in vivo*, and the specific test method being described in Test Example 11 or 12.

In some embodiments, the antigen-binding molecule according to any one of the embodiments described above is a low-fucosylated antigen-binding molecule; preferably, the low-fucosylated antigen-binding molecule is an antibody that is at least 80%, 85%, 90%, 95%, or 100% unmodified by fucosylation; more preferably, a fucosylation modification in the antigen-binding molecule is undetectable.

In another aspect, the present disclosure provides a pharmaceutical composition comprising: a therapeutically effective amount (or a prophylactically effective amount) of the antigen-binding molecule according to any one of the embodiments described above, and one or more pharmaceutically acceptable carriers, diluents, buffers, or excipients.

In some embodiments, the pharmaceutical composition further comprises at least one second therapeutic agent.

In another aspect, the present disclosure further provides an isolated nucleic acid encoding the antigen-binding molecule according to any one of the embodiments described above.

In another aspect, the present disclosure further provides a host cell comprising the aforementioned nucleic acid. In some embodiments, the host cell may be selected from the group consisting of a prokaryotic cell and a eukaryotic cell, preferably a eukaryotic cell, more preferably a mammalian cell, preferably a mammalian cell not including a human mammalian cell, wherein the mammalian cell includes, but is not limited to CHO, 293, NSO, and a cell in which gene editing may be performed to alter the glycosylation modification of an antibody or an antigen-binding fragment thereof, thereby altering the ADCC function of the antigen-binding molecule (e.g., the antibody), e.g., knocking out genes such as Fut8 or GnT-III to regulate the glycosylation modification.

The host cell in the present disclosure cannot develop into a whole plant or animal individual.

In another aspect, the present disclosure further provides a method for treating or preventing a disease, comprising the step of administering to a subject the antigen-binding molecule or the pharmaceutical composition according to any one of the embodiments described above. In some embodiments, the method comprises administering to a subject in need thereof a therapeutically effective amount (or a prophylactically effective amount) of the antigen-binding molecule according to any one of the embodiments described above.

In another aspect, the present disclosure further provides use of the antigen-binding molecule or the pharmaceutical composition according to any one of the embodiments described above in the preparation of a medicament for treating or preventing a disease.

In another aspect, the present disclosure further provides the antigen-binding molecule or the composition according to any one of the embodiments described above for use as a medicament. The medicament is used for treating or preventing a disease.

In some embodiments, the disease according to any one of the embodiments described above is a tumor. In some embodiments, the tumor is selected from the group consisting of lung cancer (including non-small cell lung cancer and small cell lung cancer), breast cancer, pancreatic cancer, colorectal cancer (including colon cancer and rectal cancer), sarcoma, renal cell carcinoma, hepatocellular carcinoma, gastric cancer, ovarian cancer, bladder cancer, head and neck cancer, and glioblastoma. In some embodiments, the tumor is selected from the group consisting of lung cancer, breast cancer, pancreatic cancer, colon cancer, head and neck cancer, gastric cancer, and glioblastoma.

In some specific embodiments, the lung cancer is non-small cell lung cancer.

In some specific embodiments, the lung cancer is metastatic non-small cell lung cancer.

In some specific embodiments, the lung cancer is small cell lung cancer.

In some specific embodiments, the lung cancer is human lung adenocarcinoma.

In some specific embodiments, the lung cancer is gastric cancer.

In some specific embodiments, the tumor is an EGFR- and/or HGFR-associated tumor.

## BRIEF DESCRIPTION OF THE DRAWINGS

FIG. 1 is a structural schematic diagram of Format 1.

FIG. 2 is a structural schematic diagram of Format 2.

FIG. 3 is a graph showing the experimental results for the binding activity of antibodies to EGFR CHO-S cells, wherein the ordinate of the graph refers to the mean fluorescence intensity (abbreviated as MFI, the same applies hereinafter).

FIG. 4 is a graph showing the experimental results for the binding activity of the antibodies to HGFR CHO-S cells.

FIG. 5 is a graph showing the experimental results for the binding activity of the antibodies to H1975-HGF cells.

FIG. 6 is a graph showing the experimental results for the binding activity of the antibodies to MKN-45 cells.

FIG. 7 shows the experimental results for the inhibition of cellular EGFR phosphorylation by the antibodies.

FIG. 8 shows the experimental results for the inhibition of cellular HGFR phosphorylation by the antibodies.

FIG. 9 shows the experimental results for the inhibition of cellular AKT phosphorylation by the antibodies.

FIG. 10 shows the experimental results for the reduction of HGFR on cell surfaces by the antibodies.

FIG. 11 shows the inhibition of proliferation of SNU-5 cells by the antibodies.

FIG. 12 shows the experimental results for the ADCC killing of the antibodies against Hs746T cells.

FIG. 13 shows the experimental results for the ADCC killing of the antibodies against H292 cells.

FIG. 14 shows the inhibition of the growth of HCC827 mouse tumor cells by the antibodies.

## DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be construed in an inclusive sense as opposed to an exclusive or exhaustive sense; that is to say, in the sense of "including, but not limited to". Unless otherwise indicated, "comprise" includes "consist of". For example, for an M-HCDR1 comprising an amino acid sequence set forth in SEQ ID NO: 30, it specifically comprises HGFR-HCDR1 comprising the amino acid sequence set forth in SEQ ID NO: 30. The three-letter and single-letter codes used in the present disclosure for amino acids are as described in J. Biol. Chem, 243, p3558 (1968).

The term "and/or", e.g., "X and/or Y" should be understood to mean "X and Y" or "X or Y" and should be used to provide explicit support for both meanings or either meaning.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions, deletions, insertions and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. Methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antigen-binding molecule" is used in the broadest sense and encompasses a variety of molecules that specifically bind to an antigen, including but not limited to antibodies, other polypeptides having antigen-binding activity, and antibody fusion proteins in which the two are fused. Illustratively, the antigen-binding molecules herein are bispecific antigen-binding molecules (e.g., bispecific antibodies). The term "bispecific antigen-binding molecule" refers to an antigen-binding molecule capable of specifically binding to two different antigens or at least two different epitopes of the same antigen.

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), so long as they exhibit the desired antigen-binding activity. "Natural antibody" refers to a naturally-occurring immunoglobulin molecule. For example, a native IgG antibody is a heterotetrameric protein of about 150,000 Daltons composed of two light chains and two heavy chains linked by a disulfide bond. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules. The bispecific antibodies can be classified into bivalent, trivalent, tetravalent, or higher-valent bispecific antibodies according to the number of the antigen-binding regions. The bispecific antibodies can be classified into symmetric bispecific antibodies and asymmetric bispecific antibodies according to whether their structures are symmetric or asymmetric. Among them, the fragment-type bispecific antibodies, such as Fab fragments lacking Fc fragments, formed by linking 2 or more Fab fragments in one molecule, have relatively low immunogenicity, small molecular weight, and relatively high tumor tissue permeability, and typical antibody structures of this type include $F(ab)_2$, scFv-Fab, $(scFv)_2$-Fab, and the like. The IgG-like bispecific antibodies (e.g., antibodies having Fc fragments) have large relative molecular weight, and the Fc fragments facilitate the purification of the antibodies and increase their solubility and stability, and the Fc fragments may further bind to the receptor FcRn and increase the serum half-life of the antibodies. Typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, $F(ab)_2$-CrossMAb, IgG-$(scFv)_2$, Bs4Ab, DVD-Ig, Tetravalent-DART-Fc, $(scFv)_4$-Fc, CODV-Ig, mAb2, and $F(ab)_4$-CrossMAb (see Aran F. Labrijn et al., Nature Reviews Drug Discovery, volume 18, pages 585-608 (2019); Chen S1 et al., J Immunol Res., Feb. 11, 2019; 2019: 4516041).

The term "variable region" or "variable domain" refers to a domain in an antigen-binding molecule that binds to an antigen. Herein, a heavy chain variable region in the antigen-binding moiety 1 specifically binding to HGFR is designated M-VH, and a light chain variable region is designated M-VL; a heavy chain variable region in the antigen-binding moiety 2 specifically binding to EGFR is designated E-VH, and a light chain variable region is designated E-VL. VH and VL each comprise four conserved framework regions (FRs) and three complementarity determining regions (CDRs). The term "complementarity determining region" or "CDR" refers to a region in the variable domain that primarily contributes to antigen-binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Herein, the 3 CDR regions in the M-VH are designated M-HCDR1, M-HCDR2, and M-HCDR3, respectively; the 3 CDR regions in the M-VL are designated M-LCDR1, M-LCDR2, and M-LCDR3, respectively; the 3 CDR regions in the E-VH are designated E-HCDR1, E-HCDR2, and E-HCDR3, respectively; the 3 CDR regions in the E-VL are designated E-LCDR1, E-LCDR2, and E-LCDR3, respectively. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships among the various numbering schemes are well known to those skilled in the art. The numbering schemes of the present disclosure are shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in examples of the present disclosure. Although the Kabat numbering scheme is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, $F(ab')_2$, single-domain antibodies, single-chain Fab (scFab), diabodies, linear antibodies, single-chain antibody molecules (e.g., scFv), and multispecific antibodies formed from antibody fragments.

A Fab refers to a protein consisting of VH and CH1 (Fab heavy chain) and VL and CL (Fab light chain) of an immunoglobulin.

An Fv refers to an antigen-binding domain consisting of VH and VL of an immunoglobulin.

In some embodiments, the first Fab has a structure of a Fab in the present disclosure. In a substituted Fab, the CH1 and CL are replaced by a Titin chain or an Obscurin chain.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise indicated, the numbering scheme for the Fc region is the EU index.

The term "Titin chain" refers to a fragment of 78-118 amino acids in length comprising a peptide fragment of a Titin Ig-like 152 domain or a functional variant thereof in a Titin protein. The Titin chain is capable of forming a dimerized complex with the Obscurin chain.

The term "Obscurin chain" refers to a fragment of 87-117 amino acids in length comprising a peptide fragment of an Obscurin Ig-like 1 domain or a functional variant thereof in an Obscurin protein; or a fragment of 78-118 amino acids in length comprising a peptide fragment of an Obscurin-like Ig-like 1 domain or a functional variant thereof in an Obscurin-like 1 protein. The Obscurin chain is capable of binding to the Titin chain to form a dimerized complex. The Titin chain and the Obscurin chain of the present disclosure can be used to substitute the CH1 and CL in the Fab to form a substituted Fab (Fab-S). This substitution does not affect the binding of an antigen-binding molecule to an antigen.

The term "chimeric" antibody refers to an antibody in which a portion of the heavy and/or light chain in the antibody is derived from a particular source or species, while the remainder of the heavy and/or light chain is derived from different source or species.

The term "humanized" antibody refers to an antibody that retains the reactivity of a non-human antibody while having low immunogenicity in humans. For example, the humanization can be achieved by retaining the non-human CDR regions and substituting the remainder of the antibody with its human counterparts (i.e., the constant regions and the framework region portion of the variable regions).

The term "affinity" refers to the overall strength of a non-covalent interaction between a single binding site of a molecule (e.g., the antigen-binding molecule of the present disclosure) and its binding partner (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the equilibrium dissociation constant, which is obtained from the ratio of kd to ka (i.e., kd/ka) and expressed as molar concentration (M). The KD values of antibodies can be determined by methods known in the art, for example, methods for determining antibody KD include measuring surface plasmon resonance using a biosensing system, such as a system, or measuring affinity in solution by solution equilibrium titration (SET) assay.

The term "effector function" refers to those biological activities attributable to the Fc region of an antibody (either the natural sequence Fc region or the amino acid sequence variant Fc region) and which vary with the antibody isotype. Examples of effector functions of an antibody include: C1q binding and complement-dependent cytotoxicity; Fc receptor binding; antibody-dependent cell-mediated cytotoxicity (ADCC); phagocytosis; down-regulation of receptors on cell surfaces (e.g., B cell receptors); and B cell activation.

The term "monoclonal antibody" refers to a population of substantially homogeneous antibodies or a member thereof, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or a portion of a molecule that is capable of being selectively bound by an antigen-binding molecule (e.g., an antibody). An antigen may have one or more epitopes capable of interacting with different antigen-binding molecules (e.g., antibodies).

The term "epitope" refers to an area or region on an antigen that is capable of specifically binding to an antibody or an antigen-binding fragment thereof. Epitopes can be formed by contiguous amino acids (linear epitopes) or comprise non-contiguous amino acids (conformational epitopes), e.g., non-contiguous amino acids that are in spatial proximity to each other due to folding of the antigen (i.e., by tertiary folding of the antigen). The difference between the conformational epitope and the linear epitope is that in the presence of denaturing solvents, the binding of the antibody to the conformational epitope is lost. An epitope generally comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of specifically binding", "specifically binding", or "binding" means that an antibody is capable of binding to a certain antigen or an epitope thereof with higher affinity than to other antigens or epitopes. Generally, an antibody binds to an antigen or epitope thereof with an equilibrium dissociation constant (KD) of about $1 \times 10^{-7}$ M or less (e.g., about $1 \times 10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a FACS or BIACORE® surface plasmon resonance assay. However, an antibody that specifically binds to an antigen or an epitope thereof may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "antigen-binding moiety" refers to a polypeptide molecule specifically binding to a target antigen. Antigen-binding moieties include the antibodies and the fragments thereof described herein. A specific antigen-binding moiety includes an antigen-binding domain of an antibody, which comprises an antibody heavy chain variable region and an antibody light chain variable region. The term "antigen-binding moiety specifically binding to HGFR" refers to a moiety that is capable of binding to HGFR or an epitope thereof with sufficient affinity. In certain embodiments, the antigen-binding moiety specifically binding to HGFR has an equilibrium dissociation constant (KD) as follows: < about 1 μM, < about 100 nM, or < about 10 nM or less, as determined by the Biacore method. In certain embodiments, the antigen-binding moiety specifically binding to HGFR binds to a conserved epitope in HGFR from different species. The term "antigen-binding moiety specifically binding to EGFR" refers to a moiety that is capable of binding to EGFR or an epitope thereof with sufficient affinity, such that a molecule containing the moiety can be used as a diagnostic agent and/or therapeutic agent targeting EGFR.

In certain embodiments, the antigen-binding moiety specifically binding to EGFR has an equilibrium dissociation constant (KD) as follows: < about 1 μM, < about 100 nM, or < about 10 nM or less, as determined by the Biacore method. In certain embodiments, the antigen-binding moiety specifically binding to EGFR binds to a conserved epitope in EGFR from different species. Antigen-binding moieties include antibody fragments as defined herein, e.g., a Fab, a substituted Fab, or an Fv.

In this context, ordinal numerals in "antigen-binding moiety 1", "antigen-binding moiety 2", "linker 1", and "linker 2" are used for the purpose of only distinguishing different technical features and chemical entities without limiting any order, level, and quantity.

The term "linker" refers to a linker unit that links two polypeptide fragments. Herein, the linkers present in the same structural formula or different structural formulas may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond. When the term "bond" appears in a structural unit, it means that the units on both sides of the unit are directly linked.

"Tm" is the temperature of dissolution and denaturation (e.g., as determined by endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value.

"Tonset" is the onset temperature of denaturation. It refers to the temperature at which the protein begins to denature, i.e., the temperature at which the fluorescence value begins to change. "Tagg" is the onset temperature of aggregation. The temperature at which the sample begins to aggregate is monitored by detecting aggregates at two wavelengths of 266 nm and 473 nm by static light scattering. Tagg266 refers to the onset temperature of aggregation monitored at 266 nm.

The term "antibody-dependent cellular cytotoxicity", "antibody-dependent cell-mediated cytotoxicity", or "ADCC" is a mechanism for inducing cell death, which depends on the interaction of antibody-coated target cells with effector cells having lytic activity, such as natural killer (NK) cells, monocytes, macrophages, and neutrophils, via an Fcγ receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of an antibody bound on a target cell binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "fucosylation", "fucosylated" or "fucosylation modification" refers to the presence of a fucose residue within an oligosaccharide attached to a peptide chain of an antigen-binding molecule (e.g., an antibody). Fucosylation is a process of post-translational modification of glycoproteins. Representative enzyme genes related to core fucosylation include *GMD* (GDP-mannose 4,6-dehydratase) gene and *Fut8* (FUT8, fut8, $\alpha$-1,6-fucosyltransferase) gene. The core fucosylation level can be effectively regulated by inhibiting the expression of the two genes or constructing a Fut8-knockout CHO host cell (YAMANE-OHNUKI et al., "Establishment of FUT8knockout Chinese hamster ovary cells:an ideal host cellline for producing completely defucosylated antibodies with enhancedantibody-dependent cellular cytotoxicity.", BIOTECHNOLOGY AND BIOENGINEERING, 2004, p614-622). Generally, the fucosylation is located in the N-oligosaccharides of the Fc region of an antibody that comprises $\alpha$-1,6-fucose at a core N-acetylglucosamine (GlcNAc) residue (e.g., position Asn297 of human IgG1 Fc (corresponding to the EU numbering scheme)).

"Low-fucosylated" means that the proportion of antigen-binding molecules (e.g., antibodies) with fucosylation modifications in the population of antigen-binding molecules (e.g., population of antibodies) is not higher than a predetermined value, e.g., not higher than 20%, not higher than 15%, not higher than 10%, not higher than 5%, not higher than 4%, not higher than 3%, not higher than 2%, not higher than% 1, not higher than 0.5%, or not higher than 0.1%.

"Undetectable fucosylation", "non-fucosylated", or "afucosylated" means that the carbohydrate structure attached to the Fc region lacks the fucosylation modification. The skilled person should understand that "none" and "lack" in the present disclosure are not to be interpreted as "completely absent" but that the fucosylation modification is "undetectable" by the oligosaccharide determination methods known in the art.

The level of fucosylation of an antigen-binding molecule (e.g., an antibody) can be determined by determining all oligosaccharides by methods known in the art to determine the percentage of fucosylated oligosaccharides. Methods known in the art for determining fucosylation include, but are not limited to, gel electrophoresis, liquid chromatography, mass spectrometry, and the like. For example, the level of fucosylation of an antibody is determined by hydrophilic interaction chromatography (or hydrophilic interaction liquid chromatography, HILIC), for example by denaturing a sample with peptide-N-glycanase F to cleave N-linked glycans, and then analyzing N-linked glycans for fucose content.

In the present disclosure, in some embodiments, the low-fucosylated antibody of the present disclosure is an antibody with at least 80% of heavy chains not modified by fucosylation, e.g., with at least 80%-95%, 90%-95%, 95%-100%, 85%, 90%, 91%, 92%, 93%, 94%, 95%, 96%, 97%, 98%, 99% or 100% of heavy chains not modified by fucosylation. In the present disclosure, "non-fucosylated" refers to an antigen-binding molecule (e.g., an antibody) with 100% of heavy chain not modified by fucosylation, unless otherwise stated.

Low-fucosylated or non-fucosylated antigen-binding molecules (e.g., antibodies) can be prepared by methods well known in the art. For example, they can be prepared by addition, translocation, or deletion of one or more carbohydrate moieties present in the antibody, for example, by cleavage of the fucose residue of the antibody using fucosidase (see Tarentino et al., (1975) Biochem. 14: 5516). Low-fucosylated antibodies can be prepared by changing the level of glycosylation by altering the composition of glycosylation, for example, by modifying the glycan moiety attached to each Fc fragment at the N297 residue (Natsume et al., (2009) Drug Des. Devel. Ther. 3: 7). They can also be prepared without altering the antibody sequence, for example, by expressing a low-fucosylated or non-fucosylated antibody by cells that alter the glycosylation pattern of the antibody, including, for example, glycosylation engineered cells that have been genetically engineered (see, e.g., Hse et al., (1997) J. Biol. Chem. 272: 9062-9070; Yang et al., (2015) Nature Biotechnology 33, 842-844). Various glycosylation engineered cells have been disclosed in the art, for example, cell lines Ms704, Ms705, and Ms709 lacking the fucosyltransferase gene (*Fut8* ($\alpha$-(1,6)fucosyltransferase)) (see Yamane-Ohnuki et al., (2004) Biotechnol. Bioeng. 87: 614; US Patent No. 20040110704), a CHO cell line Lec13 with reduced ability to attach fucose to Asn (297)-linked sugars (see WO 03/035835), a rat myeloma cell line YB2/0 with little or no activity of adding fucose to N-acetylglucosamine, (which binds to the Fc region of antibodies) (see EP 1176195), and plant cells for the production of antibodies with modified glycosylation patterns (see US Patent No. US20120276086). In addition, cells carrying recombinant genes encoding an enzyme that uses GDP-6-deoxy-D-lyxo-4-hexose as a substrate, such as GDP-6-deoxy-D-lyxo-4-hexose reductase (RMD), can also produce low-fucosylated or non-fucosylated antibodies (see US Patent No. US8642292).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated nucleic acid" refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding the antigen-binding molecule refers to one or more nucleic acid molecules encoding the heavy chain and light chain of an antibody (or a fragment thereof) and includes one or more nucleic acid molecules in a single vector or separate vectors, or present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned; when in the process of the optimal alignment, gaps are allowed to be introduced as necessary to achieve the maximum percent sequence identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be completely identical to parent cells in terms of nucleic acid content and may contain mutations. As used herein, the term includes mutant progenies that have the same function or biological activity as the cells screened or selected from the primary transformed cells. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica, Pichia, Saccharomycescerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens, Neurospora crassa, Pichia*, any *Saccharomyces, Hansenula polymorpha*, any *Kluyveromyces, Candida albicans*, any *Aspergillus, Trichoderma reesei, Chrysosporium lucknowense*, any *Fusarium, Yarrowia lipolytica*, and *Neurospora crassa*. "Optional" or "optionally" means that the feature subsequently described may, but not necessarily, occur, and indicates the conditions that include the occurrence or non-occurrence of the feature.

The term "pharmaceutical composition" refers to a mixture comprising one or more of the antibodies or antigen-binding molecules described herein and other chemical components, for example, physiological/pharmaceutically acceptable carriers and excipients.

The term "pharmaceutically acceptable carrier (vehicle), diluent, buffer, or excipient" refers to an ingredient in a pharmaceutical formulation that is different from the active ingredient and is not toxic to the subject.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to *Macaca fascicularis.* In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection isolated from a subject, such as fluids, cells, or tissues, as well as fluids, cells, or tissues present in a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura; pericardium; peritoneum; fluids from abdominal cavity and other body cavities; fluids collected from bronchial lavage; synovial fluids; liquid solutions in contact with a subject or biological source, e.g., culture media (including conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention applied to the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antigen-binding molecule of the present disclosure is used to delay the development of or slow the progression of disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some embodiments, the effective amount is a therapeutically effective amount or a prophylactically effective amount.

"Therapeutically effective amount" is an amount sufficient to treat a disease state or condition, particularly a state or condition associated with the disease state, or to otherwise hinder, delay, or reverse the progression of the disease state or any other undesirable symptoms associated with the disease.

"Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

**Target Molecule**

"HGFR" should be understood in the broadest sense and is intended to encompass various forms of molecules of HGFR in various stages in mammals, such as, but not limited to, molecules produced by the HGFR gene during amplification, replication, transcription, splicing, processing, translation, and modification, e.g., precursor HGFR, mature HGFR, HGFR expressed on the membrane, HGFR splice variants, modified HGFR, or fragments thereof; the term also encompasses HGFR artificially prepared or expressed *in vitro.*

"EGFR" should be understood in the broadest sense and is intended to encompass various forms of molecules of EGFR in various stages in mammals, such as, but not limited to, molecules produced by the EGFR gene during amplification, replication, transcription, splicing, processing, translation, and modification, e.g., precursor EGFR, mature EGFR, EGFR expressed on the membrane, EGFR splice variants, modified EGFR, or fragments thereof; the term also encompasses EGFR artificially prepared or expressed *in vitro.*

**Antigen-Binding Molecules of the Present Disclosure**

The present disclosure provides antigen-binding molecules that have a number of advantageous properties, such as affinity, specific binding to HGFR and EGFR on cell surfaces, blocking the binding of EGF to EGFR, blocking the binding of HGF to HGFR, inhibiting cellular EGFR phosphorylation, inhibiting cellular HGFR phosphorylation, inhibiting cellular AKT phosphorylation, inhibiting proliferation of SNU-5 cells, ADCC killing against tumor cells, therapeutic activity, safety (e.g., lower cytokine release), pharmacokinetic properties, and/or druggability (e.g., yield, purity, stability, and the like).

**Exemplary Antigen-Binding Molecules**

In one aspect, the present disclosure provides an antigen-binding molecule comprising at least one antigen-binding moiety 1 specifically binding to HGFR and at least one antigen-binding moiety 2 specifically binding to EGFR. Particularly, the antigen-binding molecule of the present disclosure has one or more of the following functions:

a. high affinity for HGFR and/or EGFR. In some embodiments, the antigen-binding molecule binds to human HGFR or EGFR with a KD value of less than 1.0E-08 M (e.g., less than 8.5E-09 M, less than 7.5E-09 M, less than 2.5E-09 M, or less). The KD value is determined by the Biacore method, and the specific test method is described in Test Example 1;

b. specific binding activity *in vitro* against cells expressing HGFR and/or EGFR, and the specific test method being described in Test Example 2;

c. blocking the binding of EGF to EGFR. In some embodiments, the binding of EGF to EGFR is blocked with an IC50 of less than 2.7 $\mu$g/mL, and the specific test method is described in Test Example 3;

d. blocking the binding of HGF to HGFR. In some embodiments, the binding of HGF to HGFR is blocked with a maximum blocking force of greater than 90%, and the specific test method is described in Test Example 4;

e. inhibiting cellular EGFR phosphorylation, and the specific test method being described in Test Example 5;

f. inhibiting cellular HGFR phosphorylation, and the specific test method being described in Test Example 6;

g. inhibiting cellular AKT phosphorylation, and the specific test method being described in Test Example 7;

h. inhibiting the proliferation of SNU-5 cells, and the specific test method being described in Test Example 9;

i. ADCC killing against tumor cells, and the specific test method being described in Test Example 10; or

j. inhibiting the growth of tumor cells *in vivo,* and the specific test method being described in Test Example 11 or 12.

In one aspect, the present disclosure provides an antigen-binding molecule comprising at least one antigen-binding moiety 1 specifically binding to HGFR and at least one antigen-binding moiety 2 specifically binding to EGFR, wherein the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3; the antigen-binding moiety 2 comprises a heavy chain variable region E-VH and a light chain variable region E-VL, wherein the E-VH comprises an E-HCDR1, an E-HCDR2, and an E-HCDR3, ant the E-VL comprises an E-LCDR1, an E-LCDR2, and an E-LCDR3; wherein

in the antigen-binding moiety 1 specifically binding to HGFR, (i) the M-HCDR1 is set forth in SEQ ID NO: 30, the M-HCDR2 is set forth in SEQ ID NO: 31, the M-HCDR3 is set forth in SEQ ID NO: 32, the M-LCDR1 is set forth in SEQ ID NO: 27, the M-LCDR2 is set forth in SEQ ID NO: 33, and the M-LCDR3 is set forth in SEQ ID NO: 29; or (ii) the M-HCDR1 is set forth in SEQ ID NO: 18, the M-HCDR2 is set forth in SEQ ID NO: 19, the M-HCDR3 is set forth in SEQ ID NO: 20, the M-LCDR1 is set forth in SEQ ID NO: 21, the M-LCDR2 is set forth in SEQ ID NO: 22, and the M-LCDR3 is set forth in SEQ ID NO: 23; or (iii) the M-HCDR1 is set forth in SEQ ID NO: 24, the M-HCDR2 is set forth in SEQ ID NO: 25, the M-HCDR3 is set forth in SEQ ID NO: 26, the M-LCDR1 is set forth in SEQ ID NO: 27, the M-LCDR2 is set forth in SEQ ID NO: 28, and the M-LCDR3 is set forth in SEQ ID NO: 29; and
in the antigen-binding moiety 2 specifically binding to EGFR, the E-HCDR1 is set forth in SEQ ID NO: 6, the E-HCDR2 is set forth in SEQ ID NO: 7, the E-HCDR3 is set forth in SEQ ID NO: 8, the E-LCDR1 is set forth in SEQ ID NO: 9, the E-LCDR2 is set forth in SEQ ID NO: 10, and the E-LCDR3 is set forth in SEQ ID NO: 11.

In some embodiments, the antigen-binding molecule comprises two antigen-binding moieties 1 specifically binding to HGFR, wherein in one of the antigen-binding moieties 1, an M-VH thereof has: an M-HCDR1 set forth in SEQ ID NO: 30, an M-HCDR2 set forth in SEQ ID NO: 31, and an M-HCDR3 set forth in SEQ ID NO: 32, and an M-VL thereof has: an M-LCDR1 set forth in SEQ ID NO: 27, an M-LCDR2 set forth in SEQ ID NO: 33, and an M-LCDR3 set forth in SEQ ID NO: 29; in the other antigen-binding moiety 1, an M-VH thereof has: an M-HCDR1 set forth in SEQ ID NO: 18, an M-HCDR2 set forth in SEQ ID NO: 19, and an M-HCDR3 set forth in SEQ ID NO: 20, and an M-VL thereof has: an M-LCDR1 set forth in SEQ ID NO: 21, an M-LCDR2 set forth in SEQ ID NO: 22, and an M-LCDR3 set forth in SEQ ID NO: 23; and in the antigen-binding moiety 2 specifically binding to EGFR, an E-VH thereof has: an E-HCDR1 set forth in SEQ ID NO: 6, an E-HCDR2 set forth in SEQ ID NO: 7, and an E-HCDR3 set forth in SEQ ID NO: 8, and an E-VL thereof has: an E-LCDR1 set forth in SEQ ID NO: 9, an E-LCDR2 set forth in SEQ ID NO: 10, and an E-LCDR3 set forth in SEQ ID NO: 11; or

in some embodiments, in the antigen-binding moiety 1 specifically binding to HGFR, an M-VH thereof has: an M-HCDR1 set forth in SEQ ID NO: 18, an M-HCDR2 set forth in SEQ ID NO: 19, and an M-HCDR3 set forth in SEQ ID NO: 20, and an M-VL thereof has: an M-LCDR1 set forth in SEQ ID NO: 21, an M-LCDR2 set forth in SEQ ID NO: 22, and an M-LCDR3 set forth in SEQ ID NO: 23; and
in the antigen-binding moiety 2 specifically binding to EGFR, an E-VH thereof has: an E-HCDR1 set forth in SEQ ID NO: 6, an E-HCDR2 set forth in SEQ ID NO: 7, and an E-HCDR3 set forth in SEQ ID NO: 8, and an E-VL thereof has: an E-LCDR1 set forth in SEQ ID NO: 9, an E-LCDR2 set forth in SEQ ID NO: 10, and an E-LCDR3 set forth in SEQ ID NO: 11.

In some embodiments, the M-VH is set forth in SEQ ID NO: 16 and the M-VL is set forth in SEQ ID NO: 17, or the M-VH is set forth in SEQ ID NO: 12 and the M-VL is set forth in SEQ ID NO: 13, or the M-VH is set forth in SEQ ID NO: 14 and the M-VL is set forth in SEQ ID NO: 15; and/or (ii) the E-VH is set forth in SEQ ID NO: 3 and the E-VL is set forth in SEQ ID NO: 5 or SEQ ID NO: 4.

In some embodiments, provided is the antigen-binding molecule according to any one of the embodiments described above, wherein:

(i) in the antigen-binding molecule such as two antigen-binding moieties 1 specifically binding to HGFR, the M-VH of one of the antigen-binding moieties 1 is set forth in SEQ ID NO: 16, and the M-VL is set forth in SEQ ID NO: 17; the M-VH of the other antigen-binding moiety 1 is set forth in SEQ ID NO: 12, and the M-VL is set forth in SEQ ID NO: 13; and the E-VH is set forth in SEQ ID NO: 3, and the E-VL is set forth in SEQ ID NO: 5; or
(ii) in the antigen-binding molecule such as at least one antigen-binding moiety 1 specifically binding to HGFR and at least one antigen-binding moiety 2 specifically binding to EGFR, the E-VH is set forth in SEQ ID NO: 3, the E-VL is set forth in SEQ ID NO: 5, the M-VH is set forth in SEQ ID NO: 12, and the M-VL is set forth in SEQ ID NO: 13.

In some embodiments, the antigen-binding molecule according to any one of the embodiments described above further comprises an Fc region, wherein the Fc region comprises a first subunit Fc1 and a second subunit Fc2 capable of associating with each other. In some embodiments, the Fc region is an IgG Fc region, particularly an IgG$_1$ Fc region.

In some embodiments, the antigen-binding molecule according to any one of the embodiments described above has: a first chain having a structure of formula (a), a second chain having a structure of formula (b), a third chain having a structure of formula (c), and a fourth chain having a structure of formula (d), wherein

formula (a) is [E-VH]-[linker 1]-[M-VH]-[CH1]-[Fc1],
formula (b) is [E-VL]-[linker 2]-[M-VL]-[CL],
formula (c) is [M-VH]-[linker 3]-[Titin chain]-[Fc2], and
formula (d) is [M-VL]-[linker 4]-[Obscurin chain].

In some embodiments, the antigen-binding molecule has: a first chain set forth in SEQ ID NO: 34, a second chain set forth in SEQ ID NO: 35, a third chain set forth in SEQ ID NO: 36, and a fourth chain set forth in SEQ ID NO: 37.
In some embodiments, the antigen-binding molecule according to any one of the embodiments described above has: a first chain having a structure of formula (e), a second chain having a structure of formula (f), a third chain having a structure of formula (g), and a fourth chain having a structure of formula (h), wherein

formula (e) is [M-VH]-[CH1]-[Fc1],
formula (f) is [M-VL]-[CL],
formula (g) is [E-VH]-[linker 1]-[Titin chain]-[Fc2], and
formula (h) is [E-VL]-[linker 2]-[Obscurin chain].

In some specific embodiments, the antigen-binding molecule has: a first chain set forth in SEQ ID NO: 38, a second chain set forth in SEQ ID NO: 39, a third chain set forth in SEQ ID NO: 40, and a fourth chain set forth in SEQ ID NO: 41.

**Variants of Antigen-Binding Molecules**

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to arrive at the final construct, provided that the final construct possesses the desired characteristics, e.g., antigen-binding.

**Variants Comprising Substitutions, Insertions and Deletions**

In certain embodiments, antigen-binding molecule variants having one or more amino acid substitutions are provided. Sites of interest include CDRs and FRs. Conservative substitutions are shown in Table 2 under the heading of "Preferred substitution". Exemplary substitutions are provided in Table 2 under the heading of "Exemplary substitution", and as further described below with reference to amino acid side chain classes. Amino acid substitutions can be introduced into an antibody of interest and the products are screened for a desired activity, e.g., retained/improved antigen-binding, reduced immunogenicity, or improved ADCC or CDC.

Table 2. Amino acid substitutions

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |

(continued)

| Original residue | Exemplary substitution | Preferred substitution |
|---|---|---|
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

According to common side-chain properties, amino acids can be grouped as follows:

(1) hydrophobic: Nle, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions are those in which a member of one class substitutes a member of another class.
One type of substitutional variant involves substituting one or more CDR residues of a parent antibody (e.g., a humanized or human antibody). Generally, the resulting variant selected for further study will have changes (e.g., improvements) in certain biological properties (e.g., increased affinity, reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen-binding can be specifically identified, for example, using alanine scanning mutagenesis or modeling. Particularly, HCDR3 and LCDR3 are often targeted.
In certain embodiments, substitutions, insertions, or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. Such changes do not occur at antigen-contacting residues. In certain embodiments of the variant VH and VL sequences provided above, each CDR is unchanged, or contains no more than 1, 2 or 3 amino acid substitutions.
A useful method for identifying residues or regions of an antibody that can be used as mutagenesis targets is called "alanine scanning mutagenesis". In this method, a residue or group of residues (e.g., charged residues such as Arg, Asp, His, Lys, and Glu) are identified and substituted with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: amino- and/or carboxy-terminal fusions to 1 residue or polypeptides of 100 or more residues in length, and intrasequence insertions of single or multiple amino acid residues. Examples of terminal insertions include an antibody with an N-terminal methionyl residue. Other insertional variants of the antibody molecule include the fusion of the N- or C-terminus of the antibody fused to an enzyme (or a polypeptide that increases the serum half-life of the antibody).

**Engineering of Fab**

In one aspect, in the antigen-binding molecule of the present disclosure, the antigen-binding moiety specifically binding to HGFR is a Fab, and the antigen-binding moiety specifically binding to EGFR is a substituted Fab comprising a heavy chain variable region, a light chain variable region, a Titin chain, and an Obscurin chain. In the substituted Fab, the original CH1 and CL of the Fab are substituted with the Titin chain and the Obscurin chain.

In another aspect, in the antigen-binding molecule of the present disclosure, the antigen-binding moiety specifically binding to EGFR is a Fab, and the antigen-binding moiety specifically binding to HGFR is a substituted Fab comprising a heavy chain variable region, a light chain variable region, a Titin chain, and an Obscurin chain. In the substituted Fab, the original CH1 and CL of the Fab are substituted with the Titin chain and the Obscurin chain.

**Engineering of Fc Region**

In one aspect, the Fc region of the antigen-binding molecule of the present disclosure comprises one or more amino acid substitutions that reduce its binding to an Fc receptor, e.g., its binding to an Fcγ receptor, and reduce or eliminate effector functions. A natural IgG Fc region, specifically an $IgG_1$ Fc region or an $IgG_4$ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows above 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcγRI, FcγRIIa, FcγRIIB, or FcγRIIIa. In some embodiments, the engineered Fc region has reduced binding affinity for a complement (e.g., C1q) as compared to the native Fc region. In some embodiments, the engineered Fc region has no reduced binding affinity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the $IgG_1$ Fc region, amino acid residue substitutions at positions such as positions 238, 265, 269, 270, 297, 327, and 329 can reduce effector functions.

In some embodiments, the Fc region is a human $IgG_1$ Fc region, and the amino acid residues at positions 234 and 235 are A, as numbered according to the EU index. For the $IgG_4$ Fc region, amino acid residue substitutions at positions such as position 228 can reduce effector functions. The antigen-binding molecule may comprise different antigen-binding moieties fused to the two subunits of the Fc region and may result in undesired homodimerization. To improve yield and purity, it is thus advantageous to introduce modifications that promote heterodimerization in the Fc region of the antigen-binding molecule of the present disclosure. In some embodiments, the Fc region of the present disclosure comprises modifications according to the knob-into-hole (KIH) technique, which involves introducing a protuberance structure (knob) at the interface of the first subunit and a pore structure (hole) at the interface of the second subunit; or a protuberance structure (knob) at the interface of the second subunit and a pore structure (hole) at the interface of the first subunit. As such, the protuberance structure can be positioned in the pore structure, thereby promoting the formation of heterodimers and inhibiting the production of homodimers. The protuberance structure is constructed by substituting a small amino acid side chain at the interface of the first subunit with a larger side chain (e.g., tyrosine or tryptophan). The pore structure is created at the interface of the second subunit by substituting a large amino acid side chain with a smaller amino acid side chain (e.g., alanine or threonine). The protuberance and pore structures are prepared by altering the nucleic acid encoding the polypeptide. In some embodiments, the optional amino acid substitutions are shown in the table below:

Table 3. Combinations of KIH mutations

| First subunit | T366Y | T366W | T394W | F405W | T366W | T366Y F405A | T366W F405W | F405W Y407A |
|---|---|---|---|---|---|---|---|---|
| Second subunit | Y407T | Y407A | F405A | T394S | T366S L368A Y407V | T394W Y407T | T394W Y407A | T366W T394S |

In addition to the knob-into-hole technique, other techniques for modifying the CH3 domain of the heavy chain to achieve heterodimerization are also known in the art, e.g., WO 96/27011, WO 98/050431, EP 1870459, WO 2007/110205, WO 007/147901, WO 2009/089004, WO 2010/129304, WO 2011/90754, WO 2011/143545, WO 2012/058768, WO 2013/157954, and WO 013/096291.

The antigen-binding molecule may further comprise a disulfide bond engineering. For example, the first subunit of the Fc region comprises a 354C mutation and the second subunit comprises a 349C mutation, such that an engineered disulfide bond is produced between the first and second subunits of the Fc region, promoting the formation of heterodimerization of the first and second subunits of the Fc region.

The Fc region of the antigen-binding molecule may further introduce other amino acid modifications, such as mutations of allogeneic amino acid residues that reduce immunogenicity. In some embodiments, 356E and 358M mutations are introduced into the Fc of IgG1.

The C-terminus of the Fc region may be an intact C-terminus ending with amino acid residues PGK or a truncated C-terminus, e.g., a truncated C-terminus in which one or two C-terminal amino acid residues have been removed. In a preferred aspect, the C-terminus of the heavy chain is a truncated C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with or without a K447 residue and/or G446 + K447 residues.

## Recombination Method

The antigen-binding molecule may be produced using recombination methods. For these methods, one or more isolated nucleic acids encoding the antigen-binding molecule are provided. In the case of a native antibody, a native antibody fragment or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain(s) of the antibody). These nucleic acids can be on the same expression vector or different expression vectors.

In the case of a bispecific antibody with heterodimeric heavy chains, four nucleic acids are required, one for the first light chain, one for the first heavy chain comprising the first Fc-region polypeptide, one for the second light chain, and one for the second heavy chain comprising the second Fc-region polypeptide. The four nucleic acids can be contained in one or more nucleic acid molecules or expression vectors. Generally, these nucleic acids are located on two or three expression vectors, that is, one vector can comprise more than one of these nucleic acids.

In one embodiment, the present disclosure provides an isolated nucleic acid encoding the antibody as described above. Such nucleic acids may independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule, wherein the method comprises culturing a host cell comprising a nucleic acid encoding the antibody, as provided above, under conditions suitable for antibody expression, and optionally collecting the antibody from the host cell (or host cell medium).

For recombinant production of the antigen-binding molecule, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombinant methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US 5959177, US 6040498, US 6420548, US 7125978, and US 6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

**Diagnostic and Therapeutic Compositions**

In certain embodiments, the antigen-binding molecule provided in the present disclosure can be used to detect the presence of HGFR or EGFR in a biological sample. As used herein, the term "detection" encompasses quantitative or qualitative detection. In certain embodiments, the biological sample includes a cell or tissue, such as tumor tissue.

In one embodiment, an antigen-binding molecule for use in a diagnostic or detection method is provided. In yet another aspect, a method for detecting the presence/level of HGFR or EGFR in a biological sample is provided. In certain embodiments, the method comprises contacting the biological sample with an antigen-binding molecule under suitable conditions and detecting whether a complex is formed between the detection agent and the antigen. Such methods may be *in vitro* or *in vivo* methods. In one embodiment, an antigen-binding molecule is used to select a subject suitable for treatment; for example, HGFR or EGFR is a biomarker for selecting a subject.

In certain embodiments, a labeled antigen-binding molecule is provided. Labels include, but are not limited to, labels or modules that are detected directly (such as fluorescent, chromophoric, electron-dense, chemiluminescent, and radioactive labels), and modules that are detected indirectly (e.g., modules that are detected indirectly through an enzymatic reaction or molecular interaction, such as enzymes or ligands).

In an additional aspect, a pharmaceutical composition comprising the antigen-binding molecule is provided, for example, for use in any of the following treatment methods. In one aspect, the pharmaceutical composition comprises any of the antibodies provided herein and a pharmaceutically acceptable carrier. In another aspect, the pharmaceutical composition comprises any of the antibodies provided herein and at least one additional therapeutic agent. The pharmaceutical composition of the antigen-binding molecule described in the present disclosure is prepared by: mixing the antibody of desired purity with one or more optional pharmaceutically acceptable carriers. The pharmaceutical composition is in the form of a lyophilized composition or an aqueous solution. Formulations for *in vivo* administration are generally sterile. Sterility can be readily achieved, for example, by filtration through a sterile filter membrane.

**Treatment Method and Route of Administration**

Any of the antigen-binding molecules provided herein can be used in the treatment method.

In yet another aspect, the present disclosure provides use of the antigen-binding molecule in the manufacture or preparation of a medicament. In one embodiment, the medicament is used for the treatment of HGFR- and/or EGFR-associated diseases, for example, tumors such as tumors highly expressing HGFR and/or EGFR, e.g., lung cancer, breast cancer, pancreatic cancer, colon cancer, head and neck cancer, gastric cancer, or glioblastoma. And the medicament is present in an amount effective for the diseases described above.

In some embodiments, the effective amount is a unit daily dose or a unit weekly dose. In one such embodiment, the use further comprises administering to a subject an effective amount of at least one additional therapeutic agent (e.g., one, two, three, four, five, or six additional therapeutic agents).

The "subject" according to any of the above embodiments may be a human. When the medicament is used for therapeutic purposes, the subject is an individual who has had or is suspected of having a disease of interest. When the medicament is used for prophylactic purposes, the subject is an individual susceptible to a disease of interest.

In yet another aspect, provided is a pharmaceutical composition comprising the antigen-binding molecule, e.g.,

for any of the above pharmaceutical uses or treatment methods. In another embodiment, the pharmaceutical composition further comprises at least one additional therapeutic agent.

The antigen-binding molecule of the present disclosure may be used alone or in combination with other agents for the treatment. For example, the antibody of the present disclosure may be administered (simultaneously or sequentially) in combination with at least one additional therapeutic agent.

The antigen-binding molecule of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule of the present disclosure will be formulated, administered, and applied in a manner consistent with Good Medical Practice, such as GOOD MEDICAL PRACTICE Guideline (GMP). Factors considered in this context include the specific disorder being treated, the specific mammal being treated, the clinical state of the individual subject, the cause of the disorder, the site of delivery of the agent, the method of administration, the timing of administration, and other factors known to medical practitioners. The antigen-binding molecule needs not be, but is optionally, formulated along with one or more agents currently used for preventing or treating the disorder. The effective amount of such additional agents depends on the amount of the antigen-binding molecule present in the pharmaceutical composition, the type of the disorder or treatment, and other factors discussed above. These are generally used in the same dosages and with routes of administration as described herein, or in about 1 to 99% of the dosages described herein, or in any dosage and by any route that is empirically/clinically determined to be appropriate.

For the prevention or treatment of disease, the appropriate dosage of the antigen-binding molecule of the present disclosure (when used alone or in combination with one or more other additional therapeutic agents) will depend on the type of the disease to be treated, the type of the therapeutic molecule, the severity and course of the disease, whether the therapeutic molecule is administered for prophylactic or therapeutic purposes, previous therapy, the subject's clinical history and response to the therapeutic molecule, and the discretion of the attending physician. The therapeutic molecule is suitably administered to a subject in one or a series of treatments. An exemplary unit daily dose is 50 $\mu$g to 5 g depending on the type and severity of the disease.

## Article of Manufacture

In another aspect of the present disclosure, provided is an article of manufacture, which comprises materials useful for the treatment, prevention, and/or diagnosis of the above disorders.

The article of manufacture comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper). At least one active agent in the composition is the antigen-binding molecule of the present disclosure. The label or package insert indicates that the composition is used to treat the selected condition.

Moreover, the article of manufacture may comprise:

(a) a first container with a composition contained therein, wherein the composition comprises the antigen-binding molecule of the present disclosure; and
(b) a second container with a composition contained therein, wherein the composition comprises a cytotoxic or otherwise therapeutic agent.

Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes. In a specific embodiment, the article of manufacture is a kit.

## Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

**Examples**

**Example 1. Antigen-Binding Molecules Comprising Titin Chain/Obscurin Chain**

The Titin chain/Obscurin chain of the present disclosure may be derived from any suitable polypeptide, including polypeptides derived from PCT/CN2021/070832 and its publication WO2021139758A1 (incorporated herein by reference in its entirety) and CN202110527339.7 and the patents which refer to it as a priority document. Bispecific antibodies were constructed in which the CL was the kappa light chain constant region in PCT/CN2021/070832. The amino acid sequences of the Titin chain and the Obscurin chain are shown in Tables 4-1 and 4-2; linker sequences include GGGGS (SEQ ID NO: 106), ASTKG (SEQ ID NO: 109), or RTVAS (SEQ ID NO: 110), and the amino acid sequences of the Fc1, Fc2, and CH1 in this example are shown below.

Table 4-1. Amino acid sequences of Titin chain

| No. | SEQ ID NO | Amino acid sequence |
|---|---|---|
| T.0 | 45 | GIPPKIEALPSDISIDEGKVLTVACAFTGEPTPEVTWSCGGRKIHSQEQG RFHIENTDDLTTLIIMDVQKQDGGLYTLSLGNEFGSDSATVNIHIRSI |
| T.1 | 46 | GIPPKIECLPSDISIDEGKVLTVASAFTGEPTPEVTWSTGGRKIHSQEQG RFHIENTDDLTTLIIMDVQKQDGGLYTLSLGNEFGSDSATVNIHIRSI |
| T.2 | 47 | GIPPKIEALPSDISIDEGKCLTVASAFTGEPTPEVTWSTGGRKIHSQEQG RFHIENTDDLTTLIIMDVQKQDGGLYTLSLGNEFGSDSATVNIHIRSI |
| T.3 | 48 | GIPPKIEALPSDISIDEGKVLTVASCFTGEPTPEVTWSTGGRKIHSQEQG RFHIENTDDLTTLIIMDVQKQDGGLYTLSLGNEFGSDSATVNIHIRSI |
| T.4 | 49 | GIPPKIEALPSDISIDEGKVLCVASAFTGEPTPEVTWSTGGRKIHSQEQG RFHIENTDDLTTLIIMDVQKQDGGLYTLSLGNEFGSDSATVNIHIRSI |
| T.5 | 50 | KAGIRGIPPKIECLPSDISIDEGKVLTVASAFTGEPTPEVTWSTGGRKIHS QEQGRFHIENTDDLTTLIIMDVQKQDGGLYTLSLGNEFGSDSATVNIHI RSI |
| T.6 | 51 | KAGIRGIPPKIEALPSDISIDEGKVLTVACAFTGEPTPEVTWSCGGRKIH SQEQGRFHIENTDDLTTLIIMDVQKQDGGLYTLSLGNEFGSDSATVNIH IRSI |
| T.7 | 52 | GIPPKIECLPSDISIDEGKVLTVASAFTGEPTPEVTWSTGGRKIHSQEQG RFHIENTDDLTTLIISDVQKQDGGLYSLSLGNEFGSDSATVNIHIRSI |
| T.8 | 53 | GIPPKIECLPSDISIDEGKVLTVASAFTGEPTPEVTWSTGGRKIHSQEQG RFHIENTDDLTTLIIKDVQRQDGGLYTLTLRNEFGSDSATVNIHIRSI |
| T.9 | 54 | GIWPKIECLPIDLSIDEGKVLMVASAFTGEPTPEVTWSTGGRKIHSQEQ GRFHIENTDDLTTLIIMDVQKQDGGLYTLSLGMEFGSDSATVNIHIRSI |

(continued)

| No. | SEQ ID NO | Amino acid sequence |
|---|---|---|
| T.10 | 55 | GIPPKIECLPIDISIDEGKVLTVASAFTGEPTPEVTWSTGGRKIHSQEQGR FHIENTDDLTTLIIKDVQKQDGGLYTLTLRNEFGSDSATVNIHIRSI |
| T.11 | 56 | GIPPKIECLPSDISIDEGKVLTVASAFTGEPTPEVTWSTSGKKISSEEGGR FHIESTEDLTTLIIMDVQKQDGGVYTLSLGNDLGSDSATVNIHIRSI |
| T.12 | 57 | GIPPKIECLPSDISIDEGKVLTVASAFTGEPTPEVTWSTGGRKIHSEEGG RFHIESTEDLTTLIIMDVQKQDGGVYTLSLGNEFGSDSATVNIHIRSI |
| T.13 | 58 | GIPPKIECLPSDISIDEGKVLTVASAFTGEPTPEVTWSTGGRKIHSQEQG RFHIESTEDLTTLIIMDVQKQDGGVYTLSLGNEFGSDSATVNIHIRSI |
| T.14 | 59 | GIPPKIECLPSDISIDEGKVLTVASAFTGEPTPEVTWSTGGRKIHSQEQG RFHIESTEDLTTLIISDVQKQDGGLYSLSLGNEFGSDSATVNIHIRSI |
| T.15 | 60 | KAGIRGIPPKIECLPSDISIDEGKVLTVASAFTGEPTPEVTWSTGGRKIHS QEQGRFHIESTEDLTTLIISDVQKQDGGLYSLSLGNEFGSDSATVNIHIR SI |
| T.16 | 61 | GIPPKIECLPIDISIDEGKVLTVASAFTGEPTPEVTWSTGGRKIHSQEQGR FHIENTDDSTTLTIKDVQKQDGGLYTLTLRNEFGSDSATVNIHIRSI |
| T.17 | 62 | GIPPKIECLPIDISIDEGKCLTVASAFTGEPTPEVTWSTGGRKIHSQEQGR FHIENTDDSTTLTIKDVQKQDGGLYTLTLRNEFGSDSATVNIHIRSI |
| T.18 | 63 | GIPPKIECLPIDISIDEGKVLTVASCFTGEPTPEVTWSTGGRKIHSQEQGR FHIENTDDSTTLTIKDVQKQDGGLYTLTLRNEFGSDSATVNIHIRSI |

Table 4-2. Amino acid sequences of Obscurin chain

| No. | SEQ ID NO | Amino acid sequence |
|---|---|---|
| O.0 | 64 | SGAPRFLTRPKAFVVSVGKDATLSCQIVGNPTPQVSWEKDQQPVAAG ARFRLAQDGDLYRLTILDLALGDSGQYVCRARNAIGEAFAAVGLQV DAEA |
| OL.0 | 65 | QGSPPCFLRFPRPVRVVSGAEAELKCVVLGEPPPVVVWEKGGQQLA ASERLSFPADGAEHGLLLTAALPTDAGVYVCRARNAAGEAYAAAAV TVLEPP |
| O.1 | 66 | SGAPRFLTRPKAFVVSVGKDATLSCQIVGNPTPQVSWEKDQQPVAAG ARFRLAQDGDLYRLTILDLALGDSGQYVCRARNAIGEAFACVGLQV DAEA |

(continued)

| No. | SEQ ID NO | Amino acid sequence |
|---|---|---|
| O.2 | 67 | SGCPRFLTRPKAFVVSVGKDATLSCQIVGNPTPQVSWEKDQQPVAAG ARFRLAQDGDLYRLTILDLALGDSGQYVCRARNAIGEAFAAVGLQV DAEA |
| O.3 | 68 | SGAPRFLTCPKAFVVSVGKDATLSCQIVGNPTPQVSWEKDQQPVAAG ARFRLAQDGDLYRLTILDLALGDSGQYVCRARNAIGEAFAAVGLQV DAEA |
| O.4 | 69 | SGAPRFLTRPKAFVVSVGKDATLSSQIVGNPTPQVSWEKDQQPVAAG ARFRLAQDGDLYRLTILDLALGDSGQYVSRARNAIGEAFACVGLQV DAEA |
| O.5 | 70 | SGCPRFLTRPKAFVVSVGKDATLSSQIVGNPTPQVSWEKDQQPVAAG ARFRLAQDGDLYRLTILDLALGDSGQYVSRARNAIGEAFAAVGLQVD AEA |
| O.6 | 71 | SGAPRFLTCPKAFVVSVGKDATLSSQIVGNPTPQVSWEKDQQPVAAG ARFRLAQDGDLYRLTILDLALGDSGQYVSRARNAIGEAFAAVGLQVD AEA |
| O.7 | 72 | SGAPRFKTRPKAFVVSVGKDATLSSQIVGNPTPQVSWEKDQQPVAA GARFRLAQDGDLYRLKILDLALGDSGQYVSRARNAIGEAFACVGLQ VDAEA |
| O.8 | 73 | SGAPRFKTRPKAFVVSVGKDATLSSQIVGNPTPQVSWEKDQQPVAA GARFRLAQDGDLYRLHILDLALGDSGQYVSRARNAIGEAFACVGLQ VDAEA |
| O.9 | 74 | SGAPRFLTRPLAFVVSVGKDATLSSQIVGNPTPQVSWEKDQQPVAAG ARFRLAQDGDLYRLKILDLALGDSGQYVSRARNAIGEAFACVGLQV DAEA |
| O.10 | 75 | SGAPRFLTRPLAFVVSVGKDATLSSQIVGNPTPQVSWEKDQQPVAAG ARFRLAQDGDLYRLHILDLALGDSGQYVSRARNAIGEAFACVGLQV DAEA |
| O.11 | 76 | DQPQFSGAPRFLTRPKAFVVSVGKDATLSSQIVGNPTPQVSWEKDQQ PVAAGARFRLAQDGDLYRLTILDLALGDSGQYVSRARNAIGEAFACV GLQVDAEA |
| O.12 | 77 | DQPQFSGAPRFKTRPKAFVVSVGKDATLSSQIVGNPTPQVSWEKDQQ PVAAGARFRLAQDGDLYRLKILDLALGDSGQYVSRARNAIGEAFAC VGLQVDAEA |

(continued)

| No. | SEQ ID NO | Amino acid sequence |
|---|---|---|
| O.13 | 78 | DQPQFSGAPRFKTRPKAFVVSVGKDATLSSQIVGNPTPQVSWEKDQQ PVAAGARFRLAQDGDLYRLHILDLALGDSGQYVSRARNAIGEAFAC VGLQVDAEA |
| O.14 | 79 | DQPQFSGAPRFRTRPKAFVVSVGKDATLSSQIVGNPTPQVSWEKDQQ PVAAGARFRLAQDGDLYRLKILDLALGDSGQYVSRARNAIGEAFAC VGLQVDAEA |
| O.15 | 80 | DQPQFSGAPRFRTRPKAFVVSVGKDATLSSQIVGNPTPQVSWEKDQQ PVAAGARFRLAQDGDLYRLHILDLALGDSGQYVSRARNAIGEAFAC VGLQVDAEA |
| O.16 | 81 | DQPQFSGAPRFLTRPKAFVVSVGKDATLSCQIVGNPTPQVSWEKDQQ PVAAGARFRLAQDGDLYRLTILDLALGDSGQYVCRARNAIGEAFAAV GLQVDAEA |
| OL.1 | 82 | QGSPPEFLRFPRPVRVVSGAEAELKCVVLGEPPPVVVWEKGGQQLA ASERLSFPADGAEHGLLLTAALPTDAGCYVCRARNAAGEAYAAAAV TVLEPP |
| OL.2 | 83 | QGSPPEFLRFPRPVRVVSGAEAELKCVVLGEPPPVVVWEKGGQQLA ASERLSFPADGAEHGLLLTAALPTDAGVYVCRARNAACEAYAAAAV TVLEPP |
| OL.3 | 84 | QGSPPEFLRFPRPVRVVSGAEAELKCVVLGEPPPVVVWEKGGQQLA ASERLSFPADGAEHGLLLTAALPTDAGVYVCRARNAAGECYAAAAV TVLEPP |
| OL.4 | 85 | QGSPPEFLRFPRPVRVVSGAEAELKSVVLGEPPPVVVWEKGGQQLA ASERLSFPADGAEHGLLLTAALPTDAGCYVSRARNAAGEAYAAAAV TVLEPP |
| OL.5 | 86 | QGSPPEFLRFPRPVRVVSGAEAELKSVVLGEPPPVVVWEKGGQQLA ASERLSFPADGAEHGLLLTAALPTDAGVYVSRARNAACEAYAAAAV TVLEPP |
| OL.6 | 87 | QGSPPEFLRFPRPVRVVSGAEAELKSVVLGEPPPVVVWEKGGQQLA ASERLSFPADGAEHGLLLTAALPTDAGVYVSRARNAAGECYAAAAV TVLEPP |
| O.17 | 88 | SGAPRFLTRPKSYTVSVGKDASLSSQIVGNPTPQVSWEKDQQPVAAG ARFRLAQDGDLYRLKILDLALGDSGQYVSRARNAIGEAFACVGLQV DAEA |

(continued)

| No. | SEQ ID NO | Amino acid sequence |
|---|---|---|
| O.18 | 89 | SEAPRFLTRPLAFVVSEGKDATLSSQIVGDPPPEVTWEKDQQPITAGA RFRLAQDGDVYRLEILDLQLSDSGQYVSRARNAIGEAFACVGLQVD AEG |
| O.19 | 90 | SGAPRFLTRPLAFVVSVGKLAMLSSQIVGNPTPQVSWEKDQQPVAAG ARFRLLQDGDLYRLKILDLALGDSGQYVSRARNAIGEAFACVGLQV DAEA |
| O.20 | 91 | SGAPRFLTRPLAFVVSVGKDATLSSQIVGNPTPQVSWEKDKQPVTAG ARFRLAQDGDLYRLKILDLQLSDSGQYVSRARNAIGEAFACLGLQVD AEA |
| O.21 | 92 | SGAPRFLTRPLAFVVSVGKDATLSSQIVGNPTPQVSWEKDQLPVTAG ARFRLAQDGDLYRLKILDLTLSDSGQYVSRARNAIGEAFACVGLEVG AEA |
| O.22 | 93 | SGAPRFLTRPLSYVVSVGKDASLSSQIVGNPTPQVSWEKDQLPVTAG ARFRLAQDGDLYRLKILDLQLSDSGQYVSRARNAIGEAFACVGLEVG AEA |
| O.23 | 94 | DQPQFSGAPRFLTRPLSYVVSVGKDASLSSQIVGNPTPQVSWEKDQL PVTAGARFRLAQDGDLYRLKILDLQLSDSGQYVSRARNAIGEAFACV GLEVGAEA |
| O.24 | 95 | SGAPRFLTRPKAFVVSVGKDATLSSQIVGNPFPQVSWEKDKQPVTAG VRFRLAQDGDLYRLKILDLQLSDSGQYVSRARNAIGEAFACLGLQVD AEA |
| O.25 | 96 | SGAPRFLTRPKASVVSVGKDATLSSQIVGNPFPQVSWEKDKQPVTAG VRFRLAQDGDLYRLKILDLQLSDSGQYVSRARNAHGEAFACLGLQV DAEA |
| O.26 | 97 | SGCPRFLTRPKASVVSVGKDATLSSQIVGNPFPQVSWEKDKQPVTAG VRFRLAQDGDLYRLKILDLQLSDSGQYVSRARNAHGEAFACLGLQV DAEA |
| O.27 | 98 | SGAPRFLTCPKASVVSVGKDATLSSQIVGNPFPQVSWEKDKQPVTAG VRFRLAQDGDLYRLKILDLQLSDSGQYVSRARNAHGEAFACLGLQV DAEA |
| O.28 | 99 | SGAPRFLTRPKASVVSVGKDATLSCQIVGNPFPQVSWEKDKQPVTAG VRFRLAQDGDLYRLKILDLQLSDSGQYVCRARNAHGEAFACLGLQV DAEA |

(continued)

| No. | SEQ ID NO | Amino acid sequence |
|---|---|---|
| O.29 | 100 | SGCPRFLTRPKASVVSVGKDATLSCQIVGNPFPQVSWEKDKQPVTAG VRFRLAQDGDLYRLKILDLQLSDSGQYVCRARNAHGEAFACLGLQV DAEA |
| O.30 | 101 | SGAPRFLTCPKASVVSVGKDATLSCQIVGNPFPQVSWEKDKQPVTAG VRFRLAQDGDLYRLKILDLQLSDSGQYVCRARNAHGEAFACLGLQV DAEA |
| O.31 | 102 | SGAPRFLTRPKASVVSVGKDATLSSQIVGNPFPQVSWEKDKQPVTAG VRFRLAQDGDLYRLKILDCQLSDSGQYVSRARNAHGEAFACLGLQC DAEA |
| O.32 | 103 | SGCPRFLTRPKASVVSVGKDATLSSQIVGNPFPQVSWEKDKQPVTAG VRFRLAQDGDLYRLKILDCQLSDSGQYVSRARNAHGEAFACLGLQC DAEA |
| O.33 | 104 | SGAPRFLTCPKASVVSVGKDATLSSQIVGNPFPQVSWEKDKQPVTAG VRFRLAQDGDLYRLKILDCQLSDSGQYVSRARNAHGEAFACLGLQC DAEA |

> Fc1 (knob, SEQ ID NO: 111)

DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK

> Fc2 hole, SEQ ID NO: 112)

DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTIS KAKGQPREPQVCTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGK;

> CH1 (SEQ ID NO: 113)

ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSC.

**1.1 DI bispecific antibodies**

Construction of DI bispecific antibodies against hNGF and hRANKL: DI-2 to DI-20 comprising a first heavy chain, a second heavy chain, a first light chain, and a second light chain as described below with reference to Example 5 in PCT/CN2021/070832:

the first heavy chain comprises [VH1-I]-[linker 1]-[Obscurin chain]-[Fc2] in order from the N-terminus to the C-terminus,
the first light chain comprises [VL1-I]-[linker 2]-[Titin chain] in order from the N-terminus to the C-terminus,
the second heavy chain comprises [VH2-D]-[CH1]-[Fc1] in order from the N-terminus to the C-terminus, and
the second light chain comprises [VL2-D]-[CL] in order from the N-terminus to the C-terminus; wherein the VH1-I and VL1-I were the heavy chain variable region and the light chain variable region of I0 in PCT/CN2021/070832, respectively, and the VH2-D and VL2-D were the heavy chain variable region and the light chain variable region of D0 in PCT/CN2021/070832, respectively. The structures of the Obscurin chain, the Titin chain, the linker 1, and the linker 2 in the DI bispecific antibodies of this example are shown in the following table.

Table 5. Obscurin chain/Titin chain and linkers in DI bispecific antibodies

| No. | First heavy chain | | First light chain | |
| --- | --- | --- | --- | --- |
| | Obscurin chain | Linker 1 | Titin chain | Linker 2 |
| DI-2 | O.20 | GGGGS | T.10 | GGGGS |
| DI-3 | O.25 | GGGGS | T.16 | GGGGS |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | 0.31 | ASTKG | T.16 | RTVAS |
| DI-19 | O.32 | ASTKG | T.17 | RTVAS |
| DI-20 | O.33 | ASTKG | T.18 | RTVAS |
| Note: the numbers of the Titin chain and the Obscurin chain in the tables are shown in Tables 4-1 and 4-2. | | | | |

The binding activity of the bispecific antibodies DI-2 to DI-20 against their antigens was assayed by the method of Test Example 4 in PCT/CN2021/070832. The antibodies were subjected to a thermal stability study. The study method was as follows: the antibodies were each diluted to a concentration of 5 mg/mL with PBS and tested for thermal stability using a high throughput differential scanning fluorimeter (UNCHAINED, Specification: Unit).

The experimental results showed that the binding activity of the modified bispecific antibodies against the antigen was not significantly changed; meanwhile, DI-4 to DI-8, DI-10 to DI-16, and DI-20 had significantly improved Tm1 (°C) and Tonset (°C) as compared to DI-2, and therefore, the bispecific antibodies had better thermal stability.

Table 6. Binding activity assay on DI bispecific antibodies

| No. | RANKL | NGF | No. | RANKL | NGF |
|-----|-------|-----|-----|-------|-----|
| | EC50 (nM) | | | EC50 (nM) | |
| DI-2 | 0.3832 | 6.633 | DI-12 | 0.4125 | 6.5156 |
| DI-3 | 0.3613 | 5.7730 | DI-13 | 0.4440 | 5.5420 |
| DI-4 | 0.3959 | 6.2930 | DI-14 | 0.4182 | 3.2610 |
| DI-5 | 0.3290 | 6.1890 | DI-15 | 0.2206 | 5.2800 |
| DI-6 | 0.2509 | 5.6720 | DI-16 | 0.1474 | 5.5140 |
| DI-7 | 0.2557 | 6.6430 | DI-17 | 0.2329 | 6.7270 |
| DI-8 | 0.3643 | 7.6250 | DI-18 | 0.2662 | 5.9080 |
| DI-9 | 0.2944 | 8.4950 | DI-19 | 0.1843 | 5.9280 |
| DI-10 | 0.3460 | 7.1660 | DI-20 | 0.3184 | 6.4250 |
| DI-11 | 0.3721 | 10.9600 | | | |

Table 7. Thermal stability test results for DI bispecific antibodies

| No. | Tm1 (°C) | Tonset (°C) | No. | Tm1 (°C) | Tonset (°C) |
|-----|----------|-------------|-----|----------|-------------|
| DI-2 | 55.6 | 48.3 | DI-11 | 57.35 | - |
| DI-4 | 60.1 | 52.493 | DI-12 | 59.9 | 51.726 |
| DI-5 | 61 | 51.967 | DI-13 | 61 | 50.988 |
| DI-6 | 60.8 | 53.012 | DI-14 | 61.2 | 52.191 |
| DI-7 | 60.34 | 52.003 | DI-15 | 60.41 | 50.558 |
| DI-8 | 60.61 | 50.425 | DI-16 | 61.5 | 50.691 |
| DI-10 | 60.2 | 52.766 | DI-20 | 60.7 | 51.859 |

Solutions of the DI bispecific antibodies were formulated with a buffer containing 10 mM acetic acid at pH 5.5 and 9% sucrose, and the solutions were incubated in an incubator at 40 °C for four weeks. After the end of the incubation, the antibodies were concentrated to the concentration at the start of the incubation, and the precipitation of the solutions was observed. The experimental results showed that there was precipitation in the solution of DI-2 bispecific antibody group, and therefore, DI-3 to DI-7 had better stability than DI-2.

Table 8. Precipitation of solutions of DI bispecific antibodies

| No. | Initial concentration | Concentration to be achieved at week 4 | Precipitation of solutions |
|-----|----------------------|----------------------------------------|----------------------------|
| DI-2 | 20 mg/mL | 20 mg/mL | Precipitation occurred |
| DI-3 | 20 mg/mL | 20 mg/mL | No precipitation |
| DI-4 | 60 mg/mL | 60 mg/mL | No precipitation |
| DI-5 | 25 mg/mL | 25 mg/mL | No precipitation |
| DI-6 | 60 mg/mL | 60 mg/mL | No precipitation |
| DI-7 | 16 mg/mL | 16 mg/mL | No precipitation |

## 1.2 PL bispecific antibodies

Construction of PL bispecific antibodies against hPDL1 and hCTLA4: PL-1 to PL-19 comprising a first heavy chain, a second heavy chain, a first light chain, and a second light chain as described below:

the first heavy chain comprises [VH1-P]-[linker 1]-[Obscurin chain]-[Fc1] in order from the N-terminus to the C-terminus,

the first light chain comprises [VL1-P]-[linker 2]-[Titin chain] in order from the N-terminus to the C-terminus,

the second heavy chain comprises [VH2-L]-[CH1]-[Fc2] in order from the N-terminus to the C-terminus, and

the second light chain comprises [VL2-L]-[CL] in order from the N-terminus to the C-terminus; wherein the VH1-P and VL1-P were the heavy chain variable region and the light chain variable region of the h1831K antibody in WO2020177733A1, respectively. The amino acid sequences of the VH1-L and VL1-L are shown below.

> VH2-L (SEQ ID NO: 114)

QVQLVESGGGVVQPGRSLRLSCAASGFTFSSYTMHWVRQAPGKGLEWVTFISYDGNNK YYADSVKGRFTISRDNSKNTLYLQMNSLRAEDTAIYYCARTGWLGPFDYWGQGTLVTV SS

> VL2-L (SEQ ID NO: 115)

EIVLTQSPGTLSLSPGERATLSCRASQSVGSSYLAWYQQKPGQAPRLLIYGAFSRATGIPD RFSGSGSGTDFTLTISRLEPEDFAVYYCQQYGSSPWTFGQGTKVEIK.

The structures of the Obscurin chain, the Titin chain, the linker 1, and the linker 2 in the PL bispecific antibodies of this example are shown in the following table.

Table 9. Obscurin chain/Titin chain and linkers in PL bispecific antibodies

| No. | First heavy chain | | First light chain | |
|---|---|---|---|---|
| | Obscurin chain | Linker 1 | Titin chain | Linker 2 |
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | 0.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | 0.31 | ASTKG | T.16 | RTVAS |
| PL-18 | O.32 | ASTKG | T.17 | RTVAS |

(continued)

| No. | First heavy chain | | First light chain | |
|---|---|---|---|---|
| | Obscurin chain | Linker 1 | Titin chain | Linker 2 |
| PL-19 | O.33 | ASTKG | T.18 | RTVAS |

Note: the numbers of the Titin chain and the Obscurin chain in the tables are shown in Tables 4-1 and 4-2.

The binding activity of PL bispecific antibodies was tested by the ELISA assay with reference to Test Example 4 in PCT/CN2021/070832, wherein hPDL1 and hCTLA4 antigens were purchased from Sino biology. The antibodies were subjected to a thermal stability study. The method was as follows: the antibodies were each diluted to a concentration of 1.4-3 mg/mL with PBS and tested for thermal stability using a high throughput differential scanning fluorimeter (UNCHAINED, Specification: Unit). The experimental results showed that the PL bispecific antibodies still had good binding activity to the antigen; meanwhile, PL-2 to PL-19 had significantly improved Tm1 (°C), Tagg 266 (°C), and Tonset (°C) as compared to PL-1, and therefore, the bispecific antibodies had better thermal stability.

Table 10. Binding activity assay on PL bispecific antibodies

| No. | hPDL 1 | hCTLA4 | No. | hPDL1 | hCTLA4 |
|---|---|---|---|---|---|
| | EC50 (nM) | | | EC50 (nM) | |
| PL-1 | 1.6 | 16.5 | PL-11 | 0.6 | 5.6 |
| PL-2 | 0.5 | 8.0 | PL-12 | 0.5 | 8.0 |
| PL-3 | 0.6 | 7.9 | PL-13 | 0.5 | 4.4 |
| PL-4 | 0.7 | 6.5 | PL-14 | 1.3 | 6.0 |
| PL-5 | 0.7 | 6.7 | PL-15 | 1.4 | 3.2 |
| PL-6 | 0.8 | 5.3 | PL-16 | 1.7 | 34.9 |
| PL-7 | 0.5 | 7.3 | PL-17 | 1.4 | 6.2 |
| PL-8 | 1.4 | 14.5 | PL-18 | 1.6 | 6.7 |
| PL-9 | 0.6 | 5.5 | PL-19 | 1.6 | 6.5 |
| PL-10 | 0.6 | 6.9 | | | |

Table 11. Thermal stability test results for PL bispecific antibodies

| No. | Tm1 (°C) | Tagg 266 (°C) | Tonset (°C) | No. | Tm1 (°C) | Tagg 266 (°C) | Tonset (°C) |
|---|---|---|---|---|---|---|---|
| PL-1 | 61.64 | 64.82 | 52.566 | PL-11 | 65.88 | - | 57.976 |
| PL-2 | 66.20 | 66.55 | 58.317 | PL-12 | 65.54 | 67.94 | - |
| PL-3 | 64.07 | 68.28 | 57.661 | PL-13 | 71.85 | 68.17 | 58.581 |
| PL-4 | 70.71 | 67.29 | 56.246 | PL-14 | 74.18 | 69.42 | 58.589 |
| PL-5 | 74.56 | 68.11 | 58.407 | PL-15 | 70.96 | 69.91 | 58.622 |
| PL-6 | 70.43 | 70.12 | 61.069 | PL-16 | 63.48 | 68.98 | 58.702 |
| PL-7 | 68.46 | 67.41 | 63.031 | PL-17 | 70.15 | 69.86 | 56.193 |
| PL-8 | 65.00 | - | - | PL-18 | - | 69.43 | - |
| PL-9 | 69.24 | 67.83 | 58.597 | PL-19 | - | 69.52 | 57.766 |
| PL-10 | 69.63 | 68.12 | 56.788 | | | | |

**1.3 HJ bispecific antibodies**

Construction of HJ bispecific antibodies against hIL5 and hTSLP: HJ-3 to HJ11 comprising a first heavy chain, a second heavy chain, a first light chain, and a second light chain as described below:

the first heavy chain comprises [VH1-H]-[linker 1]-[Titin chain]-[Fc1] in order from the N-terminus to the C-terminus, the first light chain comprises [VL1-H]-[linker 2]-[Obscurin chain] in order from the N-terminus to the C-terminus, the second heavy chain comprises [VH2-J]-[CH1]-[Fc2] in order from the N-terminus to the C-terminus, and the second light chain comprises [VL2-J]-[CL] in order from the N-terminus to the C-terminus; wherein the VH1-H and VL1-H were the heavy chain variable region and the light chain variable region of H0 in PCT/CN2021/070832, respectively, and the VH2-J and VL2-J were the heavy chain variable region and the light chain variable region of J1 in PCT/CN2021/070832, respectively. The structures of the Obscurin chain, the Titin chain, the linker 1, and the linker 2 in the HJ bispecific antibodies of this example are shown in the following table.

Table 12. Obscurin chain/Titin chain and linkers in HJ bispecific antibodies

| No. | First heavy chain | | First light chain | |
|---|---|---|---|---|
| | Titin chain | Linker 1 | Obscurin chain | Linker 2 |
| HJ-3 | T.10 | GGGGS | O.20 | GGGGS |
| HJ-5 | T.16 | GGGGS | O.25 | GGGGS |
| HJ-6 | T.16 | GGGGS | O.27 | GGGGS |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |
| HJ-11 | T.16 | ASTKG | O.29 | RTVAS |

The antigen-binding activity of HJ bispecific antibodies was tested by the method with reference to Test Example 4 in PCT/CN2021/070832. The thermal stability test was performed on the antibodies by the following process: the dilutions of the HJ bispecific antibodies were formulated with a buffer containing 10 mM acetic acid at pH 5.5 and 9% sucrose; the bispecific antibodies were concentrated by ultrafiltration and concentration to obtain different concentrations of solutions (the HJ bispecific antibody concentrations are shown in Table 19) of the HJ bispecific antibodies; the concentrated solutions were incubated in an incubator at 40 °C, and the samples were tested for SEC purity on day 0 (i.e., before the start of incubation at 40 °C, D0), day 7 (the 7th day of incubation at 40 °C, D7), day 14 (the 14th day of incubation at 40 °C, D14), day 21 (the 21st day of incubation at 40 °C, D21), and day 28 (the 28th day of incubation at 40 °C, D28); after 28 days of incubation at 40 °C, the samples were immediately taken for the determination of CE-SDS purity. The experimental results showed that the binding activity of the HJ bispecific antibodies constructed in the present disclosure against the antigen was not significantly changed; meanwhile, the HJ-5 to HJ-11 bispecific antibodies had better thermal stability than HJ-3.

Table 13-1. Binding activity assay on HJ bispecific antibodies

| No. | hIL5 | hTSLP | No. | hIL5 | hTSLP |
|---|---|---|---|---|---|
| | EC50 (nM) | | | EC50 (nM) | |
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

Table 13-2. Accelerated stability test results for HJ bispecific antibodies

| No. | D0 concentration (mg/mL) | D0 SEC purity (%) | D28 SEC purity (%) | D28Δ SEC purity (%) | D28 non-reduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

**Example 2. Preparation of Antigen Proteins and Antibodies**

**1.1 Antigen protein structure**

With human EGFR protein (UniProt epidermal growth factor receptor, Uniprot number: P00533) used as a template of EGFR, His tag was fused on the basis of the EGFR protein, and a human EGFR protein extracellular domain with the His tag (abbreviated as EGFR-His) for detection of the present invention was designed. The amino acid sequence is as follows.

Amino acid sequence of EGFR-His:

LEEKKVCQGTSNKLTQLGTFEDHFLSLQRMFNNCEVVLGNLEITYVQRNYDLSFLKTIQ
EVAGYVLIALNTVERIPLENLQIIRGNMYYENSYALAVLSNYDANKTGLKELPMRNLQEI
LHGAVRFSNNPALCNVESIQWRDIVSSDFLSNMSMDFQNHLGSCQKCDPSCPNGSCWGA
GEENCQKLTKIICAQQCSGRCRGKSPSDCCHNQCAAGCTGPRESDCLVCRKFRDEATCK
DTCPPLMLYNPTTYQMDVNPEGKYSFGATCVKKCPRNYVVTDHGSCVRACGADSYEM
EEDGVRKCKKCEGPCRKVCNGIGIGEFKDSLSINATNIKHFKNCTSISGDLHILPVAFRGD

SFTHTPPLDPQELDILKTVKEITGFLLIQAWPENRTDLHAFENLEIIRGRTKQHGQFSLAVV
SLNITSLGLRSLKEISDGDVIISGNKNLCYANTINWKKLFGTSGQKTKIISNRGENSCKATG
QVCHALCSPEGCWGPEPRDCVSCRNVSRGRECVDKCNLLEGEPREFVENSECIQCHPEC
LPQAMNITCTGRGPDNCIQCAHYIDGPHCVKTCPAGVMGENNTLVWKYADAGHVCHL
CHPNCTYGCTGPGLEGCPTNGPKIPS*HHHHHH*

SEQ ID NO: 42 Note: the underlined part indicates the EGFR protein extracellular domain; the italicized part indicates the His tag.

With human HGFR protein (UniProt hepatocyte growth factor receptor, Uniprot number: P08581) used as a template of HGFR, His or Fc tag was fused on the basis of the HGFR protein, and a HGFR protein extracellular domain with the His tag (abbreviated as HGFR-His) or a HGFR protein extracellular domain with the Fc tag (abbreviated as HGFR-Fc) for detection of the present invention was designed. The amino acid sequence is as follows.

Amino acid sequence of HGFR-His:

ECKEALAKSEMNVNMKYQLPNFTAETPIQNVILHEHHIFLGATNYIYVLNEEDLQKVAE
YKTGPVLEHPDCFPCQDCSSKANLSGGVWKDNINMALVVDTYYDDQLISCGSVNRGTC
QRHVFPHNHTADIQSEVHCIFSPQIEEPSQCPDCVVSALGAKVLSSVKDRFINFFVGNTIN
SSYFPDHPLHSISVRRLKETKDGFMFLTDQSYIDVLPEFRDSYPIKYVHAFESNNFIYFLTV
QRETLDAQTFHTRIIRFCSINSGLHSYMEMPLECILTEKRKKRSTKKEVFNILQAAYVSKP
GAQLARQIGASLNDDILFGVFAQSKPDSAEPMDRSAMCAFPIKYVNDFFNKIVNKNNVR
CLQHFYGPNHEHCFNRTLLRNSSGCEARRDEYRTEFTTALQRVDLFMGQFSEVLLTSISTF
IKGDLTIANLGTSEGRFMQVVVSRSGPSTPHVNFLLDSHPVSPEVIVEHTLNQNGYTLVIT
GKKITKIPLNGLGCRHFQSCSQCLSAPPFVQCGWCHDKCVRSEECLSGTWTQQICLPAIY
KVFPNSAPLEGGTRLTICGWDFGFRRNNKFDLKKTRVLLGNESCTLTLSESTMNTLKCTV
GPAMNKHFNMSIIISNGHGTTQYSTFSYVDPVITSISPKYGPMAGGTLLTLTGNYLNSGNS
RHISIGGKTCTLKSVSNSILECYTPAQTISTEFAVKLKIDLANRETSIFSYREDPIVYEIHPTK
SFISGGSTITGVGKNLNSVSVPRMVINVHEAGRNFTVACQHRSNSEIICCTTPSLQQLNLQ
LPLKTKAFFMLDGILSKYFDLIYVHNPVFKPFEKPVMISMGNENVLEIKGNDIDPEAVKG
EVLKVGNKSCENIHLHSEAVLCTVPNDLLKLNSELNIEWKQAISSTVLGKVIVQPDQNFT
*HHHHHH*

SEQ ID NO: 43 Note: the underlined part indicates the HGFR protein extracellular domain; the italicized part indicates the His tag.
With human HGF protein (UniProt hepatocyte growth factor, Uniprot number: P14210) used as a template of HGF, a HGF protein with His tag (abbreviated as HGF-His) for detection of the present disclosure was designed. The amino acid sequence is as follows.
Amino acid sequence of HGF-His:

QRKRRNTIHEFKKSAKTTLIKIDPALKIKTKKVNTADQCANRCTRNKGLPFTCKAFVFDK
ARKQCLWFPFNSMSSGVKKEFGHEFDLYENKDYIRNCIIGKGRSYKGTVSITKSGIKCQP
WSSMIPHEHSFLPSSYRGKDLQENYCRNPRGEEGGPWCFTSNPEVRYEVCDIPQCSEVEC
MTCNGESYRGLMDHTESGKICQRWDHQTPHRHKFLPERYPDKGFDDNYCRNPDGQPRP

WCYTLDPHTRWEYCAIKTCADNTMNDTDVPLETTECIQGQGEGYRGTVNTIWNGIPCQ
RWDSQYPHEHDMTPENFKCKDLRENYCRNPDGSESPWCFTTDPNIRVGYCSQIPNCDMS
HGQDCYRGNGKNYMGNLSQTRSGLTCSMWDKNMEDLHRHIFWEPDASKLNENYCRN
PDDDAHGPWCYTGNPLIPWDYCPISRCEGDTTPTIVNLDHPVISCAKTKQLRVVNGIPTR
TNIGWMVSLRYRNKHICGGSLIKESWVLTARQCFPSRDLKDYEAWLGIHDVHGRGDEK
CKQVLNVSQLVYGPEGSDLVLMKLARPAVLDDFVSTIDLPNYGCTIPEKTSCSVYGWGY
TGLINYDGLLRVAHLYIMGNEKCSQHHRGKVTLNESEICAGAEKIGSGPCEGDYGGPLVC
EQHKMRMVLGVIVPGRGCAIPNRPGIFVRVAYYAKWIHKIILTYKVPQS*HHHHHH*

SEQ ID NO: 44

Note: the underlined part indicates the HGF protein; the italicized part indicates the His tag.

**1.2 Purification of proteins**

1.2.1 Purification steps of recombinant protein:

The cell expression supernatant sample was centrifuged at high speed to remove impurities, and the buffer was exchanged for PBS, followed by the addition of imidazole to make a final concentration of 5 mM. A nickel column was equilibrated with a PBS solution containing 5 mM imidazole and washed with 2-5 column volumes. The cell supernatant sample after buffer exchange was loaded on the Ni Sepharose excel column (GE, 17-3712-02). The column was washed with a PBS solution containing 5 mM imidazole until A280 reading dropped to the baseline. The chromatographic column was then washed with a mixture of PBS and 10 mM imidazole to remove non-specifically bound impure proteins, and the effluent was collected. The target protein was eluted with a PBS solution containing 300 mM imidazole and the elution peaks were collected. The collected eluate was concentrated and further purified using a gel chromatographic column Superdex200 (GE, 28-9893-35) with PBS as mobile phase. The polymer peaks were removed and the elution peak was collected. The obtained protein was identified by electrophoresis, peptide mapping, and LC-MS, and then aliquoted for later use if it was determined to be correct.

1.2.2 Purification steps of antibody:

The cell expression supernatant sample was centrifuged at high speed to remove impurities, and the supernatant was subjected to MabSelect Sure (GE, 17-5438-01) affinity chromatography. The MabSelect Sure chromatographic column was firstly regenerated with 0.2 M NaOH, then washed with pure water, and equilibrated with PBS. After the supernatant was bound, the column was washed with PBS until the A280 reading dropped to baseline. The target protein was eluted with 0.1 M acetate buffer at pH 3.5 and neutralized with 1 M Tris-HCl. The elution sample was properly concentrated and further purified using the gel chromatographic column Superdex200 (GE, 28-9893-35) equilibrated with PBS, and the target protein was concentrated to the appropriate concentration in the receiver tube where the target protein was collected. This method was used to purify the antibodies involved in the present invention.

**Example 3. Preparation and Identification of Bispecific Antibodies Specifically Binding to HGFR and EGFR**

Antibodies specifically binding to HGFR and EGFR were constructed using a molecule specifically binding to HGFR and a molecule specifically binding to EGFR.

1. **Molecule specifically binding to** EGFR

Molecules specifically binding to EGFR could be derived from any suitable antibody, such as zalutumumab (abbreviated as Zal) or a variant thereof (e.g., Zal.1 which was obtained by mutating the first amino acid residue of the Zal light chain from A to D), wherein:

> amino acid sequence of Zal heavy chain:

QVQLVESGGGVVQPGRSLRLSCAASGFTFSTYGMHWVRQAPGKGLEWVAVIWDDGSY
KYYGDSVKGRFTISRDNSKNTLYLQMNSLRAEDTAVYYCARDGITMVRGVMKDYFDY
WGQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKRVEPKSCDKTHTC
PPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK

SEQ ID NO: 1

> amino acid sequence of Zal light chain:

AIQLTQSPSSLSASVGDRVTITCRASQDISSALVWYQQKPGKAPKLLIYDASSLESGVPSR
FSGSESGTDFTLTISSLQPEDFATYYCQQFNSYPLTFGGGTKVEIKRTVAAPSVFIFPPSDEQ
LKSGTASVVCLLNNFYPREAKVQWKVDNALQSGNSQESVTEQDSKDSTYSLSSTLTLSK
ADYEKHKVYACEVTHQGLSSPVTKSFNRGEC

SEQ ID NO: 2

> amino acid sequence of Zal heavy chain variable region:

QVQLVESGGGVVQPGRSLRLSCAASGFTFSTYGMHWVRQAPGKGLEWVAVIWDDGSY
KYYGDSVKGRFTISRDNSKNTLYLQMNSLRAEDTAVYYCARDGITMVRGVMKDYFDY
WGQGTLVTVSS

SEQ ID NO: 3

> amino acid sequence of Zal light chain variable region:

AIQLTQSPSSLSASVGDRVTITCRASQDISSALVWYQQKPGKAPKLLIYDASSLESGVPSR
FSGSESGTDFTLTISSLQPEDFATYYCQQFNSYPLTFGGGTKVEIK

SEQ ID NO: 4

> amino acid sequence of Zal. 1 light chain variable region:

*D*IQLTQSPSSLSASVGDRVTITCRASQDISSALVWYQQKPGKAPKLLIYDASSLESGVPSR
FSGSESGTDFTLTISSLQPEDFATYYCQQFNSYPLTFGGGTKVEIK

SEQ ID NO: 5

> amino acid sequence of Zal. 1 heavy chain variable region set forth in SEQ ID NO: 3.

The CDR sequences of Zal and Zal.1 are shown in Table 14:

Table 14. CDRs of molecules specifically binding to EGFR Antibody Heavy chain Light chain Zal.1, Zal HCDR1 TYGMH LCDR1 RASQDISSALV (SEQ ID NO: 6) (SEQ ID NO: 9)

| Antibody | Heavy chain | | Light chain | |
|---|---|---|---|---|
| | HCDR1 | TYGMH (SEQ ID NO: 6) | LCDR1 | RASQDISSALV (SEQ ID NO: 9) |
| Zal.1, Zal | HCDR2 | VIWDDGSYKYYGDSVKG (SEQ ID NO: 7) | LCDR2 | DASSLES (SEQ ID NO: 10) |
| | HCDR3 | DGITMVRGVMKDYFDY (SEQ ID NO: 8) | LCDR3 | QQFNSYPLT (SEQ ID NO: 11) |
| Note: the above CDRs are determined according to the Kabat numbering scheme. | | | | |

**2. Molecule specifically binding to HGFR**

Molecules specifically binding to HGFR could be derived from any suitable antibody, including antibodies such as onartuzumab (abbreviated as Omab) described in International Publication No. WO2013003680A1 (incorporated herein by reference in its entirety), antibodies such as Ab10 described in International Publication No. WO2016165580A1 (incorporated herein by reference in its entirety), and mutant antibodies of the above antibodies. Among them, the amino acid sequences of the variable regions of Omab and Ab10 are as follows:

> amino acid sequence of Omab heavy chain variable region:

EVQLVESGGGLVQPGGSLRLSCAASGYTFTSYWLHWVRQAPGKGLEWVGMIDPSNSDT
RFNPNFKDRFTISADTSKNTAYLQMNSLRAEDTAVYYCATYRSYVTPLDYWGQGTLVTV
SS

SEQ ID NO: 12

> amino acid sequence of Omab light chain variable region:

DIQMTQSPSSLSASVGDRVTITCKSSQSLLYTSSQKNYLAWYQQKPGKAPKLLIYWASTR
ESGVPSRFSGSGSGTDFTLTISSLQPEDFATYYCQQYYAYPWTFGQGTKVEIK

SEQ ID NO: 13

> amino acid sequence of Ab10 heavy chain variable region:

QVQLVESGGGVVQPGRSLRLSCAASGFSLSNYGVHWVRQAPGKGLEWLAVIWSGGST
NYAAAFVSRLTISKDNSKNTVYLQMNSLRAEDTAVYYCARNHDNPYNYAMDYWGQGT
TVTVSS

SEQ ID NO: 14

> amino acid sequence of Ab10 light chain variable region:

DIVLTQSPDSLAVSLGERATINCRADKSVSTSTYNYLHWYQQKPGQPPKLLIYLASNLAS
GVPDRFSGSGSGTDFTLTISSLQAEDVAVYYCQHSRDLPPTFGQGTKLEIKR

SEQ ID NO: 15

The Ab10 mutant antibody Ab10.1 was obtained by mutating the CDRs of Ab10; the constant regions of Ab10.1 were identical to those of Ab10. The amino acid sequences of the variable regions of Ab 10. 1 are as follows:

> amino acid sequence of Ab 10. 1 heavy chain variable region:

QVQLVESGGGVVQPGRSLRLSCAASGFSLSDYGVHWVRQAPGKGLEWLAVIWSGGST
NYADAFVSRLTISKDNSKNTVYLQMNSLRAEDTAVYYCARNHDNPYIYAMDYWGQGT
TVTVSS

SEQ ID NO: 16

> amino acid sequence of Ab 10.1 light chain variable region:

DIVLTQSPDSLAVSLGERATINCRADKSVSTSTYNYLHWYQQKPGQPPKLLIYRGSFLAT

GVPDRFSGSGSGTDFTLTISSLQAEDVAVYYCQHSRDLPPTFGQGTKLEIK

SEQ ID NO: 17

Note: in the above sequences of the molecules specifically binding to HGFR, the underlined parts are the CDR region parts determined according to the Kabat numbering scheme, and the italicized and bold parts are the mutated amino acid residues.

The CDRs of molecules specifically binding to HGFR are shown in Table 15:

Table 15. CDRs of molecules specifically binding to HGFR

| Antibody | Heavy chain | | Light chain | |
|---|---|---|---|---|
| Omab | HCDR1 | SYWLH (SEQ ID NO: 18) | LCDR1 | KSSQSLLYTSSQKNYLA (SEQ ID NO: 21) |
| | HCDR2 | MIDPSNSDTRFNPNFKD (SEQ ID NO: 19) | LCDR2 | WASTRES (SEQ ID NO: 22) |
| | HCDR3 | YRSYVTPLDY (SEQ ID NO: 20) | LCDR3 | QQYYAYPWT (SEQ ID NO: 23) |
| Ab10 | HCDR1 | NYGVH (SEQ ID NO: 24) | LCDR1 | RADKSVSTSTYNYLH (SEQ ID NO: 27) |
| | HCDR2 | VIWSGGSTNYAAAFVS (SEQ ID NO: 25) | LCDR2 | LASNLAS (SEQ ID NO: 28) |
| | HCDR3 | NHDNPYNYAMDY (SEQ ID NO: 26) | LCDR3 | QHSRDLPPT (SEQ ID NO: 29) |
| Ab10.1 | HCDR1 | DYGVH (SEQ ID NO: 30) | LCDR1 | RADKSVSTSTYNYLH (SEQ ID NO: 27) |
| | HCDR2 | VIWSGGSTNYADAFVS (SEQ ID NO: 31) | LCDR2 | RGSFLAT (SEQ ID NO: 33) |
| | HCDR3 | NHDNPYIYAMDY (SEQ ID NO: 32) | LCDR3 | QHSRDLPPT (SEQ ID NO: 29) |
| Note: the above CDRs are determined according to the Kabat numbering scheme. | | | | |

### 3. Bispecific antibodies specifically binding to HGFR and EGFR

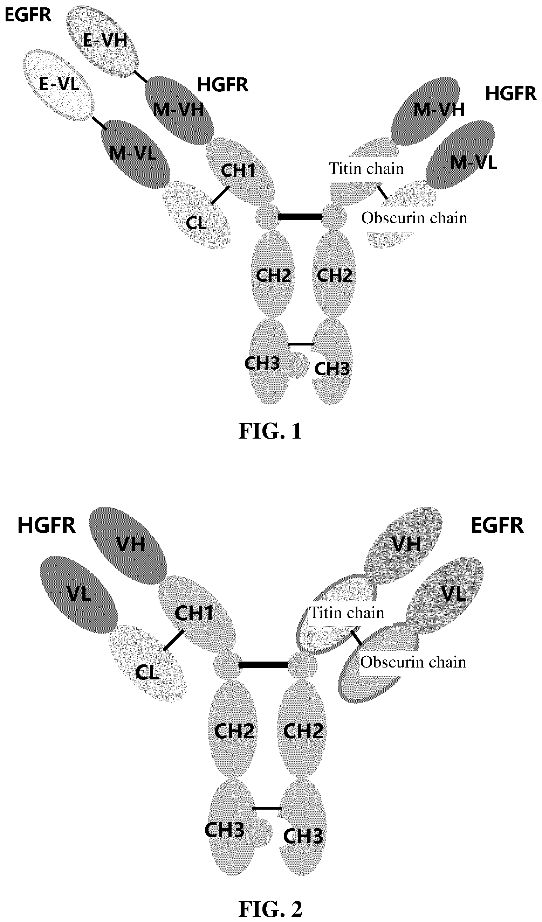

The bispecific antigen-binding molecule specifically binding to HGFR and EGFR and having a Format 1 or Format 2 structure was constructed using a molecule specifically binding to HGFR and a molecule specifically binding to EGFR. The structural schematic diagram of Format 1 is shown in FIG. 1, and the structural schematic diagram of Format 2 is shown in FIG. 2, wherein

Format 1 contains 4 chains with the following structures, wherein:

chain 1 is
VH (anti-EGFR antibody)-linker 1-VH (anti-HGFR antibody 1)-IgG$_1$ (CH1)-IgG$_1$Fc (knob); chain 2 is VL (anti-EGFR antibody)-linker 1-VL (anti-HGFR antibody 1)-CL;
chain 3 is VH (anti-HGFR antibody 2)-linker 2-Titin chain-IgG$_1$Fc (hole);
chain 4 is VL (anti-HGFR antibody 2)-linker 2-Obscurin chain.

Format 2 contains 4 chains with the following structures, wherein:

chain 1 is VH (anti-HGFR antibody)-IgG$_1$ (CH1)-IgG$_1$Fc (knob);
chain 2 is VL (anti-HGFR antibody)-CL;
chain 3 is VH (anti-EGFR antibody)-linker 2-Titin chain-IgG$_1$Fc (hole); and
chain 4 is VL (anti-EGFR antibody)-linker 2-Obscurin chain.

Illustratively, EM1 having the Format 1 structure and EM2 having the Format 2 structure were constructed using Ab10.1 (anti-HGFR antibody 1), Omab (anti-HGFR antibody 2), Zal.1 (anti-EGFR antibody), human IgG$_1$ heavy chain constant region/kappa light chain constant region, and T.16 (Titin chain)/O.28 (Obscurin chain).

The amino acid sequences of the 4 chains of EM1 are as follows.

Amino acid sequence of chain 1 of EM1:

QVQLVESGGGVVQPGRSLRLSCAASGFTFSTYGMHWVRQAPGKGLEWVAVIWDDGSY KYYGDSVKGRFTISRDNSKNTLYLQMNSLRAEDTAVYYCARDGITMVRGVMKDYFDY WGQGTLVTVSSGGGGSGGGGGQVQLVESGGGVVQPGRSLRLSCAASGFSLSDYGVHWV RQAPGKGLEWLAVIWSGGSTNYADAFVSRLTISKDNSKNTVYLQMNSLRAEDTAVYYC ARNHDNPYIYAMDYWGQGTTVTVSS*ASTKGPSVFPLAPSSKSTSGGTAALGCLVKDYFPEP VTVSWNSGALTSGVHTFPAVLQSSGLYSLSSVVTVPSSSLGTQTYICNVNHKPSNTKVDKKVEPK SC*DKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTI SKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK

SEQ ID NO: 34

Amino acid sequence of chain 2 of EM1:

DIQLTQSPSSLSASVGDRVTITCRASQDISSALVWYQQKPGKAPKLLIYDASSLESGVPSR FSGSESGTDFTLTISSLQPEDFATYYCQQFNSYPLTFGGGTKVEIKGGGGSGGGGGDIVLTQ SPDSLAVSLGERATINCRADKSVSTSTYNYLHWYQQKPGQPPKLLIYRGSFLATGVPDRF SGSGSGTDFTLTISSLQAEDVAVYYCQHSRDLPPTFGQGTKLEIKRTVAAPSVFIFPPSDEQ LKSGTASVVCLLNNFYPREAKVQWKVDNALQSGNSQESVTEQDSKDSTYSLSSTLTLSK ADYEKHKVYACEVTHQGLSSPVTKSFNRGEC

SEQ ID NO: 35

Amino acid sequence of chain 3 of EM1:

EVQLVESGGGLVQPGGSLRLSCAASGYTFTSYWLHWVRQAPGKGLEWVGMIDPSNSDT RFNPNFKDRFTISADTSKNTAYLQMNSLRAEDTAVYYCATYRSYVTPLDYWGQGTLVTV SSGGGGSGIPPKIECLPIDISIDEGKVLTVASAFTGEPTPEVTWSTGGRKIHSQEQGRFHIEN TDDSTTLTIKDVQKQDGGLYTLTLRNEFGSDSATVNIHIRSIDKTHTCPPCPAPELLGGPSV FLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVCTLPPSREEM TKNQVSLSCAVKGFYPSDIAVEWESNGQPENNYKTTPPVLDSDGSFFLVSKLTVDKSRW QQGNVFSCSVMHEALHNHYTQKSLSLSPGK

SEQ ID NO: 36

Amino acid sequence of chain 4 of EM1:

DIQMTQSPSSLSASVGDRVTITCKSSQSLLYTSSQKNYLAWYQQKPGKAPKLLIYWASTR ESGVPSRFSGSGSGTDFTLTISSLQPEDFATYYCQQYYAYPWTFGQGTKVEIKGGGGSSG

APRFLTRPKASVVSVGKDATLSCQIVGNPFPQVSWEKDKQPVTAGVRFRLAQDGDLYRL KILDLQLSDSGQYVCRARNAHGEAFACLGLQVDAEA

SEQ ID NO: 37

The amino acid sequences of the 4 chains of EM2 are as follows.
Amino acid sequence of chain 1 of EM2:

EVQLVESGGGLVQPGGSLRLSCAASGYTFTSYWLHWVRQAPGKGLEWVGMIDPSNSDT
RFNPNFKDRFTISADTSKNTAYLQMNSLRAEDTAVYYCATYRSYVTPLDYWGQGTLVTV
SS*ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSC*DKTHTCPPCPAPELLGGPSVFLFP
PKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPCREEMTKN
QVSLWCLVKGFYPSDIAVEWESNGQPENNYKTTPPVLDSDGSFFLYSKLTVDKSRWQQG
NVFSCSVMHEALHNHYTQKSLSLSPGK

SEQ ID NO: 38

Amino acid sequence of chain 2 of EM2:

DIQMTQSPSSLSASVGDRVTITCKSSQSLLYTSSQKNYLAWYQQKPGKAPKLLIYWASTR
ESGVPSRFSGSGSGTDFTLTISSLQPEDFATYYCQQYYAYPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC

SEQ ID NO: 39

Amino acid sequence of chain 3 of EM2:

QVQLVESGGGVVQPGRSLRLSCAASGFTFSTYGMHWVRQAPGKGLEWVAVIWDDGSY
KYYGDSVKGRFTISRDNSKNTLYLQMNSLRAEDTAVYYCARDGITMVRGVMKDYFDY
WGQGTLVTVSSGGGGSGIPPKIECLPIDISIDEGKVLTVASAFTGEPTPEVTWSTGGRKIHS
QEQGRFHIENTDDSTTLTIKDVQKQDGGLYTLTLRNEFGSDSATVNIHIRSIDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV
CTLPPSREEMTKNQVSLSCAVKGFYPSDIAVEWESNGQPENNYKTTPPVLDSDGSFFLVS
KLTVDKSRWQQGNVFSCSVMHEALHNHYTQKSLSLSPGK

SEQ ID NO: 40

Amino acid sequence of chain 4 of EM2:

DIQLTQSPSSLSASVGDRVTITCRASQDISSALVWYQQKPGKAPKLLIYDASSLESGVPSR
FSGSESGTDFTLTISSLQPEDFATYYCQQFNSYPLTFGGGTKVEIKGGGGSSGAPRFLTRPK
ASVVSVGKDATLSCQIVGNPFPQVSWEKDKQPVTAGVRFRLAQDGDLYRLKILDLQLSD
SGQYVCRARNAHGEAFACLGLQVDAEA

SEQ ID NO: 41

Note: in the above sequences, the single underlined part indicates the variable region, the dashed underlined part indicates CL, the dotted underlined part indicates the Fc region, the italicized part indicates CH1, the double underlined part indicates the linker, and the wavy line part indicates the Obscurin chain/Titin-T chain.

Amino acid sequence of linker 1: GGGGSGGGG (SEQ ID NO: 105);

Amino acid sequence of linker 2: GGGGS (SEQ ID NO: 106);

Amino acid sequence of IgGlFc (knob):

DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGK

(SEQ ID NO: 107)

Amino acid sequence of IgG$_1$Fc (hole):

DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK

(SEQ ID NO: 108)

CL:

RTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

(SEQ ID NO: 120).

**Test Examples**

Test Example 1: *In Vitro* Binding Affinity and Kinetics Experiment by Biacore Assay

The affinity of the test molecule for human EGFR (SEQ ID NO: 42) or human HGFR protein (SEQ ID NO: 43) was determined by Biacore T200 (GE) as follows:

The antibody molecules were affinity-captured on a Protein A biosensor chip of which antigens flowed through the surface for 180 s, and then the antibody molecules were dissociated for 600 s. The reaction signals were detected in real time using a Biacore T200 instrument to obtain association and dissociation curves. After dissociation was completed for each cycle, the biosensor chip was washed with 10 mM Gly-HCl (pH 1.5) for regeneration. Data were fitted using a 1:1 model, and affinity values of the test molecules were obtained. In this example, the positive control MCLA was a bispecific antibody specifically binding to HGFR and EGFR (see PB8532 in WO2019031965 for specific sequences).

The experimental results are shown in Table 16. The experimental results showed that the mutant antibodies and bispecific antigen-binding molecules constructed in the present disclosure all had very strong affinity, wherein the affinity of Ab10.1 was at least 4 times higher than that of the parent antibody Ab10.

Table 16. *In vitro* affinity of antibodies of the present invention for human EGFR or human HGFR

| Antibody | Antigen | ka (1/Ms) | kd (1/s) | KD (M) |
|---|---|---|---|---|
| EM1 | EGFR-His | 1.42E+05 | 1.03E-03 | 7.29E-09 |
| | HGFR-His | 5.48E+04 | 1.24E-04 | 2.26E-09 |
| EM2 | EGFR-His | 1.16E+05 | 9.48E-04 | 8.17E-09 |
| | HGFR-His | 5.05E+04 | 1.53E-04 | 3.04E-09 |

(continued)

| Antibody | Antigen | ka (1/Ms) | kd (1/s) | KD (M) |
|---|---|---|---|---|
| MCLA | EGFR-His | 6.85E+04 | 4.73E-03 | 6.91E-08 |
| | HGFR-His | 2.95E+04 | 3.03E-04 | 1.03E-08 |
| Zal | EGFR-His | 7.16E+04 | 1.44E-03 | 2.01E-08 |
| Ab10.1 | HGFR-His | 4.08E+04 | 3.09E-04 | 7.56E-09 |
| Ab10 | HGFR-His | 2.64E+04 | 8.70E-04 | 3.30E-08 |

**Test Example 2: *In Vitro* Binding Experiment of Antibodies to Cells**

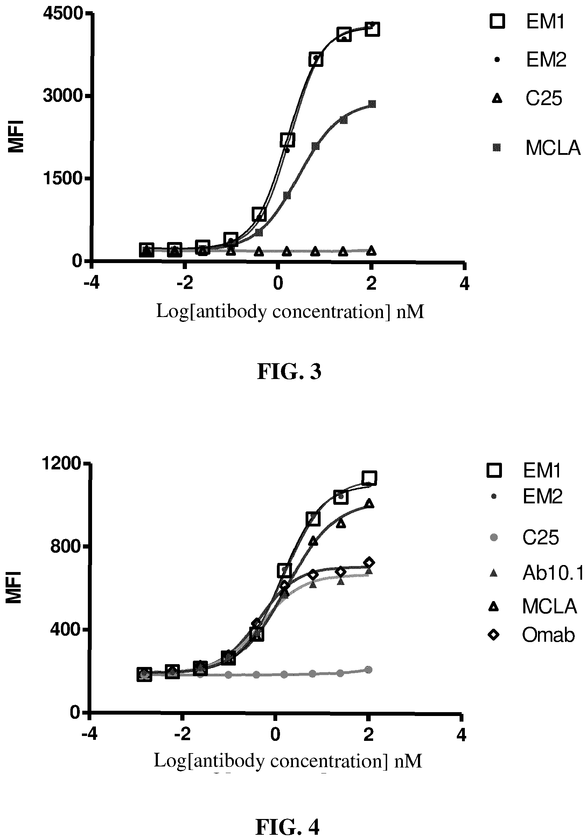

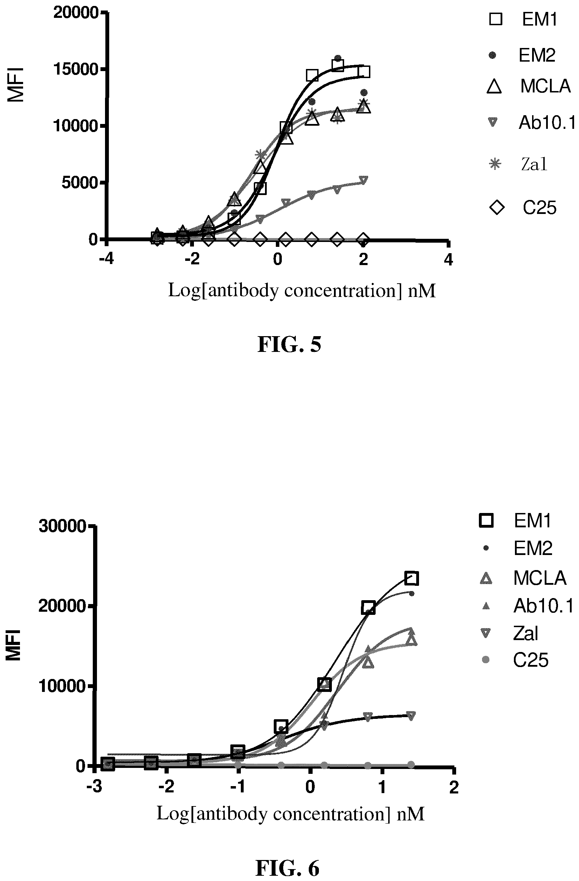

The binding activity of antigen-binding molecules specifically binding to HGFR and EGFR to cells was detected using a flow cytometer. EGFR CHO-S stably transfected cell strain, HGFR CHO-S stably transfected cell strain, and H1975-HGF or MKN-45 tumor cell strain at $1 \times 10^6$ cells/mL were blocked with 1% BSA PBS buffer, followed by the addition of diluted antibody samples at different concentrations (C25 was a negative control, which was an IgG1 antibody protein of irrelevant targets, the same applied hereinafter), and the mixtures were incubated for 1 h. After the mixtures were washed twice, R-PE-goat anti-human (H + L) antibody (Invitrogen, CAT# H10104) was added, and the mixtures were incubated for 0.5 h. After the plate was washed twice, fluorescence signals were read using a flow cytometer.
The experimental results are shown in FIGs. 3 to 6. The experimental results showed that the antigen-binding molecules specifically binding to HGFR and EGFR of the present disclosure had stronger binding ability to tumor cells than that of the positive antibody MCLA.

**Test Example 3: Blocking Experiment of Antibodies Antagonizing EGFR Binding to EGF**

The ability of the antibodies to block EGF/EGFR was tested by ELISA. 1 $\mu$g/mL rabbit anti-His antibody (GenScript, A01857) was coated overnight at 4 °C and then blocked. After the plate was washed, 2 $\mu$g/mL EGFR-His (SEQ ID NO: 42) was added, and the plate was incubated for 1 h. After the plate was washed, 2 $\mu$g/mL EGF-mFc (Aero, EGF-H525b) and diluted antibodies at different concentrations were added, and the plate was incubated for 1 h. After the plate was washed, horseradish peroxidase-goat anti-mouse IgG antibody (Jackson, 115-035-062) was added, and the plate was incubated for 1 h. After the plate was washed, a tetramethylbenzidine solution was added for color development. Finally, a stop solution was added. OD450 values were measured on a microplate reader. In this example, the positive control JNJ was a bispecific antibody specifically binding to HGFR and EGFR (JNJ also known as amivantamab, see WHO Drug Information, 33 (2): 237-239 for specific sequences).
The experimental results are shown in Table 17. The experimental results showed that the antigen-binding molecules specifically binding to HGFR and EGFR of the present disclosure could effectively block the binding of EGF to EGFR.

Table 17. Experimental results of bispecific antigen-binding molecules blocking the binding of EGF to EGFR

| Antibody | IC$_{50}$ ($\mu$g/mL) | Max (%) |
|---|---|---|
| EM1 | 2.37 | 93.5 |
| EM2 | 2.60 | 94.5 |
| JNJ | 3.66 | 90.5 |

**Test Example 4: Blocking Experiment of Antibodies Antagonizing HGFR Binding to HGF**

The ability of the antibodies to block HGF/HGFR was tested by ELISA. 5 $\mu$g/mL HGFR-His (for the sequence, see SEQ ID NO: 43) was coated overnight at 4 °C and then blocked. After the plate was washed, 2 $\mu$g/mL bio-HGF-His (for the sequence, see SEQ ID NO: 44) and diluted antibodies at different concentrations were added, and the plate was incubated for 1 h. After the plate was washed, horseradish peroxidase-streptavidin (Jackson, 016-030-084) was added, and the plate was incubated for 1 h. After the plate was washed, a tetramethylbenzidine solution was added for color development. Finally, a stop solution was added. OD450 values were measured on a microplate reader.
The experimental results are shown in Table 18. The experimental results showed that the antigen-binding molecule specifically binding to HGFR and EGFR of the present disclosure blocked the binding of HGFR to HGF with a greater maximum blocking force than that of the positive antibody JNJ.

Table 18. Experimental results of bispecific antigen-binding molecules blocking the binding of HGF to HGFR

| Antibody | IC50 ($\mu$g/mL) | Max (%) |
|---|---|---|
| EM1 | 0.25 | 95.5 |
| JNJ | 0.27 | 82.3 |
| MCLA | 1.36 | 94.1 |

**Test Example 5: Inhibition Experiment of Cellular EGFR Phosphorylation by Antibodies**

H292 cells (also known as NCI-H292, human lung cancer cells (lymph node metastases) in Cell Bank of Chinese Academy of Sciences, the same applied hereinafter) were plated in a 96-well plate (Corning, 3599) at 12000 cells/well in a medium of RPMI1640 + 10% FBS, and then the 96-well plate was incubated in an incubator at 37 °C with 5% $CO_2$ for 24 h. The next day, the medium was discarded and replaced with a serum-free medium (RPMI640 + 25 mM HEPES + 0.1 mM NEAA + 1 mM sodium pyruvate solution), and the cells were starved for 16-20 h. On the third day, antibodies were each formulated at a first concentration of 6 $\mu$M and subjected to 5-fold gradient dilution, and starvation medium was replaced. The cells were incubated in the incubator at 37 °C with 5% $CO_2$ for 1 h, and 50 $\mu$L of 100 ng/mL rEGF (R&D, 236-EG-200) was added during this period. The cells were incubated in the incubator at 37 °C with 5% $CO_2$ for another 15 min. Finally, the phosphorylated EGFR was detected according to the instructions of PHOSPHO-EGFR (TYR1068) KITS (Cisbio, 64EG1PEG).

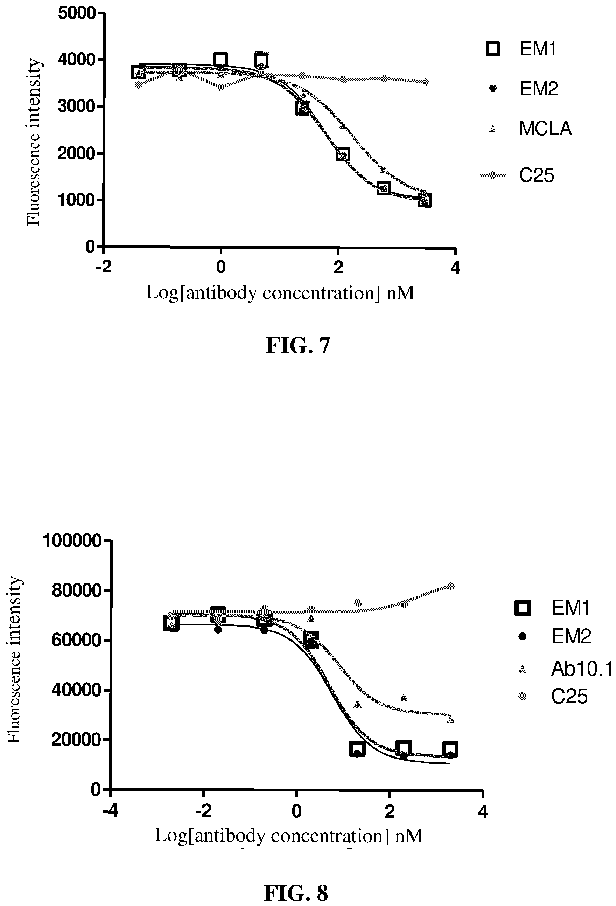

The experimental results are shown in FIG. 7. The experimental results showed that the antigen-binding molecules specifically binding to HGFR and EGFR of the present disclosure were capable of effectively inhibiting the cellular EGFR phosphorylation.

**Test Example 6: Inhibition Experiment of Cellular HGFR Phosphorylation by Antibodies**

H292 cells were plated in a 96-well plate (Corning, 3599) at 12000 cells/well in a medium of RPMI1640 + 10% FBS, and then the 96-well plate was incubated in an incubator at 37 °C with 5% $CO_2$ for 24 h. The next day, the medium was discarded and replaced with a serum-free medium (RPMI640 + 25 mM HEPES + 0.1 mM NEAA + 1 mM sodium pyruvate solution), and the cells were starved for 16-20 h. On the third day, antibodies were each formulated at a first concentration of 1 $\mu$M and subjected to 10-fold gradient dilution, and starvation medium was replaced. The cells were incubated in the incubator at 37 °C with 5% $CO_2$ for 1 h, and 50 $\mu$L of 200 ng/mL rHGF (R&D, 294-HG-005) was added during this period. The cells were incubated in the incubator at 37 °C with 5% $CO_2$ for another 30 min. Finally, the phosphorylated HGFR was detected according to the instructions of p-c-Met (Tyr1234/1235) Assay Kit (PerkinElmer, ALSU-PCMET-A50).

The experimental results are shown in FIG. 8. The experimental results showed that the antigen-binding molecules specifically binding to HGFR and EGFR of the present disclosure were capable of effectively inhibiting the cellular HGFR phosphorylation with stronger inhibition ability than that of Ab 10.1.

**Test Example 7: Inhibition Experiment of Cellular AKT Phosphorylation by Antibodies**

H292 cells were plated in a 96-well plate (Corning, 3599) at 12000 cells/well in a medium of RPMI1640 + 10% FBS, and then the 96-well plate was incubated in an incubator at 37 °C with 5% $CO_2$ for 24 h. The next day, the medium was discarded and replaced with a serum-free medium (RPMI640 + 25 mM HEPES + 0.1 mM NEAA + 1 mM sodium pyruvate solution), and the cells were starved for 16-20 h. On the third day, antibodies were each formulated at a first concentration of 4 $\mu$M and subjected to 5-fold gradient dilution, and starvation medium was replaced. The cells were incubated in the incubator at 37 °C with 5% $CO_2$ for 1 h, and 50 $\mu$L of 200 ng/mL rHGF (R&D, 294-HG-005) and 50 ng/mL rEGF (R&D, 236-EG-200) were added during this period and mixed well. The cells were incubated in the incubator at 37 °C with 5% $CO_2$ for another 1 h. Finally, the phosphorylated AKT was detected according to the instructions of PHOSPHO-AKT (SER473) KITS (Cisbio, 64AKSPEG). The intracellular AKT phosphorylation level was reflected by the ratio of the fluorescence signal value at 665 nm to the fluorescence signal value at 620 nm, i.e., ratio = fluorescence signal value at 665 nm/fluorescence signal value at 620 nm $\times 10^4$, in which the higher the ratio was, the higher the intracellular AKT phosphorylation level was.

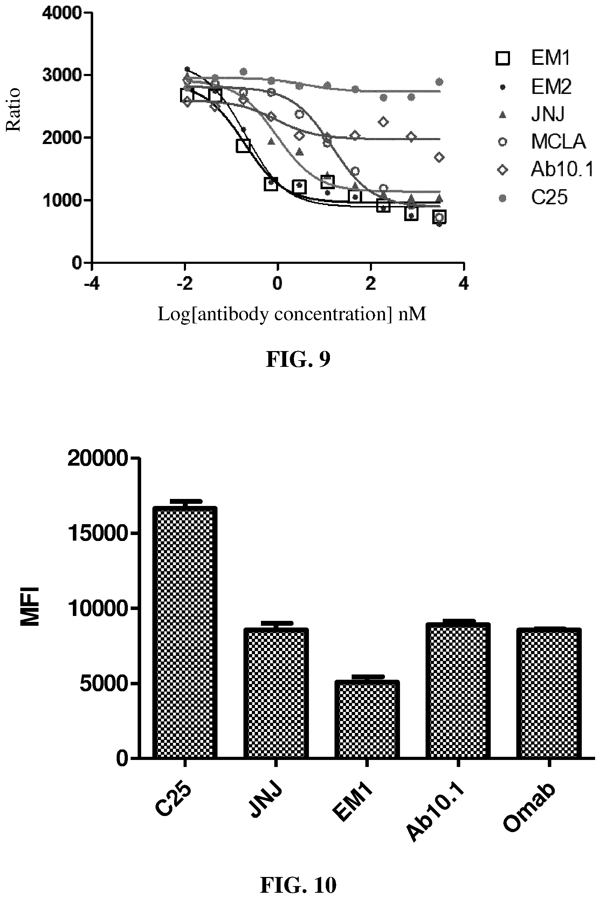

The experimental results are shown in FIG. 9. The experimental results showed that the antigen-binding molecules specifically binding to HGFR and EGFR of the present disclosure were capable of effectively inhibiting the cellular AKT phosphorylation with stronger inhibition ability than that of the positive control antibody.

**Test Example 8: Reduction of HGFR Expression on Cell Surfaces by Antibodies**

HCC827 cells (human non-small cell lung cancer cells, derived from ATCC) were plated in a 6-well plate at 3 $\times$ 10$^5$ cells/well in a medium of RPMI1640 + 10% FBS, and then the 6-well plate was incubated in an incubator at 37 °C with 5% $CO_2$ for 24 h. The antibodies were each formulated to a concentration of 200 nM, treated, and reacted for 18 h. After the treatment, the cells were washed once with PBS, and 500 $\mu$L of enzyme-free cell dissociation buffer (Gibco, #13151) was added to collect the cells. The cells were isolated from the enzyme-free cell dissociation buffer by centrifugation. Immunofluorescence staining was performed using a goat anti-human HGFR antibody (R&D systems, AF276) at 4 °C for 1 h. Subsequently, the cells were washed twice with PBS containing 2% FBS. ALEXA488-donkey anti-sheep secondary antibody (Thermo Fisher, A-11055) was then added, and the reaction was performed at 4 °C for 1 h. After washing twice, the cells were fixed with BD Cytofix (BD, #554655). After the cells were washed with PBS once again, the fluorescence signal values were read using a flow cytometer.

The experimental results are shown in FIG. 10. The experimental results showed that the antigen-binding molecules specifically binding to HGFR and EGFR of the present disclosure were capable of more effectively reducing HGFR on the cell surfaces, thereby inhibiting receptor signaling pathways as compared to the positive control antibody.

**Test Example 9: Inhibition Experiment of Proliferation of SNU-5 Cells by Antibodies**

SNU-5 cells (human gastric cancer cells, derived from ATCC) were plated in a 96-well plate (Corning, 3903) at 4000 cells/well in a medium of IMDM + 5% FBS, and then the 96-well plate was incubated in an incubator at 37 °C with 5% $CO_2$ for 24 h. Antibodies were each formulated to a concentration of 5 $\mu$M, subjected to 5-fold gradient dilution, and added to the 96-well plate described above. The cells were then incubated in the incubator at 37 °C with 5% $CO_2$ for 5 days. Finally, CellTiter-Glo® Luminescent Cell Viability Assay (Promega, G7573) was formulated according to the instructions. 50 $\mu$L of CTG was added to each well, and the cells were incubated at room temperature for 10 min. After the plate was sealed by a sealing membrane, the luminescence intensity (abbreviated as Lum) was detected by using a multi-label microplate detection system (PerkinElmer, victor3).

The calculation formula was as follows: Lum = assay value - medium reading;

$$\%\mathrm{inhibition} = (\mathrm{Lum0} - \mathrm{Lum})/\mathrm{Lum0} \times 100\%,$$

wherein Lum0 was the luminescence intensity of the well with the antibody at a concentration of 0, and Lum was the luminescence intensity of the well with the antibody at each dilution concentration.

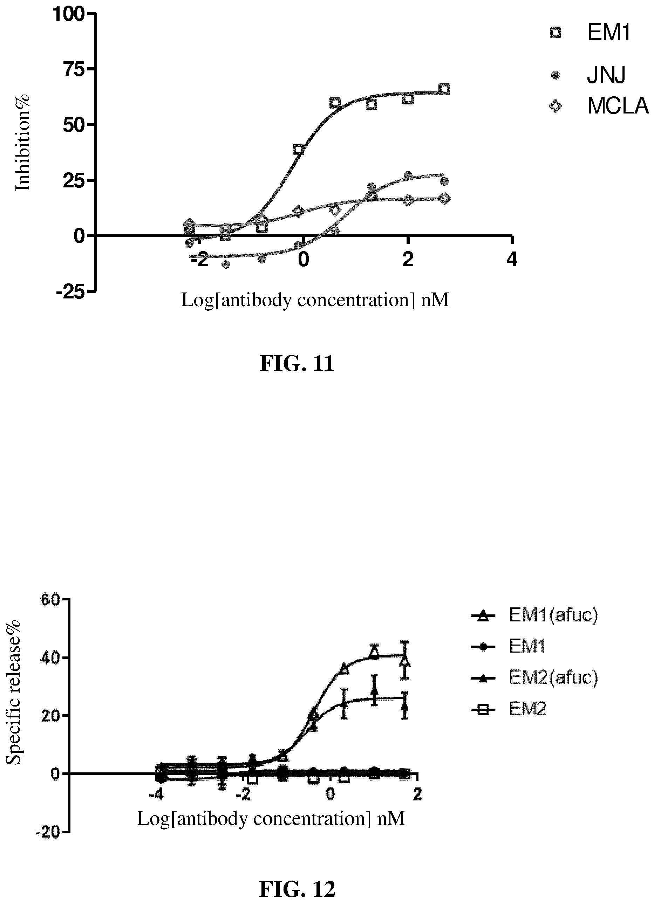

The experimental results are shown in FIG. 11. The experimental results showed that the antigen-binding molecule specifically binding to HGFR and EGFR of the present disclosure had a stronger ability to inhibit the proliferation of SNU-5 cells than that of the positive antibody.

**Test Example 10: ADCC Killing Experiment of Antibodies Against Tumor Cells**

Antibodies were formulated to a concentration of 200 nM and subjected to 5-fold gradient dilution. Tumor cells: Hs746T cells (human gastric cancer cells, Nanjing Kebai Biotechnology Co., Ltd.) and H292 cells were collected. After the cells were washed once with PBS, the cell density was adjusted to 1 $\times$ 10$^6$ cells/mL with a buffer (PBS + 10% FBS + 20 mM HEPES), and then 3 $\mu$L of BATDA reagent (PerkinElmer, AD0116) was added to 2 mL of cells. The mixture was incubated in an incubator at 37 °C with 5% $CO_2$ for 15 min. After centrifugation, the cells were washed three times with a washing buffer (PBS + 20 mM HEPES) and counted, and the cell density was adjusted to 1 $\times$ 10$^5$ cells/mL. NK92--FCGR3A (176V) cells (NK92 cells stably transformed with FCGR3A (176V), purchased from Nanjing Kebai Biotechnology Co., Ltd.) were collected, and washed once with PBS. The cells were then resuspended with a medium and counted, and the cell density was adjusted to 5 $\times$ 10$^5$ cells/mL. Finally, 100 $\mu$L of NK92--FCGR3A (176V) cells, 50 $\mu$L of diluted antibodies, and 50 $\mu$L of labeled tumor cells were added to a 96-well plate (coming, 3788). Meanwhile, a spontaneous control release well that did not contain NK92--FCGR3A (176V) cells but contained 100 $\mu$L of medium was set; a positive control well that contained 10 $\mu$L of lysis buffer + 50 $\mu$L of labeled tumor cells + 140 $\mu$L of medium was set; a background control well that contained 50 $\mu$L of labeled cell supernatant + 150 $\mu$L of medium was set. The 96-well plate was then centrifuged at 300 rpm for 2 min and incubated in an incubator at 37 °C with 5% $CO_2$ for 2 h. 100 $\mu$L of supernatant was transferred into a new 96-well plate (coming, 3788). After the plate was centrifuged at 300 g for 5 min, 20 $\mu$L of supernatant

was transferred into a detection plate (PerkinElmer, AD0116), and 200 µL of europium solution (PerkinElmer, AD0116) was then added. The plate was shaken and incubated at room temperature for 15 min, and finally, TRF was detected, wherein EM1 (afuc) was defucosylated EM1, and EM2 (afuc) was defucosylated EM2.

The calculation formula was as follows: specific release% = (experimental release - spontaneous release)/(maximum release - spontaneous release) × 100.

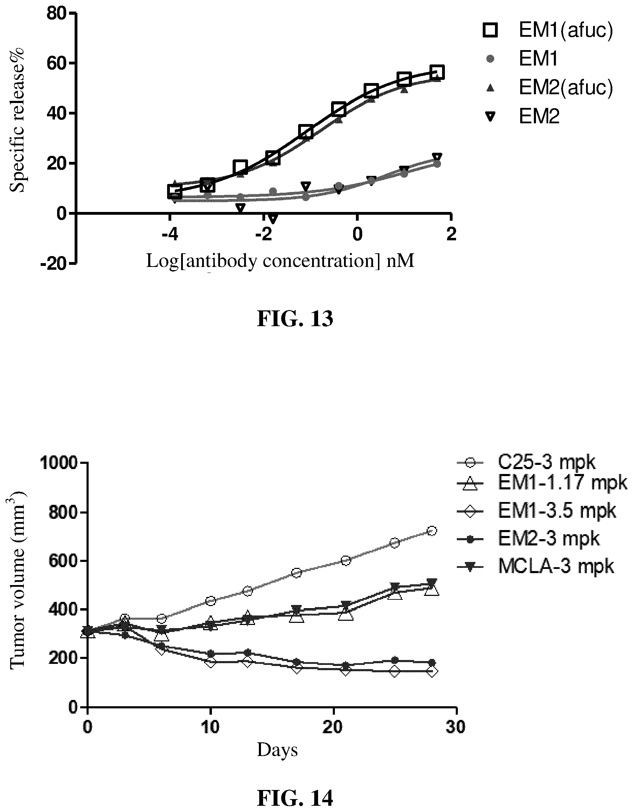

The experimental results are shown in FIGs. 12 and 13. The experimental results showed that the antigen-binding molecules specifically binding to HGFR and EGFR of the present disclosure could significantly improve the ADCC killing ability against cells after defucosylation.

**Test Example 11: Experiment on *In Vivo* Pharmacodynamics of Antibodies in Mouse H1975-HGF Model**

H1975-HGF cells (H1975 cells (human lung adenocarcinoma cells, ATCC) stably transformed with human HGF) were inoculated subcutaneously into the right flank of CD1 nude female mice (Vital River) at $5 \times 10^6$ cells.

When the tumor size was 189 mm$^3$, the animals were randomly grouped into 10 mice per group according to the tumor size, including a C25-3mpk group (a negative control, which was an IgG1 antibody protein of irrelevant targets, administered at a dose of 3 mg/kg), a JNJ-3mpk group (a positive control administered at a dose of 3 mg/kg), and an EM1-3.5mpk group (an EM1 test group administered at a dose of 3.5 mg/kg). Starting from the day when the animals were grouped (day 0), the mice were administered by intraperitoneal injection twice a week for 2 to 3 weeks. The tumor volumes and body weights of the mice were measured twice a week and the results were recorded. The data were statistically analyzed by T-test, wherein the C25-3mpk, JNJ-3mpk, and EM1-3.5mpk were equimolar.

The tumor volume (T) was calculated as follows: T = 1/2 long × (short) ;

$$\text{Relative tumor proliferation rate T/C\%} = (T - T0)/(C - C0) \times 100\%;$$

$$\text{Tumor growth inhibition (TGI\%)} = 1 - \text{T/C \%};$$

wherein T and T0 were tumor volumes of the administration group, wherein T0 was the tumor volume at the beginning of the experiment; c and C0 were tumor volumes of the negative control group, wherein C0 was the tumor volume of the negative control group at the beginning of the experiment.

The experimental results are shown in Table 19. The experimental results showed that the antigen-binding molecule specifically binding to HGFR and EGFR of the present disclosure could significantly inhibit the growth of tumor cells, and on day 21, the mean tumor volume of the equimolar antigen-binding molecule group specifically binding to HGFR and EGFR of the present disclosure was less than half of that of the positive control, indicating the tumor-inhibiting efficacy is stronger than that of the positive control antibody JNJ.

Table 19. Experiment on *in vivo* pharmacodynamics of antibodies in mouse H1975-HGF model

| Group | Mean tumor volume Day 0 | Mean tumor volume D21 | Tumor growth inhibition (%) D21 |
|---|---|---|---|
| C25-3mpk | 189.46 | 2355.30 | - |
| JNJ-3mpk | 190.07 | 551.37 | 83.32% |
| EM1-3.5mpk | 189.63 | 261.10 | 96.70% |

**Test Example 12: Experiment on *In Vivo* Pharmacodynamics of Antibodies in Mouse HCC827 Model**

HCC827 cells (human non-small cell lung cancer cells, derived from ATCC) were inoculated subcutaneously into the right flank of NUNU female mice (Vital River) at $8.555 \times 10^6$ cells.

When the tumor size was 200 mm$^3$, the animals were randomly grouped into 10 mice per group according to the tumor size, including a C25-3mpk group (a negative control, wherein C25 was IgG1 antibody protein of irrelevant targets and administered at a dose of 3 mg/kg), an MCLA-3mpk group (a positive control administered at a dose of 3 mg/kg), an EM1-3.5mpk group (an EM1 high-dose test group administered at a dose of 3.5 mg/kg), an EM1-1.17mpk group (an EM1 low-dose test group administered at a dose of 1.17 mg/kg), and an EM2-3 mpk group (an EM2 test group administered at a dose of 3 mg/kg). Starting from the day when the animals were grouped (day 0), the mice were administered by intraperitoneal injection twice a week for 2 to 3 weeks. The tumor volumes and body weights of the mice were measured twice a week and the results were recorded. wherein the C25-3mpk, MCLA-3mpk, EM1-3.5mpk, and EM2-3 mpk were equimolar.

The experimental results are shown in FIG. 14. The experimental results showed that the antigen-binding molecules specifically binding to HGFR and EGFR of the present disclosure could significantly inhibit the growth of tumor cells, and the tumor-inhibiting effect was stronger than that of the positive control antibody.

## Claims

1. An antigen-binding molecule, comprising:

at least one antigen-binding moiety 1 specifically binding to HGFR, and
at least one antigen-binding moiety 2 specifically binding to EGFR;
wherein
the antigen-binding moiety 1 comprises a heavy chain variable region M-VH and a light chain variable region M-VL, wherein the M-VH comprises an M-HCDR1, an M-HCDR2, and an M-HCDR3, and the M-VL comprises an M-LCDR1, an M-LCDR2, and an M-LCDR3, wherein:

(i) the M-HCDR1, the M-HCDR2, and the M-HCDR3 comprise amino acid sequences of a HCDR1, a HCDR2, and a HCDR3 set forth in SEQ ID NO: 16, respectively, and
the M-LCDR1, the M-LCDR2, and the M-LCDR3 comprise amino acid sequences of a LCDR1, a LCDR2, and a LCDR3 set forth in SEQ ID NO: 17, respectively, or
(ii) the M-HCDR1, the M-HCDR2, and the M-HCDR3 comprise amino acid sequences of a HCDR1, a HCDR2, and a HCDR3 set forth in SEQ ID NO: 12, respectively, and
the M-LCDR1, the M-LCDR2, and the M-LCDR3 comprise amino acid sequences of a LCDR1, a LCDR2, and a LCDR3 set forth in SEQ ID NO: 13, respectively, or
(iii) the M-HCDR1, the M-HCDR2, and the M-HCDR3 comprise amino acid sequences of a HCDR1, a HCDR2, and a HCDR3 set forth in SEQ ID NO: 14, respectively, and

the M-LCDR1, the M-LCDR2, and the M-LCDR3 comprise amino acid sequences of a LCDR1, a LCDR2, and a LCDR3 set forth in SEQ ID NO: 15, respectively;
the antigen-binding moiety 2 comprises a heavy chain variable region E-VH and a light chain variable region E-VL, wherein the E-VH comprises an E-HCDR1, an E-HCDR2, and an E-HCDR3, and the E-VL comprises an E-LCDR1, an E-LCDR2, and an E-LCDR3,
wherein the E-HCDR1, the E-HCDR2, and the E-HCDR3 comprise amino acid sequences of a HCDR1, a HCDR2, and a HCDR3 set forth in SEQ ID NO: 3, respectively, and
the E-LCDR1, the E-LCDR2, and the E-LCDR3 comprise amino acid sequences of a LCDR1, a LCDR2, and a LCDR3 set forth in SEQ ID NO: 5, respectively;
preferably,
the E-HCDR1, the E-HCDR2, the E-HCDR3, the E-LCDR1, the E-LCDR2, and the E-LCDR3 are defined according to Kabat numbering scheme, the E-HCDR1 comprises an amino acid sequence set forth in SEQ ID NO: 6, the E-HCDR2 comprises an amino acid sequence set forth in SEQ ID NO: 7, the E-HCDR3 comprises an amino acid sequence set forth in SEQ ID NO: 8, the E-LCDR1 comprises an amino acid sequence set forth in SEQ ID NO: 9, the E-LCDR2 comprises an amino acid sequence set forth in SEQ ID NO: 10, and the E-LCDR3 comprises an amino acid sequence set forth in SEQ ID NO: 11; and
the M-HCDR1, the M-HCDR2, the M-HCDR3, the M-LCDR1, the M-LCDR2, and
the M-LCDR3 are defined according to the Kabat numbering scheme, wherein

(i) the M-HCDR1 comprises an amino acid sequence set forth in SEQ ID NO: 30, the M-HCDR2 comprises an amino acid sequence set forth in SEQ ID NO: 31, the M-HCDR3 comprises an amino acid sequence set forth in SEQ ID NO: 32, the M-LCDR1 comprises an amino acid sequence set forth in SEQ ID NO: 27, the M-LCDR2 comprises an amino acid sequence set forth in SEQ ID NO: 33, and the M-LCDR3 comprises an amino acid sequence set forth in SEQ ID NO: 29, or
(ii) the M-HCDR1 comprises an amino acid sequence set forth in SEQ ID NO: 18, the M-HCDR2 comprises an amino acid sequence set forth in SEQ ID NO: 19, the M-HCDR3 comprises an amino acid sequence set forth in SEQ ID NO: 20, the M-LCDR1 comprises an amino acid sequence set forth in SEQ ID NO: 21, the M-LCDR2 comprises an amino acid sequence set forth in SEQ ID NO: 22, and the M-LCDR3 comprises an amino acid sequence set forth in SEQ ID NO: 23, or
(iii) the M-HCDR1 comprises an amino acid sequence set forth in SEQ ID NO: 24, the M-HCDR2 comprises an amino acid sequence set forth in SEQ ID NO: 25, the M-HCDR3 comprises an amino acid sequence set forth in SEQ ID NO: 26, the M-LCDR1 comprises an amino acid sequence set forth in SEQ ID NO: 27, the M-LCDR2 comprises an amino acid sequence set forth in SEQ ID NO: 28, and the M-LCDR3 comprises an amino acid sequence set forth in SEQ ID NO: 29.

2. The antigen-binding molecule according to claim 1, wherein:

(i) the antigen-binding molecule comprises two antigen-binding moieties 1 specifically binding to HGFR;

in one of the antigen-binding moieties 1, an M-VH thereof has: an M-HCDR1 comprising the amino acid sequence set forth in SEQ ID NO: 30, an M-HCDR2 comprising the amino acid sequence set forth in SEQ ID NO: 31, and an M-HCDR3 comprising the amino acid sequence set forth in SEQ ID NO: 32, and an M-VL thereof has: an M-LCDR1 comprising the amino acid sequence set forth in SEQ ID NO: 27, an M-LCDR2 comprising the amino acid sequence set forth in SEQ ID NO: 33, and an M-LCDR3 comprising the amino acid sequence set forth in SEQ ID NO: 29;
in the other antigen-binding moiety 1, an M-VH thereof has: an M-HCDR1 comprising the amino acid sequence set forth in SEQ ID NO: 18, an M-HCDR2 comprising the amino acid sequence set forth in SEQ ID NO: 19, and an M-HCDR3 comprising the amino acid sequence set forth in SEQ ID NO: 20, and an M-VL thereof has: an M-LCDR1 comprising the amino acid sequence set forth in SEQ ID NO: 21, an M-LCDR2 comprising the amino acid sequence set forth in SEQ ID NO: 22, and an M-LCDR3 comprising the amino acid sequence set forth in SEQ ID NO: 23; and
in the antigen-binding moiety 2, an E-VH thereof has: an E-HCDR1 comprising the amino acid sequence set forth in SEQ ID NO: 6, an E-HCDR2 comprising the amino acid sequence set forth in SEQ ID NO: 7, and an E-HCDR3 comprising the amino acid sequence set forth in SEQ ID NO: 8, and an E-VL thereof has: an E-LCDR1 comprising the amino acid sequence set forth in SEQ ID NO: 9, an E-LCDR2 comprising the amino acid sequence set forth in SEQ ID NO: 10, and an E-LCDR3 comprising the amino acid sequence set forth in SEQ ID NO: 11; or

(ii) in the antigen-binding moiety 1, an M-VH thereof has: an M-HCDR1 comprising the amino acid sequence set forth in SEQ ID NO: 18, an M-HCDR2 comprising the amino acid sequence set forth in SEQ ID NO: 19, and an M-HCDR3 comprising the amino acid sequence set forth in SEQ ID NO: 20, and an M-VL thereof has: an M-LCDR1 comprising the amino acid sequence set forth in SEQ ID NO: 21, an M-LCDR2 comprising the amino acid sequence set forth in SEQ ID NO: 22, and an M-LCDR3 comprising the amino acid sequence set forth in SEQ ID NO: 23; and

in the antigen-binding moiety 2, an E-VH thereof has: an E-HCDR1 comprising the amino acid sequence set forth in SEQ ID NO: 6, an E-HCDR2 comprising the amino acid sequence set forth in SEQ ID NO: 7, and an E-HCDR3 comprising the amino acid sequence set forth in SEQ ID NO: 8, and an E-VL thereof has: an E-LCDR1 comprising the amino acid sequence set forth in SEQ ID NO: 9, an E-LCDR2 comprising the amino acid sequence set forth in SEQ ID NO: 10, and an E-LCDR3 comprising the amino acid sequence set forth in SEQ ID NO: 11.

3. The antigen-binding molecule according to claim 1 or 2, wherein:

(i) the M-VH comprises an amino acid sequence having at least 90% sequence identity to SEQ ID NO: 16, and the M-VL comprises an amino acid sequence having at least 90% sequence identity to SEQ ID NO: 17; or

the M-VH comprises an amino acid sequence having at least 90% sequence identity to SEQ ID NO: 12, and the M-VL comprises an amino acid sequence having at least 90% sequence identity to SEQ ID NO: 13; or
the M-VH comprises an amino acid sequence having at least 90% sequence identity to SEQ ID NO: 14, and the M-VL comprises an amino acid sequence having at least 90% sequence identity to SEQ ID NO: 15; and/or

(ii) the E-VH comprises an amino acid sequence having at least 90% sequence identity to SEQ ID NO: 3, and the E-VL comprises an amino acid sequence having at least 90% sequence identity to SEQ ID NO: 5 or SEQ ID NO: 4;

preferably,

(i) the antigen-binding molecule comprises two antigen-binding moieties 1 specifically binding to HGFR;

the M-VH of one of the antigen-binding moieties 1 comprises the amino acid sequence set forth in SEQ ID NO: 16, and the M-VL comprises the amino acid sequence set forth in SEQ ID NO: 17; the M-VH of the other antigen-binding moiety 1 comprises the amino acid sequence set forth in SEQ ID NO: 12, and the M-VL comprises the amino acid sequence set forth in SEQ ID NO: 13; and the E-VH comprises the amino acid sequence set forth in SEQ ID NO: 3, and the E-VL comprises the amino acid sequence set forth in SEQ ID NO: 5; or

(ii) the E-VH comprises the amino acid sequence set forth in SEQ ID NO: 3, and the E-VL comprises the amino acid sequence set forth in SEQ ID NO: 5; and

the M-VH comprises the amino acid sequence set forth in SEQ ID NO: 12, and the M-VL comprises the amino acid sequence set forth in SEQ ID NO: 13.

4. The antigen-binding molecule according to any one of claims 1 to 3, wherein the antigen-binding molecule comprises:

two antigen-binding moieties 1 specifically binding to HGFR, an antigen-binding moiety 2 specifically binding to EGFR, and an Fc region comprising a first subunit Fc1 and a second subunit Fc2 capable of associating with each other; preferably, the antigen-binding moiety 2 is an Fv specifically binding to EGFR; one of the two antigen-binding moieties 1 is a first Fab specifically binding to HGFR, and the other is a substituted Fab specifically binding to HGFR; the substituted Fab comprises an M-VH, an M-VL, a Titin chain, and an Obscurin chain, the Titin chain and the Obscurin chain being capable of forming a dimer, wherein the C-terminus of the M-VH of the substituted Fab is fused to the N-terminus of the Titin chain directly or via a linker, and the C-terminus of the M-VL of the substituted Fab is fused to the N-terminus of the Obscurin chain directly or via a linker, or the C-terminus of the M-VH of the substituted Fab is fused to the N-terminus of the Obscurin chain directly or via a linker, and the C-terminus of the M-VL of the substituted Fab is fused to the N-terminus of the Titin chain directly or via a linker; more preferably, the C-terminus of an E-VH of the Fv specifically binding to EGFR is fused to the N-terminus of a heavy chain of the first Fab directly or via a linker, and the C-terminus of an E-VL of the Fv specifically binding to EGFR is fused to the N-terminus of a light chain of the first Fab directly or via a linker, the C-terminus of the heavy chain of the first Fab is fused to the N-terminus of the Fc1 directly or via a linker, and the C-terminus of the Titin chain or the C-terminus of the Obscurin chain is fused to the N-terminus of the Fc2 directly or via a linker.

5. The antigen-binding molecule according to any one of claims 1 to 3, wherein the antigen-binding molecule comprises:

an antigen-binding moiety 1 specifically binding to HGFR, an antigen-binding moiety 2 specifically binding to EGFR, and an Fc region comprising a first subunit Fc1 and a second subunit Fc2 capable of associating with each other; preferably, the antigen-binding moiety 1 is a Fab; the antigen-binding moiety 2 is a substituted Fab comprising an E-VH, an E-VL, a Titin chain, and an Obscurin chain, the Titin chain and the Obscurin chain being capable of forming a dimer; the C-terminus of the E-VH is fused to the N-terminus of the Titin chain directly or via a linker, and the C-terminus of the E-VL is fused to the N-terminus of the Obscurin chain directly or via a linker, or the C-terminus of the E-VH is fused to the N-terminus of the Obscurin chain directly or via a linker, and the C-terminus of the E-VL is fused to the N-terminus of the Titin chain directly or via a linker; more preferably, the C-terminus of a heavy chain of the Fab specifically binding to HGFR is fused to the N-terminus of the Fc1 directly or via a linker; the C-terminus of the Titin chain or the C-terminus of the Obscurin chain is fused to the N-terminus of the Fc2 directly or via a linker.

6. The antigen-binding molecule according to claim 4 or 5, wherein

the Titin chain comprises an amino acid sequence set forth in SEQ ID NO: 45 or a variant thereof, wherein the variant of SEQ ID NO: 45 has one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 60S, 64T, 66S/66K, 70R, 75V, 77S, 79T, 81R, 82M, 83D, and 84L as compared to SEQ ID NO: 45; and the Obscurin chain comprises an amino acid sequence set forth in SEQ ID NO: 64 or a variant thereof, or an amino acid sequence set forth in SEQ ID NO: 65 or a variant thereof,

wherein the variant of SEQ ID NO: 64 has one or more amino acid residue substitutions selected from the group consisting of 2E, 3C, 7K/7R, 9C, 11L, 12S, 13Y/13S, 14T, 17E, 20L, 22M/22S, 25S, 30D, 32P/32F, 34E, 36T, 41K, 42L, 44I, 45T, 48V, 53L, 58V, 62E/62K/62H, 66C, 67Q/67T, 69S, 76S, 82H, 88C, 89L, 92E, 93C, 94G, and 97G as compared to SEQ ID NO: 64;

the variant of SEQ ID NO: 65 has one or more amino acid residue substitutions selected from the group consisting of 6E, 26S, 74C, 77S, 84C, and 86C as compared to SEQ ID NO: 65;

preferably,

the Titin chain comprises an amino acid sequence set forth in any one selected from the group consisting of SEQ ID NO: 45 to SEQ ID NO: 63, and

the Obscurin chain comprises an amino acid sequence set forth in any one selected from the group consisting of SEQ ID NO: 64 to SEQ ID NO: 104;

more preferably,

the Titin chain comprises the amino acid sequence set forth in SEQ ID NO: 61, and

the Obscurin chain comprises the amino acid sequence set forth in SEQ ID NO: 99.

7. The antigen-binding molecule according to any one of claims 1 to 6, wherein the antigen-binding molecule comprises an Fc region comprising a first subunit Fc1 and a second subunit Fc2 capable of associating with each other, and the Fc1 and the Fc2 each independently have one or more amino acid substitutions that reduce homodimerization of the Fc region;

preferably,

(i) the Fc1 has a protuberance structure according to the knob-into-hole technique, and the Fc2 has a pore structure according to the knob-into-hole technique, or the Fc2 has a protuberance structure according to the knob-into-hole technique, and the Fc1 has a pore structure according to the knob-into-hole technique; preferably, the Fc1 has an amino acid residue at position 366 being W, and the Fc2 has an amino acid residue at position 366 being S, an amino acid residue at position 368 being A, and an amino acid residue at position 407 being V; the amino acid residue positions are numbered according to the EU index; and/or

(ii) an engineered disulfide bond is present between the first subunit Fc1 and the second subunit Fc2; preferably, the Fc1 has an amino acid residue at position 354 being C, and the Fc2 has an amino acid residue at position 349 being C; the amino acid residue positions are numbered according to the EU index;

more preferably,

the Fc1 has an amino acid residue at position 354 being C, an amino acid residue at position 356 being E, an amino acid residue at position 358 being M, and an amino acid residue at position 366 being W, and the Fc2 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V; the amino acid residue positions are numbered according to the EU index.

8. The antigen-binding molecule according to claim 7, comprising:

a first chain having a structure of formula (a),
a second chain having a structure of formula (b),
a third chain having a structure of formula (c), and
a fourth chain having a structure of formula (d),
wherein:

formula (a) is [E-VH]-[linker 1]-[M-VH]-[CH1]-[Fc1],
formula (b) is [E-VL]-[linker 2]-[M-VL]-[CL],
formula (c) is [M-VH]-[linker 3]-[Titin chain]-[Fc2], and
formula (d) is [M-VL]-[linker 4]-[Obscurin chain];

preferably, the linkers in formula (a), formula (b), formula (c), and formula (d) are identical or different peptide linkers;

more preferably, the linkers in formula (a), formula (b), formula (c), and formula (d) comprise an amino acid sequence set forth in SEQ ID NO: 105 or SEQ ID NO: 106;

particularly, the antigen-binding molecule has: a first chain comprising an amino acid sequence set forth in SEQ ID NO: 34, a second chain comprising an amino acid sequence set forth in SEQ ID NO: 35, a third chain comprising an amino acid sequence set forth in SEQ ID NO: 36, and a fourth chain comprising an amino acid sequence set forth in SEQ ID NO: 37.

9. The antigen-binding molecule according to any one of claims 1 to 3 and 5 to 7, comprising:

a first chain having a structure of formula (e),
a second chain having a structure of formula (f),
a third chain having a structure of formula (g), and
a fourth chain having a structure of formula (h),
wherein:

formula (e) is [M-VH]-[CH1]-[Fc1],
formula (f) is [M-VL]-[CL],
formula (g) is [E-VH]-[linker 1]-[Titin chain]-[Fc2], and
formula (h) is [E-VL]-[linker 2]-[Obscurin chain];

preferably, the linkers in formula (g) and formula (h) are identical or different peptide linkers;
more preferably, the linkers in formula (h) and formula (g) comprise an amino acid sequence set forth in SEQ ID NO: 106;
particularly,
the antigen-binding molecule has: a first chain comprising an amino acid sequence set forth in SEQ ID NO: 38, a second chain comprising an amino acid sequence set forth in SEQ ID NO: 39, a third chain comprising an amino acid sequence set forth in SEQ ID NO: 40, and a fourth chain comprising an amino acid sequence set forth in SEQ ID NO: 41.

10. An antigen-binding molecule, comprising:

an Fv,
a first Fab,
a substituted Fab, and
an Fc region,
wherein
the Fc region comprises a first subunit Fc1 and a second subunit Fc2 capable of associating with each other,
the Fv comprises a heavy chain variable region Fv-VH and a light chain variable region Fv-VL,
the first Fab comprises a heavy chain variable region Fab1-VH and a light chain variable region Fab1-VL, and
the substituted Fab comprises a heavy chain variable region Fab-S-VH, a light chain variable region Fab-S-VL, a Titin chain, and an Obscurin chain, the Titin chain and
the Obscurin chain being capable of forming a dimer, wherein:

the C-terminus of the Fab-S-VH is fused to the N-terminus of the Titin chain directly or via a linker, and the C-terminus of the Fab-S-VL is fused to the N-terminus of the Obscurin chain directly or via a linker, or the C-terminus of the Fab-S-VH is fused to the N-terminus of the Obscurin chain directly or via a linker, and the C-terminus of the Fab-S-VL is fused to the N-terminus of the Titin chain directly or via a linker; and the C-terminus of a heavy chain of the first Fab is fused to the N-terminus of the Fc1 directly or via a linker; the C-terminus of the Fv-VH of the Fv is fused to the N-terminus of the heavy chain of the first Fab directly or via a linker, the C-terminus of the Titin chain or the C-terminus of the Obscurin chain is fused to the N-terminus of the Fc2 directly or via a linker, and the Fab-S-VH is on the same chain as the Fc2;
preferably, the first Fab and the substituted Fab each independently bind to a first antigen, and the Fv binds to a second antigen; more preferably, the first antigen is HGFR, and the second antigen is EGFR.

11. The antigen-binding molecule according to claim 10, wherein:

the Titin chain comprises an amino acid sequence set forth in SEQ ID NO: 45 or a variant thereof, wherein the variant of SEQ ID NO: 45 has one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 60S, 64T, 66S/66K, 70R, 75V, 77S, 79T, 81R, 82M, 83D, and 84L as compared to SEQ ID NO: 45; and

the Obscurin chain comprises an amino acid sequence set forth in SEQ ID NO: 64 or a variant thereof, or an amino acid sequence set forth in SEQ ID NO: 65 or a variant thereof, wherein the variant of SEQ ID NO: 64 has one or more amino acid residue substitutions selected from the group consisting of 2E, 3C, 7K/7R, 9C, 11L, 12S, 13Y/13S, 14T, 17E, 20L, 22M/22S, 25S, 30D, 32P/32F, 34E, 36T, 41K, 42L, 44I, 45T, 48V, 53L, 58V, 62E/62K/62H, 66C, 67Q/67T, 69S, 76S, 82H, 88C, 89L, 92E, 93C, 94G, and 97G as compared to SEQ ID NO: 64; the variant of SEQ ID NO: 65 has one or more amino acid residue substitutions selected from the group consisting of 6E, 26S, 74C, 77S, 84C, and 86C as compared to SEQ ID NO: 65;

preferably,

the Titin chain comprises an amino acid sequence set forth in any one selected from the group consisting of SEQ ID NO: 45 to SEQ ID NO: 63, and the Obscurin chain comprises an amino acid sequence set forth in any one selected from the group consisting of SEQ ID NO: 64 to SEQ ID NO: 104;

more preferably,

the Titin chain comprises the amino acid sequence set forth in SEQ ID NO: 61, and

the Obscurin chain comprises the amino acid sequence set forth in SEQ ID NO: 99.

12. The antigen-binding molecule according to claim 10 or 11, wherein the Fc1 and the Fc2 each independently have one or more amino acid substitutions that reduce homodimerization of the Fc region;

preferably,

(i) the Fc1 has a protuberance structure according to the knob-into-hole technique, and the Fc2 has a pore structure according to the knob-into-hole technique, or the Fc2 has a protuberance structure according to the knob-into-hole technique, and the Fc1 has a pore structure according to the knob-into-hole technique; preferably, the Fc1 has an amino acid residue at position 366 being W, and the Fc2 has an amino acid residue at position 366 being S, an amino acid residue at position 368 being A, and an amino acid residue at position 407 being V; the amino acid residue positions are numbered according to the EU index; and/or

(ii) an engineered disulfide bond is present between the first subunit Fc1 and the second subunit Fc2; preferably, the Fc1 has an amino acid residue at position 354 being C, and the Fc2 has an amino acid residue at position 349 being C; the amino acid residue positions are numbered according to the EU index;

more preferably,

the Fc1 has an amino acid residue at position 354 being C, an amino acid residue at position 356 being E, an amino acid residue at position 358 being M, and an amino acid residue at position 366 being W, and the Fc2 has an amino acid residue at position 349 being C, an amino acid residue at position 356 being E, an amino acid residue at position 358 being M, an amino acid residue at position 366 being S, an amino acid residue at position 368 being A, and an amino acid residue at position 407 being V; the amino acid residue positions are numbered according to the EU index.

13. The antigen-binding molecule according to any one of claims 10 to 12, comprising:

a first chain having a structure of formula (k),
a second chain having a structure of formula (1),
a third chain having a structure of formula (m), and
a fourth chain having a structure of formula (n),
wherein:

formula (k) is [Fv-VH]-[linker 1]-[Fab1-VH]-[CH1]-[Fc1],
formula (1) is [Fv-VL]-[linker 2]-[Fab1-VL]-[CL],
formula (m) is [Fab-S-VH]-[linker 3]-[Titin chain]-[Fc2], and
formula (n) is [Fab-S-VL]-[linker 4]-[Obscurin chain];

preferably, the linkers in formula (k), formula (1), formula (m), and formula (n) are identical or different peptide linkers;
more preferably, the linkers in formula (k), formula (1), formula (m), and formula (n) comprise an amino acid sequence set forth in SEQ ID NO: 105 or SEQ ID NO: 106.

**14.** An antigen-binding molecule, comprising:

an antigen-binding moiety 2 specifically binding to EGFR, and
two antigen-binding moieties 1 specifically binding to HGFR, the antigen-binding moieties 1 binding to different epitopes of HGFR;
wherein preferably, one of the antigen-binding moieties 1 competes with an anti-HGFR antibody comprising a heavy chain variable region set forth in SEQ ID NO: 16 and a light chain variable region set forth in SEQ ID NO: 17 for binding to human HGFR; the other antigen-binding moiety 1 competes with an anti-HGFR antibody comprising a heavy chain variable region set forth in SEQ ID NO: 12 and a light chain variable region set forth in SEQ ID NO: 13 for binding to the human HGFR.

**15.** The antigen-binding molecule according to any one of claims 1 to 14, wherein the antigen-binding molecule is a low-fucosylated antigen-binding molecule;

preferably, at least 80% of the antigen-binding molecule is not modified by fucosylation;
more preferably, a fucosylation modification in the antigen-binding molecule is undetectable.

**16.** A pharmaceutical composition, comprising:

a therapeutically effective amount of the antigen-binding molecule according to any one of claims 1 to 15, and one or more pharmaceutically acceptable carriers, diluents, buffers, or excipients;
wherein preferably, the pharmaceutical composition further comprises at least one second therapeutic agent.

**17.** An isolated nucleic acid, encoding the antigen-binding molecule according to any one of claims 1 to 15.

**18.** A host cell, comprising the isolated nucleic acid according to claim 17.

**19.** A method for treating a disease, wherein the method comprises the step of administering to a subject a therapeutically effective amount of the antigen-binding molecule according to any one of claims 1 to 15 or the pharmaceutical composition according to claim 16;
preferably, the disease is a tumor; more preferably, the tumor is selected from the group consisting of lung cancer, breast cancer, pancreatic cancer, colorectal cancer, sarcoma, renal cell carcinoma, hepatocellular carcinoma, gastric cancer, ovarian cancer, bladder cancer, head and neck cancer, and glioblastoma.

FIG. 1

FIG. 2

FIG. 3

FIG. 4

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

## INTERNATIONAL SEARCH REPORT

| International application No. |
|---|
| **PCT/CN2022/105714** |

**A.     CLASSIFICATION OF SUBJECT MATTER**

C07K 16/00(2006.01)i;  A61K 39/395(2006.01)i;  C07K 16/28(2006.01)i;  C12N 15/13(2006.01)i

According to International Patent Classification (IPC) or to both national classification and IPC

**B.     FIELDS SEARCHED**

Minimum documentation searched (classification system followed by classification symbols)

C07K,  A61K,  C12N

Documentation searched other than minimum documentation to the extent that such documents are included in the fields searched

Electronic data base consulted during the international search (name of data base and, where practicable, search terms used)

CNTXT, CNABS, VEN, ENTXT, 万方, WANFANG, CNKI, PubMed, GenBank, ISI web of knowledge: HGFR, EGFR, c-MET, 抗原结合分子, 抗体, 双特异, Titin, Obscurin, SEQ ID NOs: 3 and 5-33

**C.     DOCUMENTS CONSIDERED TO BE RELEVANT**

| Category* | Citation of document, with indication, where appropriate, of the relevant passages | Relevant to claim No. |
|---|---|---|
| Y | CN 110028584 A (BEIJING KEXIN BIOTECHNOLOGY CO., LTD.) 19 July 2019 (2019-07-19)<br>claims 1-2 and 4-9, and description, paragraphs 25, 44-46, and 62, and embodiment 1 | 1-3, 15-19 |
| Y | CN 113004391 A (DRAGONFLY THERAPEUTICS INC.) 22 June 2021 (2021-06-22)<br>claim 1, and description, paragraph 55 | 1-3, 15-19 |
| Y | CN 111196853 A (SUZHOU SUNCADIA BIOPHARMACEUTICALS CO., LTD. et al.) 26 May 2020 (2020-05-26)<br>claim 13 | 1-3, 15-19 |
| Y | CN 104955838 A (JANSSEN BIOTECH, INC.) 30 September 2015 (2015-09-30)<br>claims 1-65 | 1-3, 15-19 |
| Y | WO 2019031965 A1 (MERUS N.V.) 14 February 2019 (2019-02-14)<br>claims 1-45 | 1-3, 15-19 |
| A | CN 112513074 A (GENMAB AS) 16 March 2021 (2021-03-16)<br>entire document | 1-19 |

☐ Further documents are listed in the continuation of Box C.          ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 September 2022** | **10 October 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/105714**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

   a.  ☑ forming part of the international application as filed:

   ☑ in the form of an Annex C/ST.25 text file.

   ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

   [1]  The actually submitted sequence table is an XML file in Standard ST.26.

Form PCT/ISA/210 (continuation of first sheet) (January 2015)

**INTERNATIONAL SEARCH REPORT**

International application No.

**PCT/CN2022/105714**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **19**
   because they relate to subject matter not required to be searched by this Authority, namely:

   [1]   Claim 19 relates to a method for treating a living human or animal body, belonging to the cases listed in PCT Rule 39.1(iv) for which no international search is required. The present report relates to conducting a search on the basis of "a use in the preparation of a pharmaceutical composition".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/105714**

| Patent document cited in search report | | | Publication date (day/month/year) | Patent family member(s) | | | Publication date (day/month/year) |
|---|---|---|---|---|---|---|---|
| CN | 110028584 | A | 19 July 2019 | None | | | |
| CN | 113004391 | A | 22 June 2021 | KR | 20210029298 | A | 15 March 2021 |
| | | | | US | 2020216544 | A1 | 09 July 2020 |
| | | | | SG | 10202102251Y | A | 29 April 2021 |
| | | | | IL | 272553 | A | 31 March 2020 |
| | | | | CA | 3112990 | A1 | 21 February 2019 |
| | | | | JP | 2020531438 | A | 05 November 2020 |
| | | | | CA | 3073117 | A1 | 21 February 2019 |
| | | | | AU | 2021201451 | A1 | 25 March 2021 |
| | | | | US | 2021261668 | A1 | 26 August 2021 |
| | | | | EP | 3882270 | A2 | 22 September 2021 |
| | | | | KR | 20200037388 | A | 08 April 2020 |
| | | | | JP | 2021098732 | A | 01 July 2021 |
| | | | | EP | 3668893 | A1 | 24 June 2020 |
| | | | | AU | 2018318698 | A1 | 20 February 2020 |
| | | | | WO | 2019035939 | A1 | 21 February 2019 |
| | | | | BR | 112020003050 | A2 | 01 September 2020 |
| | | | | CN | 111065649 | A | 24 April 2020 |
| | | | | SG | 11201913969S | A | 30 January 2020 |
| | | | | IL | 281305 | A | 29 April 2021 |
| CN | 111196853 | A | 26 May 2020 | BR | 112017021245 | A2 | 26 June 2018 |
| | | | | CN | 106188293 | A | 07 December 2016 |
| | | | | JP | 2018516539 | A | 28 June 2018 |
| | | | | CA | 2982777 | A1 | 20 October 2016 |
| | | | | CN | 106687480 | A | 17 May 2017 |
| | | | | KR | 20170138451 | A | 15 December 2017 |
| | | | | MX | 2017012965 | A | 06 June 2018 |
| | | | | RU | 2017135257 | A | 17 May 2019 |
| | | | | US | 2018110875 | A1 | 26 April 2018 |
| | | | | EP | 3284751 | A1 | 21 February 2018 |
| | | | | TW | 201638108 | A | 01 November 2016 |
| | | | | AU | 2016248357 | A1 | 26 October 2017 |
| CN | 104955838 | A | 30 September 2015 | HU | S2200016 | I1 | 28 May 2022 |
| | | | | HU | E041499 | T2 | 28 May 2019 |
| | | | | US | 2014255408 | A1 | 11 September 2014 |
| | | | | SG | 11201503938V | A | 29 June 2015 |
| | | | | CA | 2893505 | A1 | 30 May 2014 |
| | | | | UA | 117121 | C2 | 25 June 2018 |
| | | | | US | 2014141000 | A1 | 22 May 2014 |
| | | | | PT | 3447069 | T | 13 November 2020 |
| | | | | LT | 2922872 | T | 27 December 2018 |
| | | | | ES | 2831374 | T3 | 08 June 2021 |
| | | | | CY | 1121270 | T1 | 29 May 2020 |
| | | | | JP | 2021006561 | A | 21 January 2021 |
| | | | | CN | 113201073 | A | 03 August 2021 |
| | | | | EP | 3808767 | A1 | 21 April 2021 |
| | | | | IL | 238796 | D0 | 30 June 2015 |
| | | | | EA | 201590985 | A1 | 30 November 2015 |
| | | | | HR | P20182128 | T1 | 08 February 2019 |
| | | | | DK | 3447069 | T3 | 16 November 2020 |

Form PCT/ISA/210 (patent family annex) (January 2015)

# EP 4 372 001 A1

**INTERNATIONAL SEARCH REPORT**
**Information on patent family members**

International application No.

**PCT/CN2022/105714**

| Patent document cited in search report | | | Publication date (day/month/year) | Patent family member(s) | | | Publication date (day/month/year) |
|---|---|---|---|---|---|---|---|
| | | | | CL | 2015001356 | A1 | 06 November 2015 |
| | | | | JP | 2022062155 | A | 19 April 2022 |
| | | | | SI | 2922872 | T1 | 31 January 2019 |
| | | | | WO | 2014081954 | A1 | 30 May 2014 |
| | | | | JP | 2016505537 | A | 25 February 2016 |
| | | | | AU | 2021202394 | A1 | 20 May 2021 |
| | | | | MX | 2015006387 | A | 03 December 2015 |
| | | | | US | 2017101475 | A1 | 13 April 2017 |
| | | | | NL | 301173 | I1 | 04 May 2022 |
| | | | | DK | 2922872 | T3 | 02 January 2019 |
| | | | | NZ | 708352 | A | 25 October 2019 |
| | | | | KR | 20150087365 | A | 29 July 2015 |
| | | | | PL | 2922872 | T3 | 29 March 2019 |
| | | | | PH | 12015501118 | A1 | 01 February 2016 |
| | | | | NI | 201500069 | A | 19 October 2015 |
| | | | | AU | 2019200441 | A1 | 07 February 2019 |
| | | | | SI | 3447069 | T1 | 26 February 2021 |
| | | | | PE | 20151181 | A1 | 19 August 2015 |
| | | | | JP | 2019048817 | A | 28 March 2019 |
| | | | | KR | 20220032654 | A | 15 March 2022 |
| | | | | EP | 3447069 | A1 | 27 February 2019 |
| | | | | AU | 2013347962 | A1 | 28 May 2015 |
| | | | | RS | 58192 | B1 | 29 March 2019 |
| | | | | EP | 2922872 | A1 | 30 September 2015 |
| | | | | HR | P20201848 | T1 | 08 January 2021 |
| | | | | LT | PA2022507 | I1 | 27 June 2022 |
| | | | | RS | 61057 | B1 | 31 December 2020 |
| | | | | ES | 2700231 | T3 | 14 February 2019 |
| | | | | LT | 3447069 | T | 10 December 2020 |
| | | | | HU | E052548 | T2 | 28 May 2021 |
| | | | | PT | 2922872 | T | 18 January 2019 |
| WO | 2019031965 | A1 | 14 February 2019 | CA | 3072404 | A1 | 14 February 2019 |
| | | | | JP | 2020530028 | A | 15 October 2020 |
| | | | | MA | 49846 | A | 17 June 2020 |
| | | | | IL | 272461 | A | 31 March 2020 |
| | | | | KR | 20200042485 | A | 23 April 2020 |
| | | | | SG | 11202001050P | A | 30 March 2020 |
| | | | | US | 2020247892 | A1 | 06 August 2020 |
| | | | | PH | 12020550055 | A1 | 19 October 2020 |
| | | | | TW | 201910354 | A | 16 March 2019 |
| | | | | EP | 3665198 | A1 | 17 June 2020 |
| | | | | AU | 2018312816 | A1 | 27 February 2020 |
| | | | | EA | 202090215 | A1 | 01 July 2020 |
| | | | | BR | 112020002695 | A2 | 25 August 2020 |
| | | | | AU | 2021202874 | A1 | 03 June 2021 |
| | | | | CN | 111094351 | A | 01 May 2020 |
| CN | 112513074 | A | 16 March 2021 | US | 2021269509 | A1 | 02 September 2021 |
| | | | | WO | 2019243626 | A1 | 26 December 2019 |
| | | | | IL | 279528 | A | 31 January 2021 |
| | | | | JP | 2021528435 | A | 21 October 2021 |

Form PCT/ISA/210 (patent family annex) (January 2015)

INTERNATIONAL SEARCH REPORT
Information on patent family members

International application No.

**PCT/CN2022/105714**

| Patent document cited in search report | Publication date (day/month/year) | Patent family member(s) | | Publication date (day/month/year) |
|---|---|---|---|---|
| | | CA | 3104390 A1 | 26 December 2019 |
| | | SG | 11202012713R A | 28 January 2021 |
| | | EP | 3810649 A1 | 28 April 2021 |
| | | MA | 52945 A | 28 April 2021 |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110794137
- US 20040110704 A
- WO 03035835 A
- EP 1176195 A
- US 20120276086 A
- US 8642292 B
- WO 9627011 A
- WO 98050431 A
- EP 1870459 A
- WO 2007110205 A
- WO 007147901 A
- WO 2009089004 A
- WO 2010129304 A
- WO 201190754 A
- WO 2011143545 A
- WO 2012058768 A

- WO 2013157954 A
- WO 013096291 A
- US 5959177 A
- US 6040498 A
- US 6420548 B
- US 7125978 B
- US 6417429 B
- CN 2021070832 W
- WO 2021139758 A1
- CN 202110527339
- WO 2020177733 A1
- WO 2013003680 A1
- WO 2016165580 A1
- WO 2019031965 A

**Non-patent literature cited in the description**

- *J. Biol. Chem,* 1968, vol. 243, 3558
- **ARAN F. LABRIJN et al.** *Nature Reviews Drug Discovery,* 2019, vol. 18, 585-608
- **CHEN S1 et al.** *J Immunol Res.,* 11 February 2019, vol. 2019, 4516041
- **KABAT et al.** Sequences of Proteins of Immunological Interest. Public Health Service, National Institutes of Health, 1991
- **MARTIN.** Protein Sequence and Structure Analysis of Antibody Variable Domains[J. *ACR,* 2001
- **LEFRANC, M.P. et al.** *Dev. Comp. Immunol.,* 2003, vol. 27, 55-77
- *Front Immunol.,* 16 October 2018, vol. 9, 2278
- *Meth. Mol. Biol.,* 2004, vol. 248, 443-463
- *Prot. Sci.,* 2000, vol. 9, 487-496
- **HARLOW ; LANE.** Antibodies. Cold Spring Harbor Press

- **YAMANE-OHNUKI et al.** Establishment of FUT8knockout Chinese hamster ovary cells:an ideal host cellline for producing completely defucosylated antibodies with enhancedantibody-dependent cellular cytotoxicity. *BIOTECHNOLOGY AND BIOENGINEERING,* 2004, 614-622
- **TARENTINO et al.** *Biochem.,* 1975, vol. 14, 5516
- **NATSUME et al.** *Drug Des. Devel. Ther.,* 2009, vol. 3, 7
- **HSE et al.** *J. Biol. Chem.,* 1997, vol. 272, 9062-9070
- **YANG et al.** *Nature Biotechnology,* 2015, vol. 33, 842-844
- **YAMANE-OHNUKI et al.** *Biotechnol. Bioeng.,* 2004, vol. 87, 614
- **YAZAKI, P ; WU, A.M.** Methods in Molecular Biology. Humana Press, 2004, vol. 248, 255-268
- Antibodies: A Laboratory Manual and Molecular Cloning: A Laboratory Manual. Cold Spring Harbor Laboratory
- *WHO Drug Information,* vol. 33 (2), 237-239